# EUROPEAN PATENT SPECIFICATION

(11) **EP 1 797 184 B1**
(45) Date of publication and mention of the grant of the patent: **26.09.2012**
(21) Application number: 05810327.6
(22) Date of filing: 07.10.2005
(51) Int. Cl.: C12N 15/11

(54) **TARGETED TRANS-SPLICING OF HIGHLY ABUNDANT TRANSCRIPTS FOR IN VIVO PRODUCTION OF RECOMBINANT PROTEINS**
GERICHTETES TRANSSPLEISSEN VON HOCHABUNDANTEN TRANSKRIPTEN ZUR IN-VIVO-HERSTELLUNG REKOMBINANTER PROTEINE
TRANSEPISSAGE CIBLE DE TRANSCRITS PRESENTS EN GRANDE ABONDANCE POUR LA PRODUCTION IN VIVO DE PROTEINES DE RECOMBINAISON

(30) Priority: 08.10.2004 US 617324 P; 21.01.2005 US 41155; 31.05.2005 US 141447
(43) Date of publication of application: 20.06.2007
(73) Proprietor: VIRxSYS Corporation, Gaithersburg, MD 20877 (US)
(72) Inventor: MCGARRITY, Gerard, J., Gaithersburg, MD 20878 (US); GARCIA-BLANCO, Mariano, A., Durham, NC 27713 (US)
(74) Representative: Lord, Hilton David
(86) International application number: PCT/US2005/036424
(87) International publication number: WO 2006/042232

(56) References cited:
- WO-A2-01/92291
- US-A- 6 013 487
- US-A- 6 083 702
- US-A1- 2004 126 774
- US-B1- 6 280 978
- PUTTARAJU M ET AL: "Spliceosome-mediated RNA trans-splicing as a tool for gene therapy" NATURE BIOTECHNOLOGY, NATURE PUBLISHING GROUP, NEW YORK, NY, US, vol. 17, no. 3, March 1999 (1999-03), pages 246-252, XP002130564 ISSN: 1087-0156

## Description

### FIELD OF THE INVENTION

The present invention provides methods and compositions for generating novel nucleic acid molecules through RNA *trans*-splicing that target abundantly expressed precursor messenger RNA molecule (target pre-mRNA) and contain the coding sequence of a therapeutic protein or polypeptide of interest. The compositions of the invention include pre-*trans*-splicing molecules (PTMs) designed to interact with the target pre-mRNA, and mediate a *trans*-splicing reaction, resulting in the generation of a novel chimeric RNA molecule (chimeric RNA) capable of encoding a therapeutic protein or polypeptide of interest. In particular, the target pre-mRNA is albumin pre-mRNA. In one aspect, the purpose of the invention is to develop *in vivo* production of chimeric RNA molecules comprising sequences that encode a protein or polypeptide of interest that is of therapeutic importance in a host animal from which the protein can be recovered in bulk amounts. The present invention also provides methods for the large-scale production of therapeutic proteins or polypeptides of interest.

The compositions of the invention further include recombinant vector systems capable of expressing the PTMs of the invention and cells expressing said PTMs. The methods of the invention encompass contacting the PTMs of the invention with the target pre-mRNA under conditions in which a portion of the target pre-mRNA to form a chimeric RNA molecule that would express a protein or polypeptide of interest.

### BACKGROUND OF THE INVENTION

### PRODUCTION OF LARGE SCALE RECOMBINANT PROTEINS

Recombinant protein production technology provides for the generation of large quantities of protein for function and structure analysis, industrial uses, drug design, diagnostics, therapy, and vaccines. Mammalian and insect expression systems have been employed to produce biologically active recombinant proteins in a short period of time with high yields. Bioreactors containing cells incubated under specific conditions have been fruitful in large-scale production of recombinant proteins.

Transgenic dairy cattle and transgenic goats offer alternatives to bioreactors as a high volume source of recombinant proteins. Recombinant proteins of interest are expressed in the milk of these transgenic animals, which can be collected and harvested for the recombinant protein of interest. Cattle can produce over 9000 liters of milk per years and are capable of producing large amounts of proteins in their milk, which is comparable to the amounts generated in bioreactors. Cattle can express complex proteins that cannot be produced economically by cell culture. Large-scale production of valuable therapeutic proteins, with flexible scale-up and significantly lower capital costs and risks are also possible. Cattle are also a safe and renewable source of recombinant proteins or polypeptides. It enables the manufacture of complex or unique molecules that cannot be produced efficiently by any other method.

Goats are also potential host animals for the production of large scale therapeutic proteins (Baldassarre et al., State of the art in the production of transgenic goats. Reprod Fertil Dev. 2004, 16:465-70). Goats have been shown to express various proteins in their milk, including, *inter alia*, human growth hormone, insulin, spider silk, antithrombin III, tissue plasminogen activator, and α1-antitrypsin. Genzyme Transgenics, for example, has expressed over 14 proteins in transgenic goats at greater than 1g/L of milk (Rathin C. Das, Production of therapeutic proteins from transgenic animals. BioBusiness, February 2001, pp.60-64).

### RNA SPLICING

DNA sequences in the chromosome are transcribed into pre-mRNAs that contain coding regions (exons) and generally also contain intervening non-coding regions (introns). Introns are removed from pre-mRNAs in a precise process called *cis*-splicing (Chow et al., 1977, Cell 12:1-8; and Berget, S.M. et al., 1977, Proc. Natl. Acad. Sci. USA 74:3171-3175). Splicing takes place as a coordinated interaction of several small nuclear ribonucleoprotein particles (snRNP's) and many protein factors that assemble to form an enzymatic complex known as the spliceosome (Moore et al., 1993, in The RNA World, R.F. Gestland and J.F. Atkins eds. (Cold Spring Harbor Laboratory Press, Cold Spring Harbor, N.Y.); Kramer, 1996, Annu. Rev. Biochem., 65:367-404; Staley and Guthrie, 1998, Cell 92:315-326).

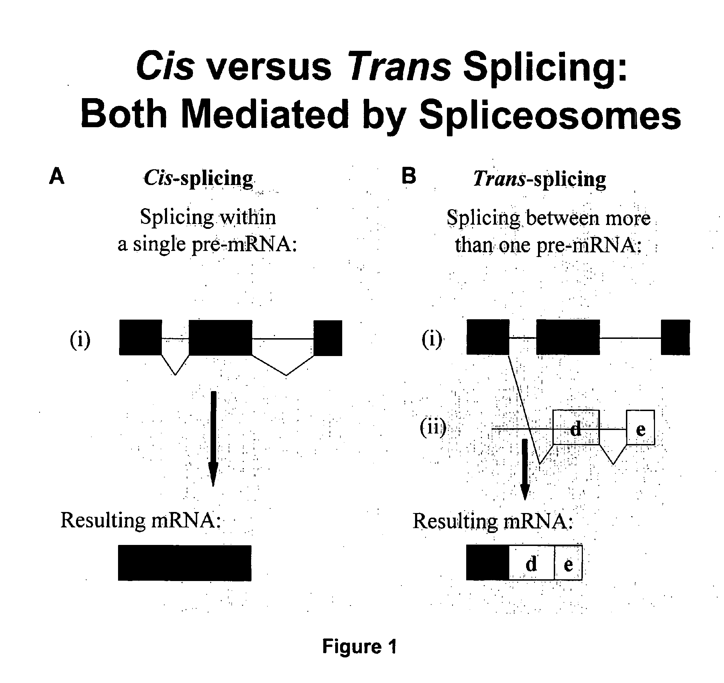

In most cases, the splicing reaction occurs within the same pre-mRNA molecule, which is termed *cis*-splicing. Splicing between two independently transcribed pre-mRNAs is termed *trans*-splicing. (*See* Figure 1) *Trans*-splicing was first discovered in trypanosomes (Sutton & Boothroyd, 1986, Cell 47:527; Murphy et al., 1986, Cell 47:517) and subsequently in nematodes (Krause & Hirsh, 1987, Cell 49:753); flatworms (Rajkovic et al., 1990, Proc. Nat'l. Acad. Sci. USA, 87:8879; Davis et al., 1995, J. Biol. Chem. 270:21813) and in plant mitochondria (Malek et al., 1997, Proc. Nat'l. Acad. Sci. USA 94:553). In the parasite *Trypanosoma brucei*, all mRNAs acquire a splice leader (SL) RNA at their 5' termini by *trans*-splicing. A 5' leader sequence is also *trans*-spliced onto some genes in *Caenorhabditis elegans.* This mechanism is appropriate for adding a single common sequence to many different transcripts.

The mechanism of splice leader *trans*-splicing, which is nearly identical to that of conventional *cis*-splicing, proceeds via two phosphoryl transfer reactions. The first causes the formation of a 2'-5' phosphodiester bond producing a 'Y' shaped branched intermediate, equivalent to the lariat intermediate in *cis*-splicing. The second reaction, exon ligation, proceeds as in conventional *cis*-splicing. In addition, sequences at the 3' splice site and some of the snRNPs that catalyze the *trans*-splicing reaction, closely resemble their counterparts involved in *cis*-splicing.

*Trans*-splicing may also refer to a different process, where an intron of one pre-mRNA interacts with an intron of a second pre-mRNA, enhancing the recombination of splice sites between two conventional pre-mRNAs. This type of *trans*-splicing was postulated to account for transcripts encoding a human immunoglobulin variable region sequence linked to the endogenous constant region in a transgenic mouse (Shimizu et al., 1989, Proc. Nat'l. Acad. Sci. USA 86:8020). In addition, *trans*-splicing of c-myb pre-RNA has been demonstrated (Vellard, M. et al. Proc. Nat'l. Acad. Sci., 1992 89:2511-2515) and more recently, RNA transcripts from cloned SV40 *trans*-spliced to each other were detected in cultured cells and nuclear extracts (Eul et al., 1995, EMBO. J. 14:3226). However, naturally occurring *trans*-splicing of mammalian pre-mRNAs is thought to be a rare event (Flouriot G. et al., 2002 J. Biol. Chem*:* Finta, C. et al., 2002 J. Biol Chem 277:5882-5890).

*In vitro trans*-splicing has been used as a model system to examine the mechanism of splicing by several groups (Konarska & Sharp, 1985, Cell 46:165-171 Solnick, 1985, Cell 42:157; Chiara & Reed, 1995, Nature 375:510; Pasman and Garcia-Blanco, 1996, Nucleic Acids Res. 24:1638). Reasonably efficient *trans*-splicing (30% of *cis*-spliced analog) was achieved between RNAs capable of base pairing to each other, splicing of RNAs not tethered by base pairing was further diminished by a factor of 10. Other *in vitro trans-*splicing reactions not requiring obvious RNA-RNA interactions among the substrates were observed by Chiara & Reed (1995, Nature 375:510), Bruzik J.P. & Maniatis, T. (1992, Nature 360:692) and Bruzik J.P. and Maniatis, T., (1995, Proc. Nat'l. Acad. Sci. USA 92:7056-7059). These reactions occur at relatively low frequencies and require specialized elements, such as a downstream 5' splice site or exonic splicing enhancers.

In addition to splicing mechanisms involving the binding of multiple proteins to the precursor mRNA which then act to correctly cut and join RNA, a third mechanism involves cutting and joining of the RNA by the intron itself, by what are termed catalytic RNA molecules or ribozymes. The cleavage activity of ribozymes has been targeted to specific RNAs by engineering a discrete "hybridization" region into the ribozyme. Upon hybridization to the target RNA, the catalytic region of the ribozyme cleaves the target. It has been suggested that such ribozyme activity would be useful for the inactivation or cleavage of target RNA *in vivo*, such as for the treatment of human diseases characterized by production of foreign of aberrant RNA. In such instances small RNA molecules are designed to hybridize to the target RNA and by binding to the target RNA prevent translation of the target RNA or cause destruction of the RNA through activation of nucleases. The use of antisense RNA has also been proposed as an alternative mechanism for targeting and destruction of specific RNAs.

Using the *Tetrahymena* group I ribozyme, targeted *trans*-splicing was demonstrated in *E. coli*. (Sullenger B.A. and Cech. T.R., 1994, Nature 341:619-622), in mouse fibroblasts (Jones, J.T. et al., 1996, Nature Medicine 2:643-648), human fibroblasts (Phylacton, L.A. et al. Nature Genetics 18:378-381) and human erythroid precursors (Lan et al., 1998, Science 280: 1593-1596). For a review of clinically relevant technologies to modify RNA, see Sullenger and Gilboa, 2002 Nature 418:252-8. The present invention relates to the use of targeted *trans-*splicing mediated by native mammalian splicing machinery, *i.e.*, spliceosomes, to reprogram or alter the coding sequence of a targeted mRNA.

U.S. Patent Nos. 6,083,702, 6,013,487 and 6,280,978 describe the use of PTMs to mediate a *trans*-splicing reaction by contacting a target precursor mRNA to generate novel chimeric RNAs.

Published International Application WO 2005/070023, which is in common ownership with the present application, is Article 54(3) prior art on the basis of its priority claims and describes use of PTMs to mediate a trans-splicing reaction with albumin pre-mRNA to generate chimeric RNAs encoding apolipoprotein A1 or a variant of apolipoprotein A1 joined to a portion of albumin.

### SUMMARY OF THE INVENTION

The present invention provides methods and compositions for generating novel nucleic acid molecules through RNA *trans*-splicing that target an abundantly expressed precursor messenger RNA molecule (target pre-mRNA) and contain the coding sequence of a therapeutic protein or polypeptide of interest. The compositions of the invention include pre-*trans*-splicing molecules (PTMs) designed to interact with the abundantly expressed target pre-mRNA, and mediate a *trans*-splicing reaction resulting in the generation of novel chimeric RNA molecule (chimeric RNA) capable of encoding a therapeutic protein or polypeptide of interest. Said therapeutic protein or polypeptide is not apolipoprotein A1 or a variant thereof. The abundantly expressed target pre-mRNA codes for albumin. As indicated above, in one aspect the purpose of the invention is to develop *in vivo* production of chimeric RNA molecules comprising sequences that encode a therapeutic protein or polypeptide of interest in a host animal from which the polypeptide or protein can be recovered in bulk amounts.

The compositions of the invention further include recombinant vector systems capable of expressing the PTMs of the invention and cells expressing said PTMs. The methods of the invention encompass contacting the PTMs of the invention with the target pre-mRNA under conditions in which a portion of the PTM is *trans*-spliced to a portion of the target pre-mRNA to form a chimeric RNA molecule that would express a protein or polypeptide of interest.

The present invention provides for methods of large scale bulk productions of therapeutic proteins or polypeptides of interest.

### BRIEF DESCRIPTION OF THE DRAWINGS

Figure 1 shows a schematic representation of cis versus trans-splicing reactions.
Figure 2 shows a schematic representation of pre-*trans*-splicing molecules (PTMs).
Figure 3 shows a schematic representation of a *trans*-splicing reaction between the target 5' splice site and PTM's 3' splice site and 3' exon replacement.
Figure 4 shows the rationale for selecting albumin transcripts as the target pre- mRNA.

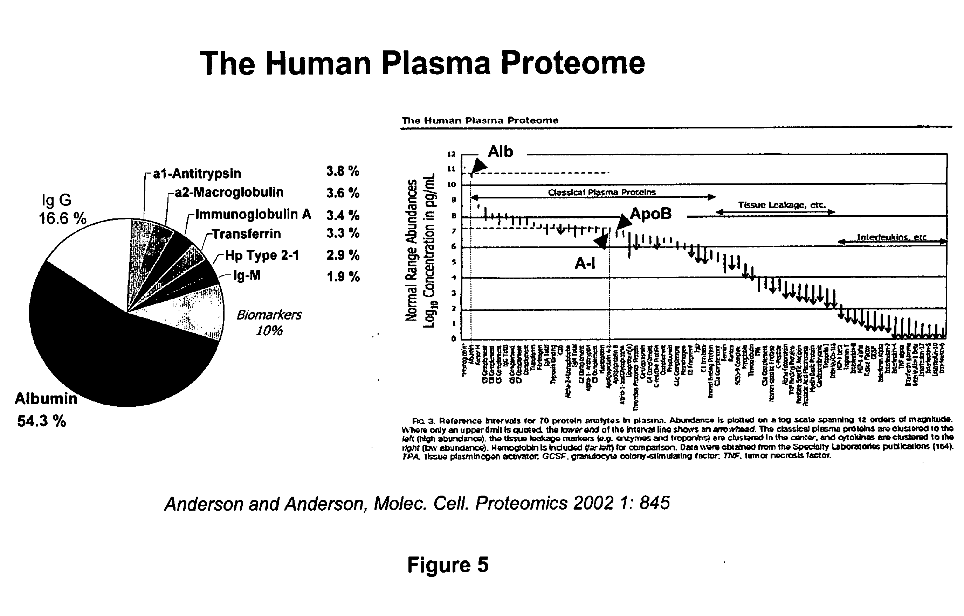

Figure 5 shows the components of human plasma.
Figure shows *trans*-splicing into the albumin target pre-mRNA.
Figure 7 shows a schematic representation of different *trans*-splicing reactions. (a) *Trans*-splicing reactions between the target 5' splice site and PTM's 3' splice site; (b) *trans-*splicing reactions between the target 3' splice site and PTM's 5' splice site; and (c) replacement of an internal exon by a double *trans*-splicing reaction in which the PTM carries both 3' and 5' splice sites. BD, binding domain; BP, branch point sequence; PPT, polypyrimidine tract; and ss, splice sites.
Figure 8 shows the present invention applied to *trans*-splicing mediated HPV- 16 E7 single chain antibody production strategy.
Figure 9 shows a schematic illustration of mouse albumin exon 1 -HPV 16 anti- E7 scFv cDNA.
Figure 10 shows a nucleotide sequence of the trans-spliced mouse albumin-HPV 16 anti-E7 scFv mRNA.
Figure 11 shows a schematic illustration of PTM containing additional endopeptidase cleavage site. The PTM structure is similar to scFv PTM except that it has an additional endopeptidase cleavage site or a native "Pro"-peptide sequence.
Figure 12 shows a schematic illustration of *trans*-splicing strategy to eliminate albumin sequence in the final product. Ex1, exon 1 of albumin; CS, additional cleavage site.
Figure 13 shows a SDS gel showing the production of HPV16 anti-E7 scFv in Hepa1-6 cells. Mouse albumin-HPV16 anti-E7 scFv cDNA (identical to the trans-spliced mRNA) was transfected into Hepal-6 and Cos-7 cells. 48 hrs post-transfection, supernatant and cell lysate was prepared and analyzed by Western blot using anti-FLAG M2 monoclonal antibody. Arrows indicate the expected ∼30 kDa mouse albumin - HPV 16 anti-E7 scFv.
Figure 14 shows the trans-spliced mAlb-HPV 16 anti-E7 scFv function in cells. HPV-positive cervical cancer cells, SiHa, or the matching HPV-negative cells were transfected with mAlb-HPV16 anti-E7 scfv expression cDNA plasmid. Cells were grown for 5 days and assayed for cell survival using MTT assay.

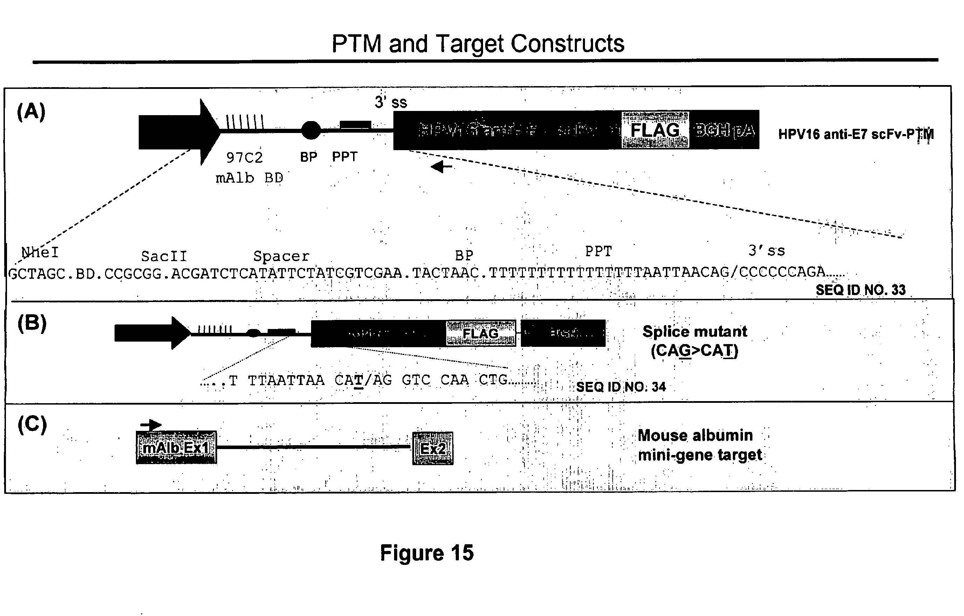

Figure 15 shows a schematic of HPV16 anti-E7 scFv PTM (A), splice mutant (B) and mouse albumin mini-gene target (C), used for *in vitro* POP studies. PTM cassette consists of a trans-splicing domain which includes mouse albumin intron 1 specific binding domain (BD), short spacer, consensus sequence branch point (BP), optimized polypyrimidne tract (PPT), 3' acceptor site (CAG) followed by the majority of the coding sequence of HPV16 anti-E7 scFv sequence. PTM Expression is driven by CMV promoter. At the 3' end, the PTM also it contains FLAG epitope followed by bovine growth hormone polyadenylation signal (BGH pA). Splice mutant is identical to the functional PTM but has a point mutation at the acceptor site (CAG>CAT). ss, 3' splice site; arrows indicate primers used for trans-splicing assays.
Figure 16 shows the precise *trans*-splicing of HPV16 anti-E7 scFv PTM into mouse albumin exon 1 in cells.
Figure 17A shows Western blot analysis of serum samples from mice injected with mAlb-HPV 16 anti-E7 scFv cDNA. 25 µl serum was passed through FLAG affinity column and analyzed by Western blot using anti-FLAG M2 monoclonal antibody.
Figure 17B shows Western blot analysis of serum from mice injected with HPV16 anti-E7 scFv PTM only. 50-100 µl serum was passed through FLAG affinity column and analyzed by Western blot using anti-FLAG M2 monoclonal antibody.
Figure 17C shows Western blot analysis of serum from mice injected with HPV16 anti-E7 scFv PTM + target. 50-100 µl serum was passed through FLAG affinity column and analyzed by Western blot using anti-FLAG M2 monoclonal antibody.
Figure 18 shows a schematic representation of a bicistronic PTM for the production of whole antibodies, the PTM cassette consists of a *trans*-splice domain (TSD) including: binding domain, short spacer, BP, PPT, coding sequence for the entire light chain, 2A self-processing peptide from the foot and mouth disease virus (FMDV) or the encephlomayocardities (ECMV) internal ribosome entry site (IRES) followed by the full length coding sequence of heavy chain. Abbreviations: BD, binding domain; BP, branch point; PPT, polypyrimidine tract; 3'ss, splice site.
Figure 19 Schematic of targeted trans-splicing of human ApoA-1 into albumin target pre-mRNA.
Figure 20 Schematic of human and mouse albumin-human Apo A-I cDNA constructs (test constructs), similar constructs with point (deletion) mutants (negative controls) and wild type human Apo A-I and milano variants (positive controls). Indicates point mutation (deletion) that result in premature termination. No full-length protein was detected on Western blot.
Figure 21 SDS gels showing human Apo A-1 expression in 293 cells.
Figure 22 Western blot showing the expression and secretion of mature human Apo A-I protein in 293 cells. Lane 1, mouse Alb-hAI; lane 2, human Alb-hAI; lane 3, wt Apo A-I and lane 4, milano variant. Upper panel, protein in supernatant and lower panel, protein in cell lysate.
Figure 23 Cholesterol efflux in 293 cells demonstrating the expression of functional human Apo A-1 protein.
Figure 24A Schematic of FACS-based PTM selection strategy.
Figure 24B Comparison of high capacity screening (HCS) protocols.
Figure 25 5'GFP-Albln1Ex2 Pre-mRNA Target Sequence. Nucleotide sequence of 5' GFP-Albln1Ex2 gene for in vitro studies. Sequences shown in italics indicate first half of the coding sequence for GFP fluorescent protein followed by human albumin intron 1 and exon 2 sequences (underlined). "/" indicates 5' and 3' splice junctions.
Figure 26 schematic diagram of the pre-mRNA target used in the HCS (SD, splice donor site; SA, splice acceptor site. Dotted lines indicate target cis-splicing).
Figure 27 Schematic illustration of the PTM cassette used in the HCS. PTM cassette consists of a *trans*-splice domain including (TSD): variable BDs, short spacer, BP, PPT, 3' half of the coding sequence for zsG, IRES followed by the full length coding sequence for second reporter DsRedExpress. Abbreviations: 3'zsG, 3' half of the zsGreen fluorescent protein coding sequence; IRES, internal ribosome entry site, BD, binding domain; BP, branch point; PPT, polypyrimidine tract. SA, splice acceptor site.
Figure 28 is a PCR analysis showing the cloning efficiency and diversity of the mouse albumin binding domain (BD) library.
Figure 29 illustrates the high capacity screening (HCS) method.
Figure 30 *Trans*-splicing efficiency of PTMs selected from HCS for mouse albumin target.
Figure 31 Bar graph showing trans-splicing efficiency and GFP fluorescence of various PTMs selected from HCS.
Figure 32 Schematic showing the human Apo A-1 PTM expression cassette used for proof of principle in vitro studies.
Figure 33 shows a schematic diagram of the mouse albumin mini-gene pre-mRNA target.
Figure 34 shows the nucleotide and amino acid sequence of wild type ApoA-1.
Figure 35 shows trans-splicing of mAlbPTMs into albumin exon 1 in stable cells.
Figure 36 Western blot analysis of trans-spliced human Apo A-1 protein.
Figure 37 PTM-mediated trans-splicing into endogenous albumin exon 1 in mice.
Figure 38 shows a schematic diagram showing a human albumin targeting strategy to increase ApoA1 expression.
Figure 39 shows the strategies to eliminate albumin sequence in the final *trans*-spliced product.
Figure 40 shows albumin-human Apo A-I cDNA, trans-spliced mRNA, old and new PTM and targets that may be used according to the present invention (NCE, non-coding exon; hAI, human Apo A-I and Ex, exon).
Figure 41 shows a schematic illustration of *trans*-splicing strategy to eliminate albumin sequence in the final product. Ex1, exon 1 of albumin; CS, additional cleavage site.
Figure 42 shows western analysis of mouse and human Alb-hApoA1 *trans-*spliced protein.
Figure 43 shows the identification of ApoA-I by Western blot analysis.
Figure 44 shows the functionality of ApoA-I as determined by cholesterol efflux assays.
Figure 45 demonstrates the use of plasmid-based delivery system for long-term production of human HDL in mice.
Figure 46 shows the generation of secreted FVIII through trans-splicing to exon 1 of albumin.
Figure 47 shows that uninoculated controls as well as animals that received a PTM that were defective in splicing showed no Factor VIII.
Figure 48 shows that animals inoculated with both albumin minigene target and the PTM encoding Factor VIII demonstrated significant plasma levels of Factor VIII.
Figure 49 shows the utilization of a single chain monoclonal antibody directed against E7 of human papillomavirus (HPV).
Figure 50 demonstrates that *in vivo trans*-spliced product was effective in inhibiting the growth of HPV-infected cells.
Figure 51 demonstrates the validity of *trans*-splicing into a highly abundant transcript.
Figure 52 shows that the PTM encoding the single chain monoclonal antibody *trans*-splices specifically into the precise nucleotide sequence in mouse albumin pre-mRNA.
Figure 53 Schematic drawings of mouse albumin-human apoAI (mAlb-hapoAI) cDNA, trans-spliced mRNA, old and new PTM and targets. NCE, non-coding exon; hAI, human apoAI and Ex, exon.
Figure 54 *Trans*-splicing between target and PTM plasmids produces functional protein in 293 cells. 293 cells transfected with different concentrations of mAlb-hapoAI cDNA or PTM + target plasmids. 48 hrs post-transfection, media was collected, processed and assayed (efflux potential) for activity as described before.
Figure 55 *Trans*-splicing efficiency of the new and old PTMs in 293 cells. 293 cells transfected with different concentrations of PTM + target plasmids. 48 hrs post-transfection, total RNA isolated and trans-splicing efficiency was quantified by qRT-PCR using specific primers.
Figure 56A RT-PCR results showing the presence of mouse mAlb-hapoAI mRNA.
Figure 56B RT-PCR results showing the presence of *trans*-spliced mRNA in mice.
Figure 56C RT-PCR results showing *trans*-splicing of human apoAI PTM into endogenous mouse albumin pre-mRNA in mice. MC, minicircles, PL, plasmid DNA; RT, reverse transcription and +/- indicate RT+ and RT- reactions.
Figure 57A Western blot analysis of serum samples from mice injected with mAlb-hapoAI cDNA. 20 µl serum passed through Proto-Blue column (to deplete albumin + IgG) and analyzed by Western blot using human apoAI specific antibody. MC, minicircles and PL, plasmid DNA RT.
Figure 57B Western blot analysis of serum samples from mice injected with PTM only and PTM + Target plasmids. 20-50 µl serum passed through Proto-Blue column (to deplete albumin + IgG) and analyzed by Western blot using human apoAI specific antibody. MC, minicircles and PL, plasmid DNA.
Figure 58A Western blot analysis of serum samples from mice injected with PTM plasmid. 50 µl serum was immunoprecipitated and analyzed by Western blot using human apoAI specific antibody. Arrows indicate 28 kDa human apoAI protein.
Figure 58B Western blot analysis of serum samples from mice injected with cDNA plasmid. 10 µl serum was immunoprecipitated and analyzed by Western blot using human apoAI specific antibody.
Figure 59 HDL analysis of serum samples from mice injected with PTM and mAlb-hapoAI DNA plasmids.

### DETAILED DESCRIPTION OF THE INVENTION

The present invention relates to novel compositions comprising pre-trans-splicing molecules (PTMs), designed for spliceosome mediated trans-splicing, and the use of such molecules for generating a novel chimeric RNA molecule comprising sequences encoding a therapeutic protein or polypeptide of interest. The therapeutic protein or polypeptide of interest is not apoliprotein A1 or a variant thereof. The PTMs are used for large-scale production of the protein or polypeptide of interest. The methods of the present invention provide for the large-scale production of therapeutic protein or polypeptide of interest. In additional embodiments, the present invention may be used to produce a therapeutic protein or polypeptide of interest *in vitro*, for example by producing the chimeric RNA and translating it in cell culture.

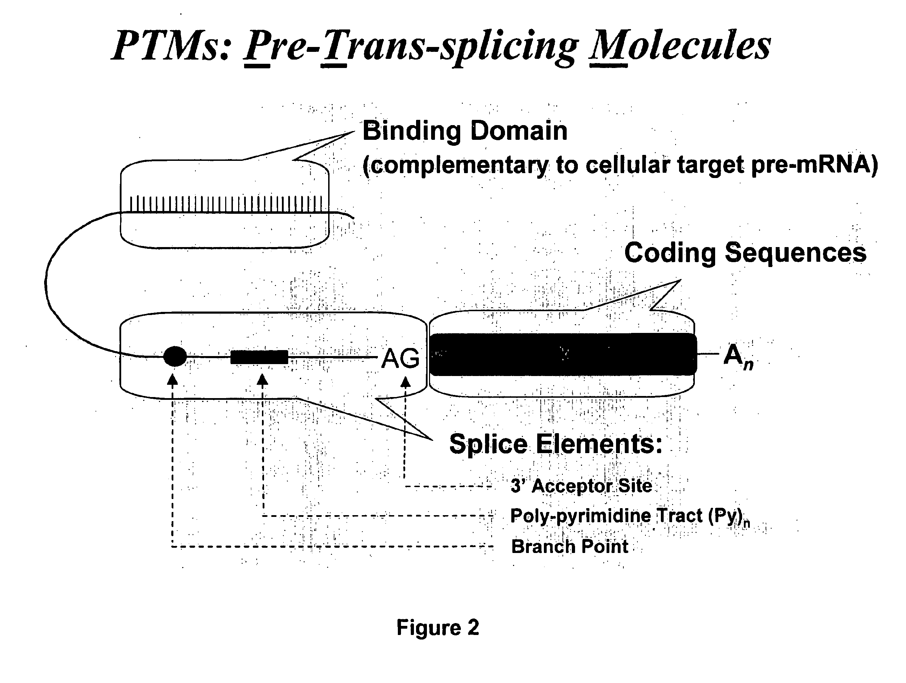

The PTMs of the invention, for use in spliceosome mediated *trans*-splicing, comprise (i) one or more target binding domains that are designed to specifically bind to albumin target pre-mRNA; (ii) either a 3' splice region that includes a 3' splice acceptor site or a 5' splice donor site; and (iii) nucleotide sequences encoding a protein or polypeptide of interest with the proviso that the protein or polypeptide is not apolipoprotein A1 or a variant thereof. The 3' splice region of a PTM will further comprise a branchpoint and a pyrimidine tract. One or more spacer regions will separate the splice sites from the target-binding domain. (See Figure 2).

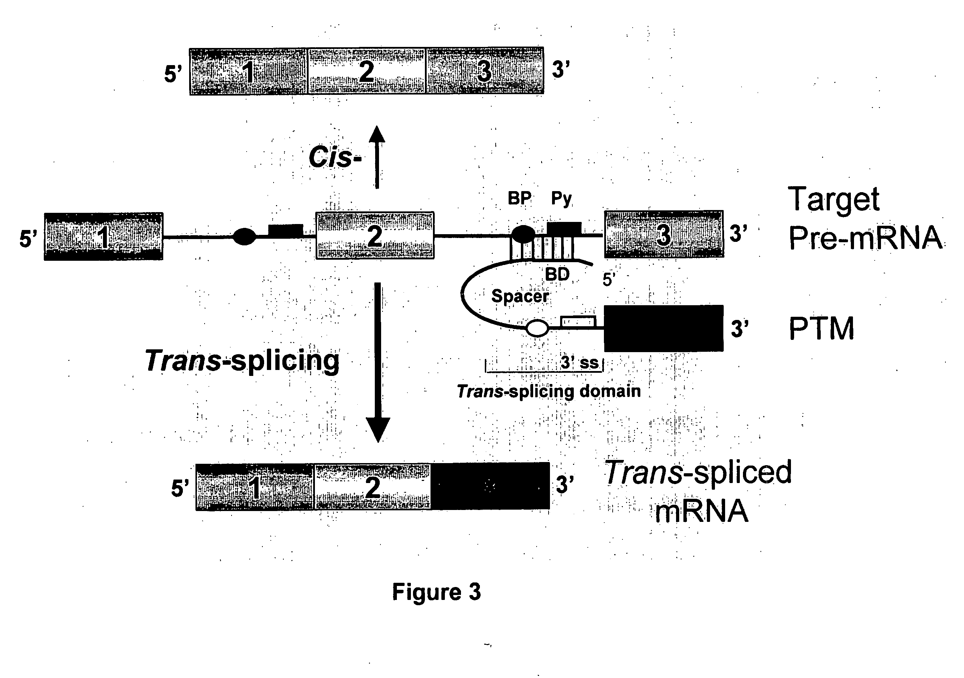

The methods of the invention encompass contacting the PTMs of the invention with an abundantly expressed target pre-mRNA, more specifically albumin pre-MRNA, under conditions in which a portion of the PTM is *trans*-spliced to a portion of the target pre-mRNA to form a novel chimeric RNA molecule comprising sequences encoding the protein or polypeptide of interest. (See Figure 3).

Albumin pre-MRNA was selected as the primary target because it is a highly expressed pre-mRNA.

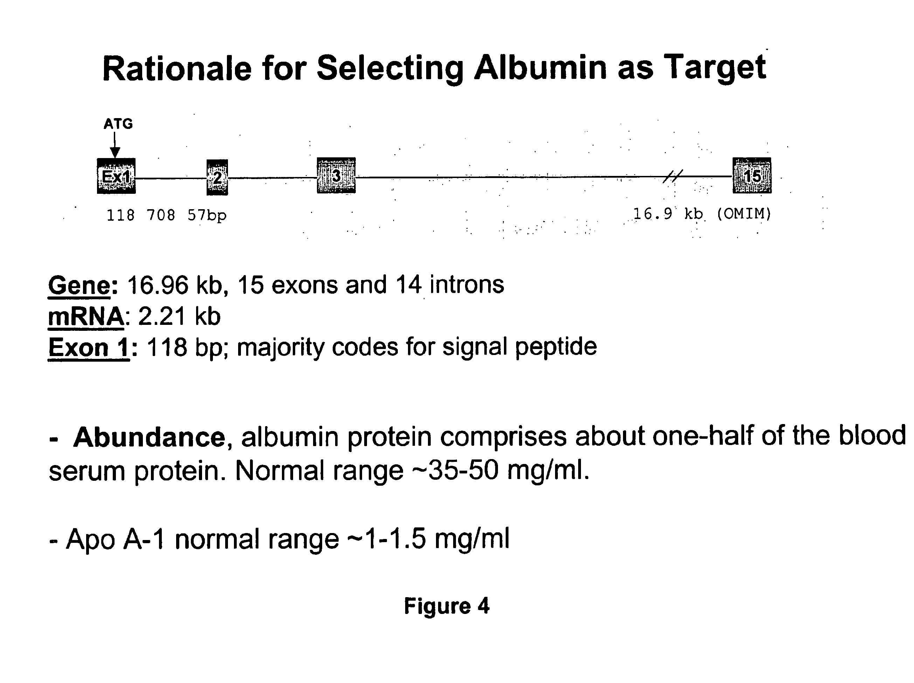

Albumin pre-mRNA was chosen because serum concentration of albumin is sufficiently high, i.e. in the range of between 45-50 mg/ml. (See Figure 4). For example, *trans*-splicing antibody sequences into albumin pre-mRNA will result in high concentrations of expressed immunoglobulin molecules into the blood. Even a moderate 5% conversion of albumin pre-mRNA target will result in the production of significantly high antibody concentration, i.e., therapeutic concentration in the blood.

The invention provides for methods of large scale bulk productions of a therapeutic protein or polypeptide of interest comprising administering to a mammal the PTMs of the present invention, contacting the PTM with the target albumin pre-mRNA under conditions in which a portion of the PTM is trans-spliced to a portion of the target pre-mRNA to form a chimeric RNA molecule that expresses the protein or polypeptide of interest, collecting the bodily fluid from the mammal, and treating the fluid to obtain the protein or polypeptide of interest.

### STRUCTURE OF THE PRE-TRANS-SPLICING MOLECULES

The present invention provides compositions for use in generating novel chimeric nucleic acid molecules through targeted *trans*-splicing. The PTMs of the invention comprise (i) one or more target binding domains that targets binding of the PTM to target albumin pre-mRNA; (ii) either a 3' splice region that includes a 3' splice acceptor site or 5' splice donor site; and (iii) nucleotide sequences encoding a therapeutic protein or polypeptide of interest, with the proviso that said therapeutic protein or polypeptide is not apoliprotein A1 or a variant thereof.

The PTMs of the invention may also include at least one of the following features: (a) binding domains targeted to intron sequences in close proximity to the 3' or 5' splice signals of the target intron, (b) mini introns, and (c) ISAR (intronic splicing activator and repressor) consensus binding sites. The PTMs of the invention will comprise one or more spacer regions to separate the RNA splice site from the target binding domain.

The general design, construction and genetic engineering of PTMs and demonstration of their ability to successful mediate spliceosome mediated *trans*-splicing reactions within the cell are described in detail in U.S. Patent Nos. 6,083,702, 6,013,487 and 6,280,978, as well as United States Patent Application Serial Nos. 09/756,095, 09/756,096, 09/756,097, 09/838,858, 10/076,248 and 09/941,492.

The target binding domain of the PTM endows the PTM with a binding affinity for the target albumin pre-mRNA. As used herein, a target binding domain is defined as any molecule, i.e., nucleotide, protein, chemical compound, etc., that confers specificity of binding and anchors the target pre-mRNA closely in space to the PTM so that the spliceosome processing machinery of the nucleus can *trans*-splice a portion of the synthetic PTM to a portion of the albumin pre-mRNA.

The target binding domain of the PTM may contain multiple binding domains that are complementary to and in anti-sense orientation to the targeted region of target pre-mRNA. The target binding domains may comprise up to several thousand nucleotides. In preferred embodiments of the invention the binding domains may comprise at least 10 to 30 and up to several hundred or more nucleotides. The specificity of the PTM may be increased significantly by increasing the length of the target binding domain. For example, the target binding domain may comprise several hundred nucleotides or more. Absolute complementarity, although preferred, is not required. A sequence "complementary" to a portion of an RNA, as referred to herein, means a sequence having sufficient complementarity to be able to hybridize with the target pre-mRNA, forming a stable duplex. The ability to hybridize will depend on both the degree of complementarity and the length of the nucleic acid (See, for example, Sambrook et al, 1989, Molecular Cloning, A Laboratory Manual, 2d Ed., Cold Spring Harbor Laboratory Press, Cold Spring Harbor, New York). Generally, the longer the hybridizing nucleic acid, the more base mismatches with an RNA it may contain and still form a stable duplex. One skilled in the art can ascertain a tolerable degree of mismatch or length of duplex by use of standard procedures to determine the stability of the hybridized complex.

In a specific embodiment, the binding domain of the PTM targets specific sequences in the target pre-mRNA so as to reduce the number of sequences derived from the target pre-mRNA. For example, targeting intron sequences of the target pre-mRNA minimizes the number of sequences derived from the target pre-mRNA in the chimeric RNA molecule.

Binding may also be achieved through other mechanisms, for example, through triple helix formation, aptamer interactions, antibody interactions or protein/nucleic acid interactions such as those in which the PTM is engineered to recognize a specific RNA binding protein, i.e., a protein bound to a specific target pre-mRNA.

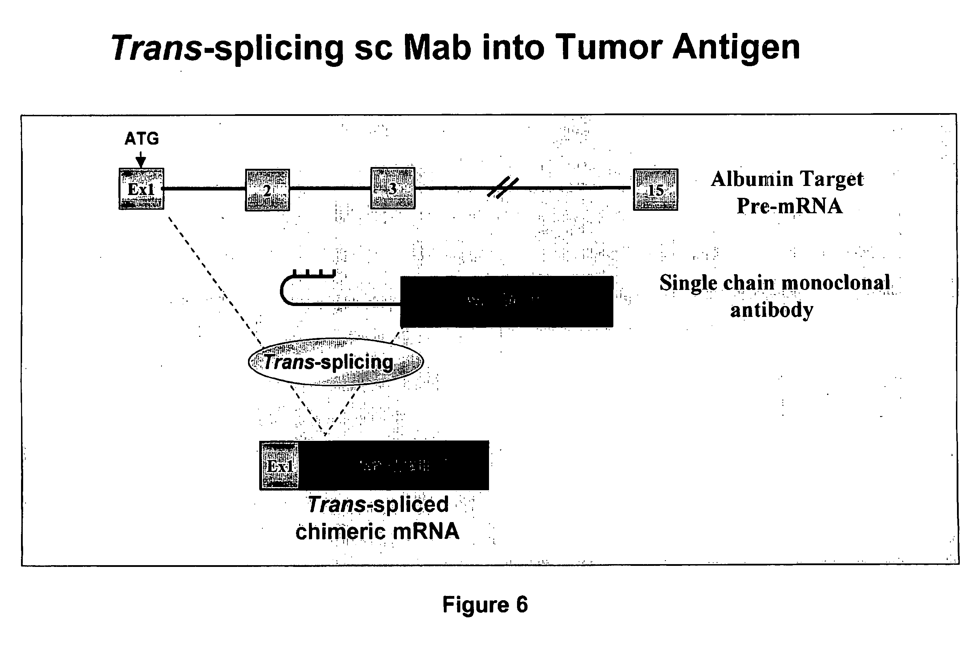

In a specific embodiment of the invention, the target binding domain is complementary and in anti-sense orientation to sequences of the albumin target pre-mRNA, which hold the PTM in close proximity to the target for *trans*-splicing. For example, a target binding domain may be defined as any molecule, i.e., nucleotide, protein, chemical compound, etc., that confers specificity of binding and anchors the albumin pre-mRNA closely in space to the PTM so that the spliceosome processing machinery of the nucleus can *trans*-splice a portion of the PTM to a portion of the albumin pre-mRNA. (See, e.g. Figure 6).

The PTM molecule also contains either a 3' splice region that includes a 3' splice acceptor AG site or a 5' splice donor site. The 3' splice region will further comprise a branchpoint and a polypyrimidine tract. Consensus sequences for the 5' splice donor site and the 3' splice region used in RNA splicing are well known in the art (See, Moore, et al., 1993, The RNA World, Cold Spring Harbor Laboratory press, p. 303-358). In addition, modified consensus sequences that maintain the ability to function as 5' donor splice sites and 3' splice regions may be used in the practice of the invention. Briefly, the 5' splice site consensus sequence is AG/GURAGU (where A=adenosine, U=uracil, G=guanine, C=cytosine, R=purine and /=the splice site) (SEQ ID NO.:1). The 3' splice site consists of three separate sequence elements: the branchpoint or branch site, a polypyrimidine tract and the 3' consensus sequence (YAG). The branch point consensus sequence in mammals is YNYURAC (Y=pyrimidine;N=any nucleotide) (SEQ ID NO.:2). The underlined A is the site of branch formation. A polypyrimidine tract is located between the branch point and the splice site acceptor and is important for different branch point utilization and 3' splice site recognition. Recently, pre-mRNA introns beginning with the dinucleotide AU and ending with the dinucleotide AC have been identified and referred to as U12 introns. U12 intron sequences, as well as any sequences that function as splice acceptor/donor sequences, may also be used to generate the PTMs of the invention.

One or more spacer region(s) to separate the RNA splice site from the target binding domain are also included in the PTM. The spacer region may be designed to include features, such as (i) stop codons which would function to block translation of any unspliced PTM and/or (ii) sequences that enhance *trans*-splicing to the target pre-mRNA.

A nucleotide sequence encoding a therapeutic protein or polypeptide of interest is also included in the PTM of the invention. The PTMs of the invention contain exon sequences which when *trans*-spliced to the target pre-mRNA will result in the formation of a chimeric RNA capable of encoding a functional protein or polypeptide of interest. The PTM may be engineered to contain the exon sequences of various recombinant proteins or polypeptides. The proteins or polypeptides of interest may be selected from the group consisting of cytokines, growth factors, such as epogen, insulin, human growth factor, hormones, enzymes and antibody polypeptides. In addition, the encoded protein or polypeptide of interest may be a therapeutic protein that is expensive to produce commercially. The protein or polypeptide of interest is not apolipoprotrein A1 or a variant thereof.

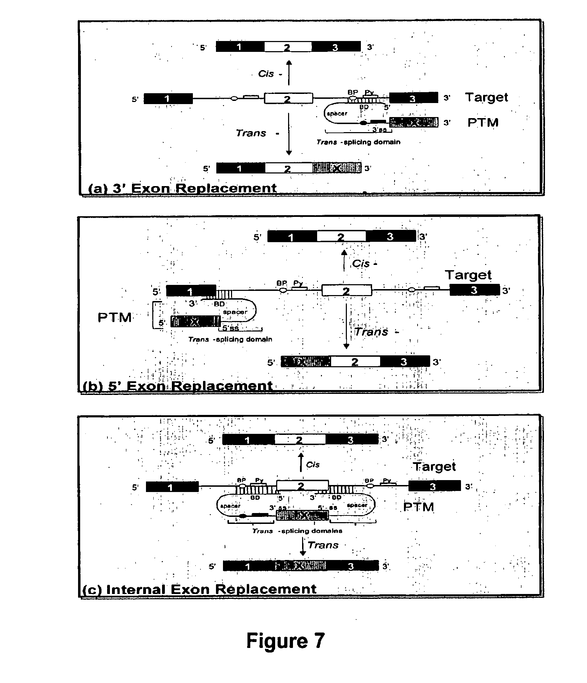

The PTMs of the invention may be engineered to contain a single exon sequence, multiple exon sequences, or alternatively the complete set of exon sequences encoding the antigen of interest. The number and identity of the sequences to be used in the PTMs will depend on the type of *trans*-splicing reaction, i.e., 5' exon replacement, 3' exon replacement or internal exon replacement that will occur (See Figure 7).

In an embodiment of the invention, a "safety" is also incorporated into the spacer, binding domain, or elsewhere in the PTM to prevent non-specific *trans*-splicing. This is a region of the PTM that covers elements of the 3' and/or 5' splice site of the PTM by relatively weak complementarity, preventing non-specific *trans*-splicing. The PTM is designed in such a way that upon hybridization of the binding/targeting portion(s) of the PTM, the 3' and/or 5 'splice site is uncovered and becomes fully active.

Such "safety" sequences comprises one or more complementary stretches of cis-sequence (or could be a second, separate, strand of nucleic acid) which binds to one or both sides of the PTM branch point, pyrimidine tract, 3' splice site and/or 5' splice site (splicing elements), or could bind to parts of the splicing elements themselves. This "safety" binding prevents the splicing elements from being active (i.e. block U2 snRNP or other splicing factors from attaching to the PTM splice site recognition elements). The binding of the "safety" may be disrupted by the binding of the target binding region of the PTM to target pre-mRNA, thus exposing and activating the PTM splicing elements (making them available to trans -splice into target pre-mRNA).

A nucleotide sequence capable of forming a stem-loop structure may also be included in the PTM of the invention.

The present invention further provides PTM molecules wherein the coding region of the PTM is engineered to contain mini-introns. The insertion of mini-introns into the coding sequence of the PTM is designed to increase definition of the exon and enhance recognition of the PTM donor site. Mini-intron sequences to be inserted into the coding regions of the PTM include small naturally occurring introns or, alternatively, any intron sequences, including synthetic mini-introns, which include 5' consensus donor sites and 3' consensus sequences which include a branch point, a 3' splice site and in some instances a pyrimidine tract.

The mini-intron sequences are preferably between about 60-150 nucleotides in length, however, mini-intron sequences of increased lengths may also be used. In a preferred embodiment of the invention, the mini-intron comprises the 5' and 3' end of an endogenous intron. In preferred embodiments of the invention the 5' intron fragment is about 20 nucleotides in length and the 3' end is about 40 nucleotides in length.

In a specific embodiment of the invention, an intron of 528 nucleotides comprising the following sequences may be utilized. Sequence of the intron construct is as follows:
5' fragment sequence: (SEQ ID NO:3) 
Gtagttcttttgttcttcactattaagaacttaatttggtgtccatgtctcttttttttctagtttgtagtgctggaaggtat
ttttggagaaattcttacatgagcattaggagaatgtatgggtgtagtgtcttgtataatagaaattgttccactgataatttactctagtttttta
tttcctcatattattttcagtggctttttcttccacatctttatattttgcaccacattcaacactgtagcggccgc.
3' fragment sequence: (SEQ ID NO:4)

Ccaactatctgaatcatgtgccccttctctgtgaacctctatcataatacttgtcacactgtattgtaattgtctctttta
ctttccccttgtatcttttgtgcatagcagagtacctgaaacaggaagtattttaaatattttgaatcaaatgagttaatagaatctttacaaataa
gaatatacacttctgcttaggatgataattggaggcaagtgaatcctgagcgtgatttgataatgacctaataatgatgggttttatttccag

In yet another specific embodiment of the invention, consensus ISAR sequences are included in the PTMs of the invention (Jones et al., NAR 29:3557-3565). Proteins bind to the ISAR splicing activator and repressor consensus sequence which includes a uridine-rich region that is required for 5' splice site recognition by U1 SnRNP. The 18 nucleotide ISAR consensus sequence comprises the following sequence:
GGGCUGAUUUUUCCAUGU (SEQ ID NO:5). When inserted into the PTMs of the invention, the ISAR consensus sequences are inserted into the structure of the PTM in close proximity to the 5' donor site ofintron sequences. In an embodiment of the invention the ISAR sequences are inserted within 100 nucleotides from the 5' donor site. In a preferred embodiment of the invention, the ISAR sequences are inserted within 50 nucleotides from the 5' donor site. In a more preferred embodiment of the invention the ISAR sequences are inserted within 20 nucleotides of the 5' donor site.

The compositions of the invention further comprise PTMs that have been engineered to include cis-acting ribozyme sequences. The inclusion of such sequences is designed to reduce PTM translation in the absence of *trans*-splicing or to produce a PTM with a specific length or defined end(s). The ribozyme sequences that may be inserted into the PTMs include any sequences that are capable of mediating a *cis*-acting (self-cleaving) RNA splicing reaction. Such ribozymes include but are not limited to hammerhead, hairpin and hepatitis delta virus ribozymes (see, Chow et al. 1994, J Biol Chem 269:25856-64).

In an embodiment of the invention, splicing enhancers such as, for example, sequences referred to as exonic splicing enhancers may also be included in the structure of the synthetic PTMs. Transacting splicing factors, namely the serine/arginine-rich (SR) proteins, have been shown to interact with such exonic splicing enhancers and modulate splicing (*See*, Tacke et al., 1999, Curr. Opin. Cell Biol. 11:358-362; Tian et al., 2001, J. Biological Chemistry 276:33833-33839; Fu, 1995, RNA 1:663-680). Nuclear localization signals may also be included in the PTM molecule (Dingwell and Laskey, 1986, Ann .Rev. Cell Biol. 2:367-390; Dingwell and Laskey, 1991, Trends in Biochem. Sci. 16:478-481). Such nuclear localization signals can be used to enhance the transport of synthetic PTMs into the nucleus where *trans*-splicing occurs.

Additional features can be added to the PTM molecule, such as polyadenylation signals to modify RNA expression/stability, or 5' splice sequences to enhance splicing, additional binding regions, "safety"-self complementary regions, additional splice sites, or protective groups to modulate the stability of the molecule and prevent degradation. Stop codons may be included in the PTM structure to prevent translation of unspliced PTMs. Elements, such as a 3' hairpin structure, circularized RNA, nucleotide base modification, or synthetic analogs, can be incorporated into PTMs to promote or facilitate nuclear localization and spliceosomal incorporation, and intracellular stability.

To facilitate collection of the protein or polypeptide of interest that is produced as a result of *trans*-splicing, sequence tags may be incorporated into the PTMs. The tags promote affinity purification of the expressed protein or polypeptide of interest product of the chimeric RNA molecule.

The PTMs may be further engineered to incorporate cleavage sites to facilitate isolation of the mature biologically active protein or polypeptide of interest from the expressed protein product of the chimeric RNA molecule.

Secretory signaling sequences could be incorporated to increase secretion of the expressed protein or polypeptide of interest.

When specific PTMs are to be synthesized *in vitro* (synthetic PTMs), such PTMs can be modified at the base moiety, sugar moiety, or phosphate backbone, for example, to improve stability of the molecule, hybridization to the target mRNA, transport into the cell, etc. For example, modification of a PTM to reduce the overall charge can enhance the cellular uptake of the molecule. In addition, modifications can be made to reduce susceptibility to nuclease or chemical degradation. The nucleic acid molecules may be synthesized in such a way as to be conjugated to another molecule such as a peptides (e.g., for targeting host cell receptors *in vivo*), or an agent facilitating transport across the cell membrane (see, e.g., Letsinger et al, 1989, Proc. Natl. Acad. Sci. USA 86:6553-6556; Lemaitre et al., 1987, Proc. Natl. Acad. Sci. 84:648-652; PCT Publication No. W088/09810, published December 15, 1988) or the blood-brain barrier (see, e.g., PCT Publication No. W089/10134, published April 25, 1988), hybridization-triggered cleavage agents (see, e.g., Krol et al., 1988, BioTechniques 6:958-976) or intercalating agents (see, e.g., Zon, 1988, Pharm. Res. 5:539-549). To this end, the nucleic acid molecules may be conjugated to another molecule, e.g., a peptide, hybridization triggered cross-linking agent, transport agent, hybridization-triggered cleavage agent, etc.

It may be necessary in certain cases to cleave amino acids originating from albumin to obtain the purified biologically active protein of interest. This can be achieved by standard methods such as engineering a protease site in the junction between portions originating from albumin and the recombinant protein of interest. One example is the TEV protease. Another method is to tag peptides with, for example, calmodulin binding domain. These methods have been widely used to obtain only those amino acids in the protein of interest and no amino acids derived from albumin or other target.

Various other well-known modifications to the nucleic acid molecules can be introduced as a means of increasing intracellular stability and half-life. Possible modifications include, but are not limited to, the addition of flanking sequences of ribonucleotides to the 5' and/or 3' ends of the molecule. In some circumstances where increased stability is desired, nucleic acids having modified internucleoside linkages such as 2'-0-methylation may be preferred. Nucleic acids containing modified internucleoside linkages may be synthesized using reagents and methods that are well known in the art (see, Uhlmann et al., 1990, Chem. Rev. 90:543-584; Schneider et al., 1990, Tetrahedron Lett. 31:335 and references cited therein).

The PTMs of the present invention are preferably modified in such a way as to increase their stability in the cells. Since RNA molecules are sensitive to cleavage by cellular ribonucleases, it may be preferable to use as the competitive inhibitor a chemically modified oligonucleotide (or combination of oligonucleotides) that mimics the action of the RNA binding sequence but is less sensitive to nuclease cleavage. In addition, the synthetic PTMs can be produced as nuclease resistant circular molecules with enhanced stability to prevent degradation by nucleases (Puttaraju et al., 1995, Nucleic Acids Symposium Series No. 33:49-51; Puttaraju et al., 1993, Nucleic Acid Research 21:4253-4258). Other modifications may also be required, for example to enhance binding, to enhance cellular uptake, to improve kinetics or to improve other desirable characteristics.

Modifications, which may be made to the structure of the synthetic PTMs include but are not limited to backbone modifications such as use of:
(i) phosphorothioates (X or Y or W or Z=S or any combination of two or more with the remainder as O). *e.g.* Y=S (Stein, C. A., et al., 1988, Nucleic Acids Res., 16:3209-3221), X=S (Cosstick, R., et al., 1989, Tetrahedron Letters, 30, 4693-4696), Y and Z=S (Brill, W. K.-D., et al., 1989, J. Amer. Chem. Soc., 111:2321-2322);
(ii) methylphosphonates (*e.g*. Z=methyl (Miller, P. S., et al., 1980, J. Biol. Chem., 255:9659-9665); (iii) phosphoramidates (Z=N-(alkyl)₂ *e.g*. alkyl methyl, ethyl, butyl) (Z=morpholine or piperazine) (Agrawal, S., et al., 1988, Proc. Natl. Acad. Sci. USA 85:7079-7083) (X or W=NH) (Mag, M., et al., 1988, Nucleic Acids Res., 16:3525-3543); (iv) phosphotriesters (Z=O-alkyl *e.g*. methyl, ethyl, *etc*) (Miller, P. S., et al., 1982, Biochemistry, 21:5468-5474); and (v) phosphorus-free linkages (*e.g*. carbamate, acetamidate, acetate) (Gait, M. J., et al., 1974, J. Chem. Soc. Perkin 1, 1684-1686; Gait, M. J., et al., 1979, J. Chem. Soc. Perkin I, 1389-1394).

In addition, sugar modifications may be incorporated into the PTMs of the invention. Such modifications include the use of: (i) 2'-ribonucleosides (R=H); (ii) 2'-O-methylated nucleosides (R=OMe) ) (Sproat, B. S., et al., 1989, Nucleic Acids Res., 17:3373-3386); and (iii) 2'-fluoro-2'-riboxynucleosides (R=F) (Krug, A., et al., 1989, Nucleosides and Nucleotides, 8:1473-1483).

Further, base modifications that may be made to the PTMs, including but not limited to use of: (i) pyrimidine derivatives substituted in the 5-position (e.g. methyl, bromo, fluoro etc) or replacing a carbonyl group by an amino group (Piccirilli, J. A., et al., 1990, Nature, 343:33-37); (ii) purine derivatives lacking specific nitrogen atoms (*e.g*., 7-deaza adenine, hypoxanthine) or functionalized in the 8-position (*e.g*., 8-azido adenine, 8-bromo adenine) (for a review see Jones, A. S., 1979, Int. J. Biolog. Macromolecules, 1:194-207).

In addition, the PTMs may be covalently linked to reactive functional groups, such as: (i) psoralens (Miller, P. S., et al., 1988, Nucleic Acids Res., Special Pub. No. 20, 113-114), phenanthrolines (Sun, J-S., et al., 1988, Biochemistry, 27:6039-6045), mustards (Vlassov, V. V., et al., 1988, Gene, 72:313-322) (irreversible cross-linking agents with or without the need for co-reagents); (ii) acridine (intercalating agents) (Helene, C., et al., 1985, Biochimie, 67:777-783); (iii) thiol derivatives (reversible disulphide formation with proteins) (Connolly, B. A., and Newman, P. C., 1989, Nucleic Acids Res., 17:4957-4974); (iv) aldehydes (Schiff's base formation); (v) azido, bromo groups (UV cross-linking); or (vi) ellipticines (photolytic cross-linking) (Perrouault, L., et al., 1990, Nature, 344:358-360).

In an embodiment of the invention, oligonucleotide mimetics in which the sugar and internucleoside linkage, *i.e*., the backbone of the nucleotide units, are replaced with novel groups can be used. For example, one such oligonucleotide mimetic which has been shown to bind with a higher affinity to DNA and RNA than natural oligonucleotides is referred to as a peptide nucleic acid (PNA) (for review see, Uhlmann, E. 1998, Biol. Chem. 379:1045-52). Thus, PNA may be incorporated into synthetic PTMs to increase their stability and/or binding affinity for the target pre-mRNA.

In another embodiment of the invention, the PTMs may be covalently linked to lipophilic groups or other reagents capable of improving uptake by cells. For example, the PTM molecules may be covalently linked to: (i) cholesterol (Letsinger, R. L., et al., 1989, Proc. Natl. Acad. Sci. USA, 86:6553-6556); (ii) polyamines (Lemaitre, M., et al., 1987, Proc. Natl. Acad. Sci, USA, 84:648-652); other soluble polymers (*e.g*. polyethylene glycol) to improve the efficiently with which the PTMs are delivered to a cell. In addition, combinations of the above identified modifications may be utilized to increase the stability and delivery of PTMs into the target cell. The PTMs of the invention can be used in methods designed to produce a novel chimeric RNA in a target cell.

The methods of the present invention comprise delivering to the target cell a PTM which may be in any form used by one skilled in the art, for example, an RNA molecule, or a DNA vector which is transcribed into a RNA molecule corresponding to the PTM of the present invention, wherein said PTM binds to target pre-mRNA and mediates a trans-splicing reaction resulting in formation of a chimeric mRNA that expresses a protein or polypeptide of interest.

### SYNTHESIS OF THE TRANS-SPLICING MOLECULES

The nucleic acid molecules of the invention can be RNA or DNA or derivatives or modified versions thereof, single-stranded or double-stranded. By nucleic acid is meant a PTM molecule, a ribozyme or t-RNA endonuclease based nucleic acid molecule, or a nucleic acid molecule encoding a PTM molecule, a ribozyme or t-RNA endonuclease based nucleic acid molecule, whether composed of deoxyribonucleotides or ribonucleosides, and whether composed of phosphodiester linkages or modified linkages. The term nucleic acid also specifically includes nucleic acids composed of bases other than the five biologically occurring bases (adenine, guanine, thymine, cytosine and uracil). In addition, the PTMs of the invention may comprise, DNA/RNA, RNA/protein or DNA/RNA/protein chimeric molecules that are designed to enhance the stability of the PTMs.

The PTMs of the invention can be prepared by any method known in the art for the synthesis of nucleic acid molecules. For example, the nucleic acids may be chemically synthesized using commercially available reagents and synthesizers by methods that are well known in the art (see, e.g., Gait, 1985, Oligonucleotide Synthesis: A Practical Approach, IRL Press, Oxford, England).

Alternatively, synthetic PTMs can be generated by *in vitro* transcription of DNA sequences encoding the PTM of interest. Such DNA sequences can be incorporated into a wide variety of vectors downstream from suitable RNA polymerase promoters such as the T7, SP6, or T3 polymerase promoters. Consensus RNA polymerase promoter sequences include the following:
T7: TAATACGACTCACTATAGGGAGA (SEQ ID NO:6)
SP6: ATTTAGGTGACACTATAGAAGNG (SEQ ID NO:7)
T3: AATTAACCCTCACTAAAGGGAGA (SEQ ID NO:8).

The base in bold is the first base incorporated into RNA during transcription. The underline indicates the minimum sequence required for efficient transcription.

RNAs may be produced in high yield via *in vitro* transcription using plasmids such as SPS65 and Bluescript (Promega Corporation, Madison, WI). In addition, RNA amplification methods such as Q-β amplification can be utilized to produce the PTM of interest.

The PTMs may be purified by any suitable means, as are well known in the art. For example, the PTMs can be purified by gel filtration, affinity or antibody interactions, reverse phase chromatography or gel electrophoresis. Of course, the skilled artisan will recognize that the method of purification will depend in part on the size, charge and shape of the nucleic acid to be purified.

The PTMs of the invention, whether synthesized chemically, *in vitro,* or *in vivo,* can be synthesized in the presence of modified or substituted nucleotides to increase stability, uptake or binding of the PTM to a target pre-mRNA. In addition, following synthesis of the PTM, the PTMs may be modified with peptides, chemical agents, antibodies, or nucleic acid molecules, for example, to enhance the physical properties of the PTM molecules. Such modifications are well known to those of skill in the art.

In instances where a nucleic acid molecule encoding a PTM is utilized, cloning techniques known in the art may be used for cloning of the nucleic acid molecule into an expression vector. Methods commonly known in the art of recombinant DNA technology I which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

The DNA encoding the PTM of interest may be recombinantly engineered into a variety of host vector systems that also provide for replication of the DNA in large scale and contain the necessary elements for directing the transcription of the PTM. The use of such a construct to transfect target cells in the patient will result in the transcription of sufficient amounts of PTMs that will form complementary base pairs with the endogenously expressed pre-mRNA targets, and thereby facilitate a *trans*-splicing reaction between the complexed nucleic acid molecules. For example, a vector can be introduced *in vivo* such that I is taken up by a cell and directs the transcription of the PTM molecule. Such a vector can remain episomal or become chromosomally integrated, as long as it can be transcribed to produce the desired RNA, *i.e*., PTM. Such vectors can be constructed by recombinant DNA technology methods standard in the art.

Vectors encoding the PTM of interest can be plasmid, viral, or others known in the art, used for replication and expression in mammalian cells. Expression of the sequence encoding the PTM can be regulated by any promoter/enhancer sequences known in the art to act in mammalian, preferably human cells. Such promoters/enhancers can be inducible or constitutive. Such promoters include but are not limited to: the SV40 early promoter region (Benoist, C. and Chambon, P. 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:14411445), the regulatory sequences of the metallothionein gene (Brinster et al., 1982, Nature 296:39-42), the viral CMV promoter, the human chorionic gonadotropin-β promoter (Hollenberg et al, 1994, Mol. Cell. Endocrinology 106:111-119), etc.

Any type ofplasmid, cosmid, YAC or viral vector can be used to prepare the recombinant DNA construct, which can be introduced directly into the tissue site. Alternatively, viral vectors can be used which selectively infect the desired target cell. Vectors for use in the practice of the invention include any eukaryotic expression vectors, including but not limited to, viral expression vectors, such as those derived from the class of retroviruses, adenoviruses or adeno-associated viruses.

A number of selection systems can also be used, including but not limited to selection for expression of the herpes simplex virus thymidine kinase, hypoxanthine-guanine phosphoribosyltransterase and adenine phosphoribosyl transferase protein in tk-, hgprt- or aprt-deficient cells, respectively. Also, anti-metabolic resistance can be used as the basis of selection for dihydrofolate transferase (dhfr), which confers resistance to methotrexate; xanthine-guanine phosphoribosyl transferase (gpt), which confers resistance to mycophenolic acid; neomycin (neo), which confers resistance to aminoglycoside G-418; and hygromycin B phosphotransferase (hygro) which confers resistance to hygromycin. In a preferred embodiment of the invention, the cell culture is transformed at a low ratio of vector to cell, such that there will be only a single vector, or a limited number of vectors, present in any one cell.

### USES AND ADMINISTRATION OF TRANS-SPLICING MOLECULES

The compositions and methods of the present invention are designed to generate novel chimeric RNA molecules containing sequences that express a therapeutic protein or polypeptide of interest. This is with the proviso that said therapeutic protein or polypeptide of interest is not apoliprotein A1 or a variant thereof. Specifically, targeted spliceosome mediated including double-*trans*-splicing reactions, 3' exon replacement and/or 5' exon replacement can be used to generate such chimeric RNAs. A chimeric RNA molecule containing sequences that express a protein or polypeptide of interest of the present invention is translated to generate a protein product comprising the protein or polypeptide of interest. Preferably, the protein or polypeptide of interest is produced in a large scale quantity in the bodily fluid of the host mammal.

Various delivery systems are known and can be used to transfer the compositions of the invention into cells, e.g. encapsulation in liposomes, microparticles, microcapsules, recombinant cells capable of expressing the composition, receptor-mediated endocytosis (see, e.g., Wu and Wu, 1987, J. Biol. Chem. 262:4429-4432), construction of a nucleic acid as part of a retroviral, adenoviral, adeno-associated viral or other vector, injection of DNA, electroporation, calcium phosphate mediated transfection, etc.

One or more PTM(s) and the delivery system would constitute the product which could be administered to mammals by conventional administration methods, such as intravenous or intraportal infection. In a specific embodiment of the invention, the chimeric RNA molecule may be distributed throughout the circulation, but would be active in hepatocytes, site of albumin synthesis. The PTM would be active in its RNA form, the binding domain of the PTM adhering to the targeted sequence in the albumin target pre-mRNA. Following *trans*-splicing, the coding domain of the PTM would be inserted or trans -spliced to a defined sequence of the target, resulting in a (chimeric mRNA that expresses a product comprising the therapeutic protein or polypeptide of interest.

The albumin gene is highly expressed in the liver, and provides abundant target pre-mRNA. By targeting albumin, the serum concentration of the expressed protein product of interest may be expressed at physiologically, biologically increased or therapeutic levels. Albumin has a serum concentration on the order of 45-50 mg/ml in large mammals, such as cows. Given a moderate *trans*-spl icing efficiency of 5%, large quantities of the expressed protein product of interest can be produced *in vivo.* Based on a plasma concentration of 45 mg/ml of albumin and a moderate *trans*-splicing efficiency of 5%, 2.5 mg/ml of the product is generated. The product, which comprises the protein or polypeptide of interest, is generally present at between 2-5 mg/ml in the serum of the animal. With higher efficiency of the splicing reaction, the yield of recombinant protein would be greater. These yields support the concept of using PTMs to generate large scale amounts of recombinant proteins and polypeptides.

Delivery of the PTM into a host may be either direct, in which case the host is directly exposed to the PTM or PTM encoding nucleic acid molecule, or indirect, in which case, host cells are first transformed with the PTM or PTM encoding nucleic acid molecule *in vitro,* then transplanted into the host. These two approaches are known, respectively, as *in vivo* or ex vivo gene delivery.

In a specific embodiment, the nucleic acid is directly administered *in vivo,* where it is expressed to produce the PTM. This can be accomplished by any of numerous methods known in the art, e.g., by constructing it as part of an appropriate nucleic acid expression vector and administering it so that it becomes intracellular, e.g. by infection using a defective or attenuated retroviral or other viral vector (see U.S. Patent No. 4,980,286), or by direct injection of naked DNA, or by use of microparticle bombardment (e.g., a gene gun; Biolistic, Dupont, Bio-Rad), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see e.g., Wu and Wu, 1987, J. Biol. Chem. 262:4429-4432).

In a specific embodiment, a viral vector that contains the PTM can be used. For example, a retroviral vector can be utilized that has been modified to delete retroviral sequences that are not necessary for packaging of the viral genome and integration into host cell DNA (see Miller et al, 1993, Meth. Enzymol. 217:581-599). Retroviral vectors also include lenti viral vectors. Alternatively, adenoviral or adeno-associated viral vectors can be used for gene delivery to cells or tissues. (See, Kozarsky and Wilson, 1993, Current Opinion in Genetics and Development 3:499-503 for a review of adeno virus-based gene delivery).

In a preferred embodiment of the invention an adeno-associated viral vector may be used to deliver nucleic acid molecules capable of encoding the PTM. The vector is designed so that, depending on the level of expression desired, the promoter and/or enhancer element of choice may be inserted into the vector.

Another approach to gene delivery into a cell involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. The resulting recombinant cells can be delivered to a mammal by various methods known in the art. In a preferred embodiment, the cell used for gene delivery is autologous to the host's cell.

The present invention also provides for compositions comprising an effective amount of a PTM or a nucleic acid encoding a PTM effective for obtaining production of the protein or polypeptide of interest, and a acceptable carrier. In a specific embodiment, the carrier is a pharmaceutical carrier, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Examples of suitable pharmaceutical carriers are described in "Remington's Pharmaceutical sciences" by E.W. Martin.

Many methods standard in the art can be thus employed, including but not limited to hybridization assays to detect formation of chimeric mRNA expression by detecting and/or visualizing the presence of chimeric mRNA (*e.g*., Northern assays, dot blots, in situ hybridization, and Reverse-Transcription PCR, etc.), etc.

In a specific embodiment, the compositions of the invention may be administered locally. This may be achieved by, for example, and not by way of limitation, local infusion during surgery, topical application, *e.g*., in conjunction with a wound dressing after surgery, by injection, by means of a catheter, by means of a suppository, or by means of an implant, said implant being of a porous, non-porous, or gelatinous material, including membranes, such as sialastic membranes, or fibers. Other control release drug delivery systems, such as nanoparticles, matrices such as controlled-release polymers, and hydrogels.

The PTM will be administered in amounts which are effective to produce the desired amounts of chimeric RNA molecule containing sequences encoding recombinant proteins or polypeptides. Effective dosages of the PTMs can be determined through procedures well known to those in the art which address such parameters as biological half-life, bioavailability and toxicity. The amount of the composition of the invention can be determined by standard clinical techniques. Such techniques include analysis of samples to determine if the level of protein or polypeptide of interest has been achieved. In addition, *in vitro* assays may optionally be employed to help identify optimal dosage ranges.

The present invention also provides a pack or kit comprising one or more containers filled with one or more of the ingredients of the compositions of the invention optionally associated with such containers) can be a notice in the form prescribed by a governmental agency regulating the manufacture, use or sale of products, which notice reflects approval by the agency of manufacture for use or sale in mammals.

The present invention further provides for methods of large scale bulk production of a therapeutic protein or polypeptide of interest comprising administering to a mammal the PTMs of the present invention, contacting the PTM with the albumin target pre- mRNA under conditions in which a portion of the PTM is *trans*-spliced to a portion of the target pre-mRNA to form a chimeric RNA molecule that expresses the protein or polypeptide of interest, collecting the bodily fluid from the mammal, and treating the fluid to obtain the protein or polypeptide of interest

Various mammals are appropriate hosts for large scale bulk production. They include ruminants, such as cattle, goats, deer, sheep, giraffes, and camel. These animals have been domesticated, and produce milk and an abundance of serum. Cattle are preferred.

The therapeutic protein or polypeptide of interest can be produced in large quantities as long as its presence in the mammal does not trigger an immune response. To minimize such an effect, the mammals may be treated with various immunosuppressive techniques well known to one of skill in the art.

In an embodiment of the invention, the expressed protein product is found circulating in the serum of the mammal. Alternatively, the expressed protein product is in the milk of the mammal.

For the serum, blood is collected from the mammal and centrifuged to separate the serum from the remaining cellular components. The protein can be concentrated and purified by standard methodologies. A similar concentration/purification procedure can be used for other materials, such as milk.

Separation of the protein or polypeptide of interest can be performed using various protein purification techniques well known to one of skill in the art. See, e.g., Scopes, R. K., Protein Purification Principles and Practices, 2d ed. (Springer- Verlag, 1987), Methods in Enzymology (S. Colowick and No. Kaplan, eds., Academic Press, Inc.), Sambrook et al., Molecular Cloning: A Laboratory Manual, 2d ed., Cold Spring Harbor Press, Cold Spring Harbor, N. Y., 1989, Handbook of Experimental Immunology, Vols. HV (D. M. Weir and C. C. Blackwell, eds., 1986, Blackwell Scientific Publications); House, Modern Synthetic Reactions, 2d ed., Benjamin/Cummings, Menlo Park, Calif., 1972.

The following examples exemplify the present invention, although those examples concerning PTMs encoding human apoliprotein A1 do not form part of the invention as claimed.

### EXAMPLE I: IN VIVO TRANS-SPLICED ALBUMIN-HPV-16 ANTI-E7 SINGLE CHAIN ANTIBODY (MALB-HPV-16 ANTI-E7 SCFV) CDNA

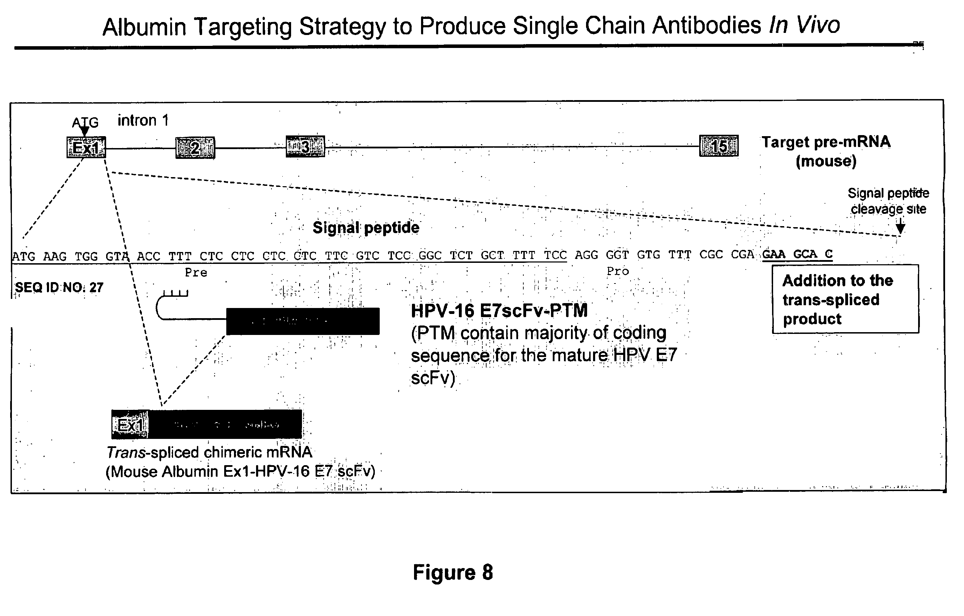

The albumin targeting strategy shown in Figure 8 has been evaluated for the production of human papilloma virus type 16 (HPV-16) anti-E7 single chain antibody *in vivo.* The concept involves targeted *trans*-splicing of HPV-16 anti-E7 scFv sequence into albumin pre-mRNA target. Albumin has been selected as a target because of its elevated expression in the liver to provide high albumin pre-mRNA concentration for abundant *trans*-splicing targets. The present study evaluated the effect of albumin sequences on expression, secretion and function of HPV-16 anti-E7 scFv *in vivo*.

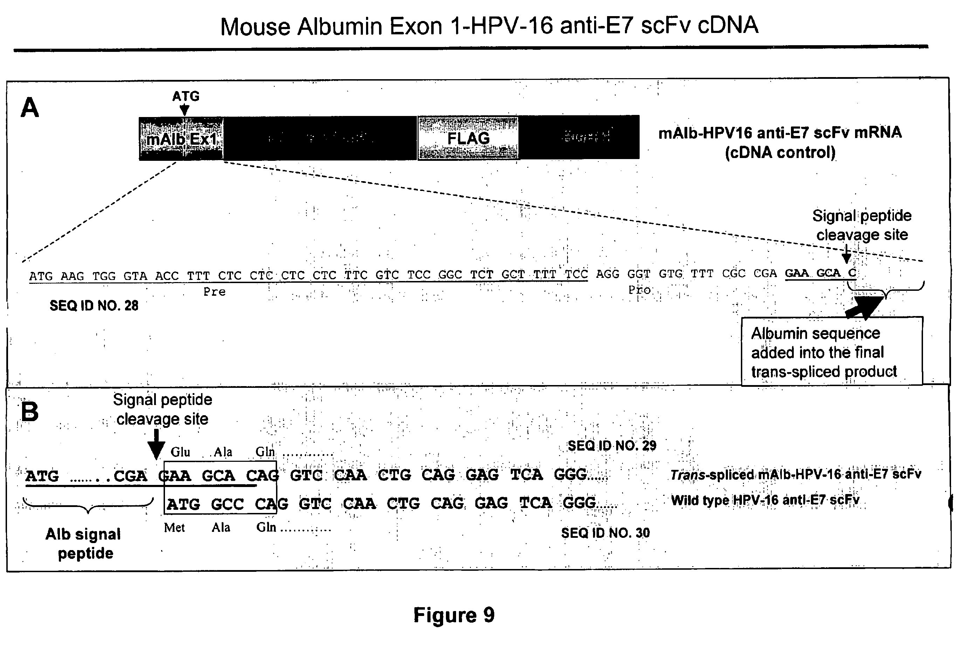

The mouse albumin-HPV-16 anti-E7 scFv (mAlb-HPV16 anti-E7 scFv) positive control cDNA (Figure 9) was constructed to imitate the final *trans*-spliced product and tested for expression, processing and secretion in Cos-7 and Hepal-6 (mouse hepatoma cells) cells. The *trans*-spliced cDNA expression plasmids was constructed using long synthetic complementary oligonucleotides and PCR product consisting of coding albumin exon 1 and HPV-16 anti-E7 scFv sequence. The coding sequence of mouse albumin exon 1 was assembled using the following long oligonucleotides: forward primer (SEQ ID NO:9):
**GCTAGC**ATGAAGTGGGTAACCTTTCTCCTCCTCCTCCTCTTCGTCTCCGGCTCTGCTT TTTCCAGGGGTGTGTTTCGCCGAGAAGCAC*AGGTCCAATGCAGGAGTCAGGG**GCT GAGC**,* and reverse primer (SEQ ID NO:10):
***GCTCAGC**CCCTGACTCCTGCAGTTGGACCT*GTGCTTCTCGGCGAAACACACCCCTG GAAAAAGCAGAGCCGGAGACGAAGAGGAGGAGGAGAAAGGTTACCCACTTCAT **GCTAGC**. (The nucleotides in bold include *Nhe*I and *Blp*I restriction sites used for cloning; underlined nucleotides include the mouse albumin exon 1 sequence, in which the majority codes for signal peptide; and the italicized nucleotides include partial HPV-16 anti-E7 scFv sequence).

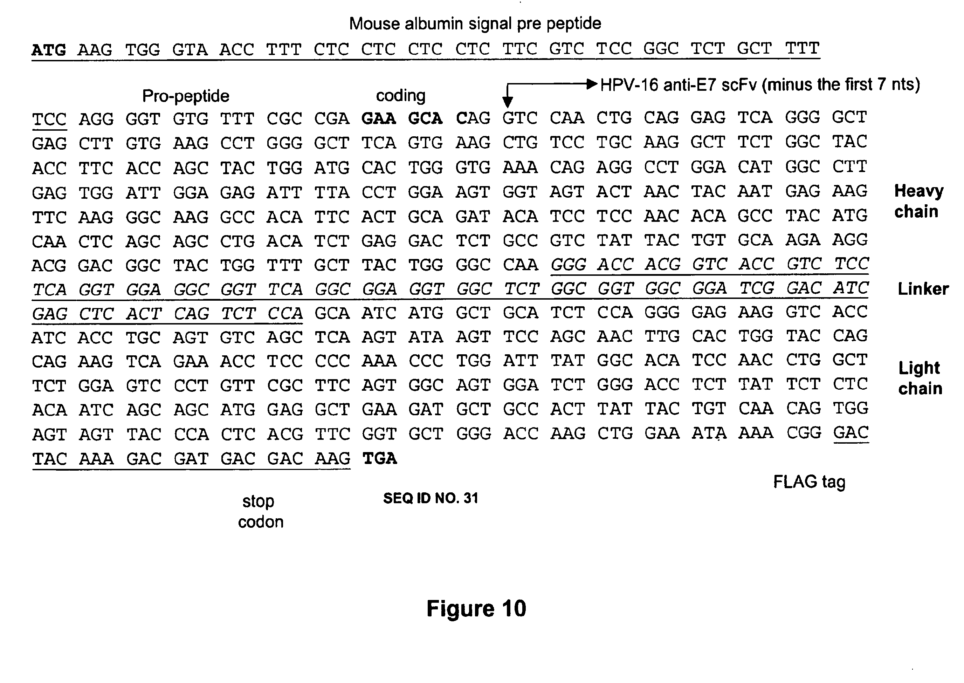

HPV-16 anti-E7 scFv coding sequence was PCR amplified using a cDNA clone and primers: Sca1 (5'- **GCTAGC**ATGGCCCAGGTCCAACTGCAGG) (SEQ ID NO:11) and Sca5 (5'- **AAGCTT** *TCA* CTTGTCGTCATCGTCTTTGTAGTCCCGTTTTATTTCC GCTTG GTCCCAGC) (SEQ ID NO:12) (nucleotides in bold, *Nhe*I and *Hind* III restriction sites for cloning; italicized nucleotides, stop codon; and the underlined nucleotides, FLAG tag). The PCR product was digested with *Blp*I and *Hind*III restriction enzymes. The resulting product was first ligated with the annealed oligo fragment and then ligated into pcDNA3.1 expression vector (Invitrogen). The authenticity of the PTM cassette sequence was verified by sequencing (Figure 10).

### EXAMPLE 2: PRODUCTION, EXPRESSION AND SECRETION OF ALBUMIN - HPV-16 ANTI-E7 SCFV ANTIBODY IN HEPA1-6 AND COS-7 CELLS:

The effect of the albumin exon 1 sequence (7 nucleotides) on expression and processing of HPV-16 anti-E7 scFv was evaluated by transfecting the *trans*-spliced cDNA plasmid along with a control plasmid (similar to the *trans*-spliced cDNA without the FLAG tag) into mouse hepatoma, Hepa1-6 and Cos-7 cells. 48 hrs post-transfection, medium was collected, passed through FLAG affinity column (Sigma, Cat# FLAGIPT-1) and analyzed by Western blot for the expression of HPV-16 anti-E7 scFv using anti-FLAG M2 monoclonal antibody (Sigma, Cat# F 3165).

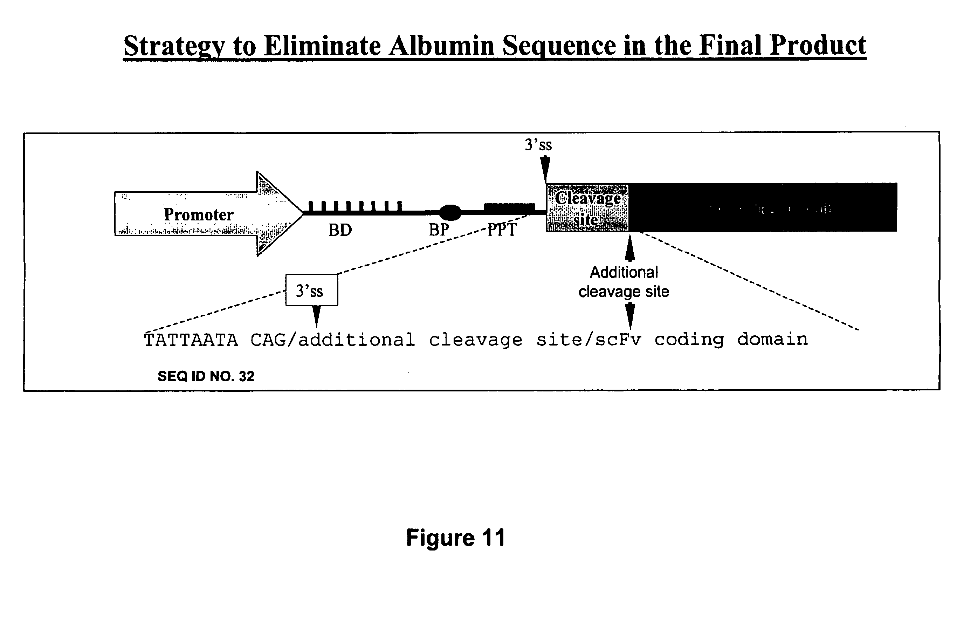

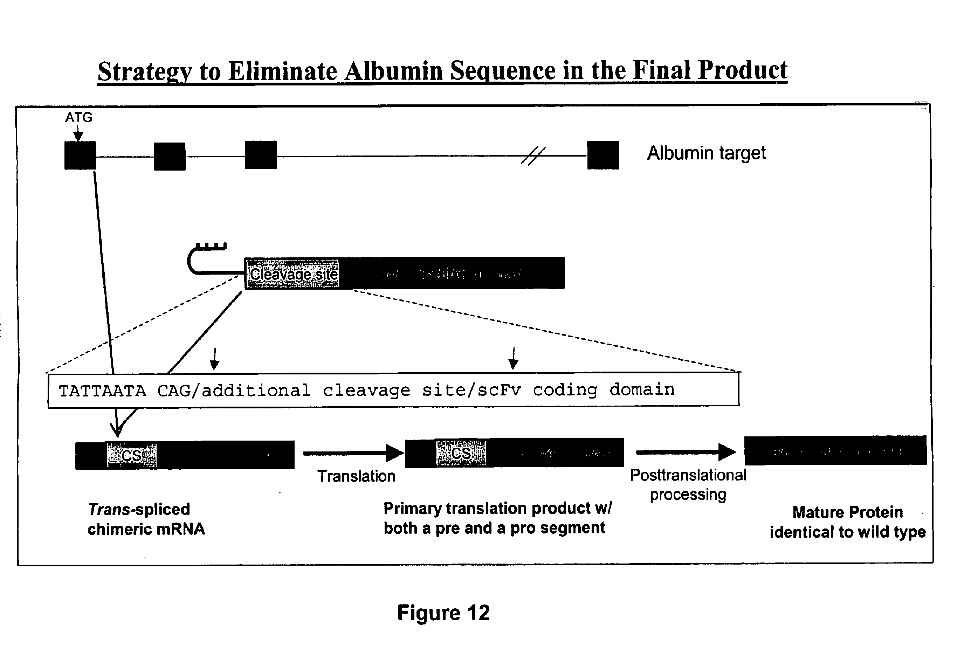

The current albumin trans-splicing strategy results in the production of chimeric mRNA and protein. The final trans-spliced product contains 7 nucleotides or 2 amino acids from albumin target mRNA. For human applications it may be desirable to eliminate the albumin sequence in the final product to preclude immunological reactions. In one exemplary strategy, illustrated in Figure 11, the PTM will be engineered to encode "Furin" like endopeptidase (or proprotein convertase) cleavage site which has been used to express proteins *in vivo* (Fuller RS, Brake AJ, Thorner J, Science, 246: 482-486, 1989; Bresnahan PA, Leduc R, Thomas L, Thorner J, Gibson HL, Brake AJ, Barr PJ, Thomas G., J Cell Biol. 111:2851-2859, 1990; van de Ven WJ, Voorberg J, Fontijn R, Pannekoek H, van den Ouweland AM, van Duijnhoven HL, Roebroek AJ, Siezen RJ, Mol Biol Rep. 14:265-75, 1990; Duckert P, Brunak S, Blom N. Protein Eng Design & Selection. 17:107-112, 2004). In another example, the PTM will be designed to include the protein's own native secretion signal, i.e., "pre-pro" signal (if it has one). This strategy was designed to take advantage of the endogenous native cellular machinery to enhance recognition, processing and secretion of the final trans-spliced protein to the site of action similar to wild type protein. For example, trans-splicing of PTM into albumin pre-mRNA target produces a chimeric mRNA and pre-pro-protein that in addition to signal peptide cleavage in rough endoplasmic reticulum undergoes several post-translational modifications in other cellular compartments and, finally, endopeptidase cleavage resulting in the release of a mature, fully processed biologically active protein that is identical to the wild type (Figure 12).

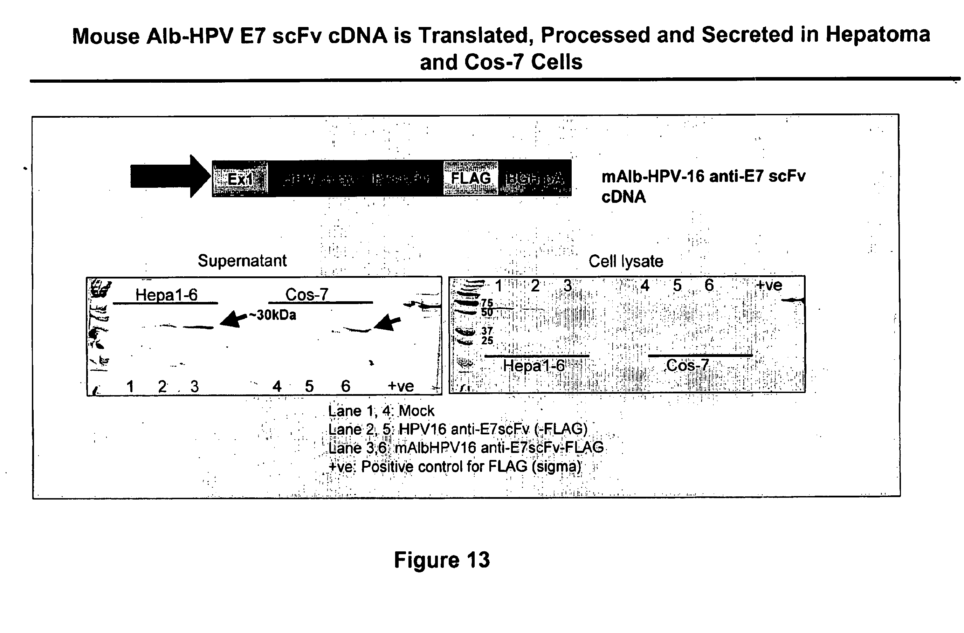

About 10 µg of total protein from the supernatant or the total cell lysate from cells transfected with cDNA expression plasmids was analyzed on a 12% SDS-PAGE and transferred onto nylon membrane and probed with anti-FLAG antibody. Western results confirmed the production of HPV-16 anti-E7 scFv, 30 kDa in size predicted for the mature protein in cells that were transfected with FLAG-tagged cDNA expression plasmid in both Hepa1-6 and Cos-7 cells (Figure 13 lanes 3 & 6, left panel). On the other hand, no such product was detected in mock and in cells that received the cDNA construct without the FLAG tag (Figure 13 lanes 1-2 and 5-6, left panel). In addition, no protein was detected in the cell lysate (Figure 13) indicating that the majority of the protein processed and secreted normally.

### EXAMPLE 3: TRANS-SPLICED ALBUMIN HPV-16 ANTI-E7 SCFV PROTEIN IS FUNCTIONALLY ACTIVE

The effect of the albumin sequence on HPV-16 anti-E7 scFv function was evaluated by its ability to down regulate HPV-16 E7 expression in cervical cancer cells. Cervical cancer cells, SiHa, (ATCC # HTB-35) that are HPV-16 E7 oncoprotein positive was transfected with mAlb-HPV-16 anti-E7 scFv cDNA expression plasmid. The matching control cells, C-33A (ATCC # HTB-31) that do not express E7 oncoprotein was also transfected with the mA1b-HPV-16 anti-E7 scFv cDNA expression plasmid. Cells were grown for 5 days and the number of relative viable cells was determined by colorimetric (MTT) assay.

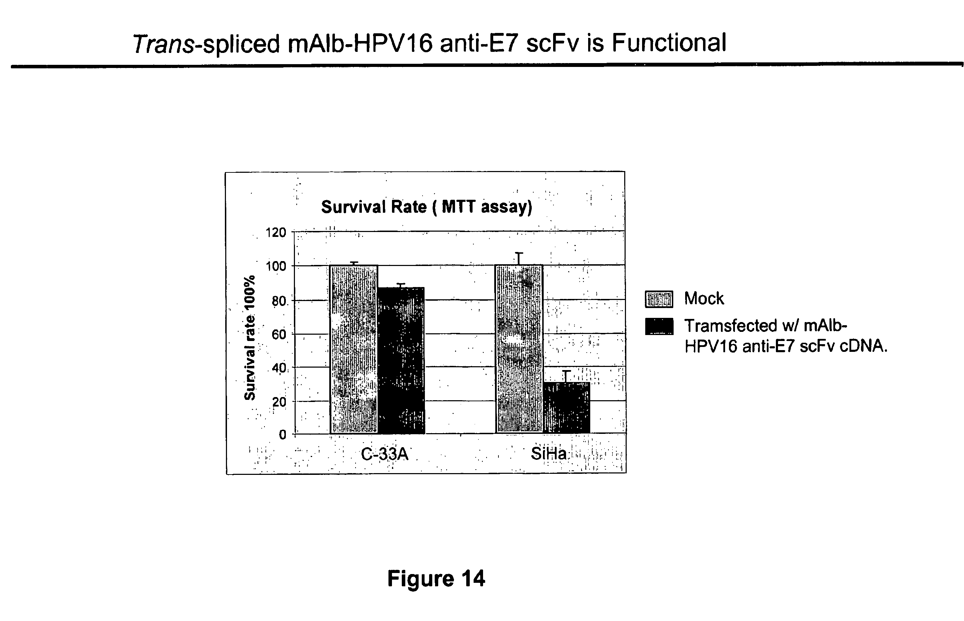

In the case of HPV-16 positive cervical cancer cells, SiHa, mA1b-HPV-16 anti-E7 scFv inhibited cell proliferation by -75% compared to about <10% inhibition in C-33A HPV-negative cells and thereby demonstrated the functionality of the *trans*-spliced albumin HPV-16 anti-E7 scFv antibody (Figure 14). These results not only confirmed the absence of any major adverse effects due to albumin sequence in the final *trans*-spliced product on HPV-16 anti-E7 scFv function, but also provide evidence of the effectiveness of the compositions of the present invention for the production of functional antibody polypeptides and/or therapeutic proteins *in vivo.*

The structure of HPV-16 anti-E7 scFv PTM expression cassette used for the proof-of-principle study is illustrated in Figure 15A. The PTM cassette consists of a *trans-*splicing domain (TSD) that includes 279 nts binding domain complementary to mouse albumin intron 1, 24 nucleotide spacer region, strong 3' splice elements such as the consensus yeast branch point (BP), an optimized polypyrimidine tract, a splice acceptor site (CAG dinucleotide) followed by the majority of the coding sequence for HPV-16 anti-E7 scFv (Figure 13). The PTM cassette also contains bovine growth hormone polyadenylation signal and FLAG tag to assist in the detection of *trans*-spliced protein. The entire cassette was cloned into the pcDNA3.1 vector backbone, which contains the cytomegalovirus (CMV) promoter (Invitrogen). In addition, the vector backbone was further modified to include the Maz4 (transcriptional pause site) sequence to reduce cryptic *cis*-splicing between vector ampicillin gene and the PTM 3' splice site.

A splice mutant (splice incompetent) was also constructed that was identical to the functional PTM described above but had a point mutation at the acceptor site (CAG>CAT) (Figures 15A and 15B). The splice mutant was used as a negative control. For *in vitro* proof-of-principle studies, a mouse albumin mini-gene target pre-mRNA was used that consisted of exon 1, intron 1 and exon 2. A schematic diagram of the pre-mRNA target is illustrated in Figure 15C.

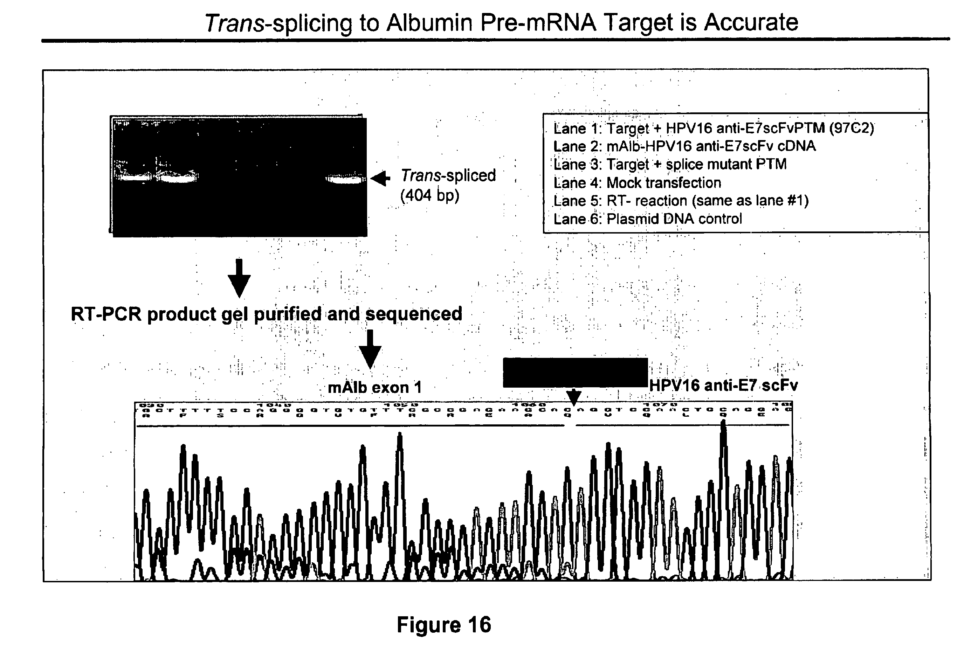

PTM mediated *trans*-splicing and production of mouse albumin-HPV-16 anti-E7 scFv chimeric mRNA was evaluated by co-transfecting Hepa1-6 cells with mouse albumin mini-gene target plasmid along with HPV-16 anti-E7 scFv PTM (functional PTM) or with the splice mutant (splice incompetent PTM) and mock transfection. Total RNA isolated from these cells was analyzed by RT-PCR using mouse albumin exon 1 (AlbA1TSF2: ACCTTTCTCCTCCTCCTCTTCGT) (SEQ ID NO:13) and HPV-16 anti-E7 scFv PTM (sca3: AGTAAGCAAACCAGTAGCCGTC) (SEQ ID NO:14) specific primers (primer binding sites indicated in Figure 15A and 15C). These primers produced the predicted 404 bp product only in cells that received both target and functional PTM (Figure 16, lane 1) which co-migrated along with a similar size band observed with cDNA control (Figure 16, lane 2) and plasmid DNA (Figure 16, lane 6). No RT-PCR product was detected in cells transfected with the splice mutant (Figure 16, lane 3) or in mock transfection (Figure 16, lane 4). The PCR product was purified and was directly sequenced, confirming the precise *trans*-splicing to the predicted splice sites of the PTM and the target pre-mRNA in these cells (Figure 16, lower panel). Thus, the above results establish that the methods of the present invention may be used to provide efficient *trans*-splicing of HPV-16 anti-E7 scFv PTM *in vitro.*

### EXAMPLE 4: IN VIVO TRANS-SPLICING TO ENDOGENOUS MOUSE ALBUMIN PRE-mRNA TARGET AND PRODUCTION OF HPV-16 ANTI-E7 SCFV IN MICE.

To demonstrate *trans*-splicing of the PTM into an endogenous mouse albumin target and production of HPV-16 anti-E7 scFv protein, the following experiments were conducted. One hundred micrograms of mAlb-HPV 16 anti-E797C2 (PTM only), 70 µg of PTM + 35 µg of mini-gene target (additional target plasmid to increase pre-mRNA concentration) or 100 µg of the control cDNA (mAlb-HPV16 anti-E7scFv) plasmid that mimics *trans*-spliced mRNA were hydrodynamically injected via tail vein into normal C57BL/6 mice. Serum samples were collected at 8, 16 and 24 hrs time points and analyzed by Western blot. Approximately, 25-100 µl serum was passed through FLAG affinity column, samples were then separated on a 12% SDS-PAGE, transferred on to nitrocellulose membrane and probed with anti-FLAG M2 monoclonal antibody. Proteins were visualized using a chemiluminescence kit (Invitrogen, Cat# WB7103).

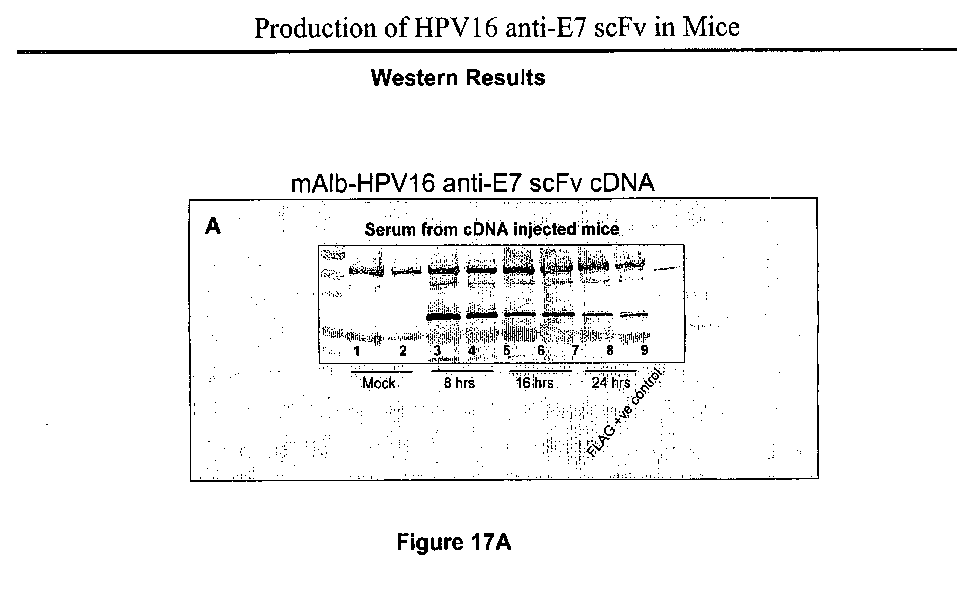

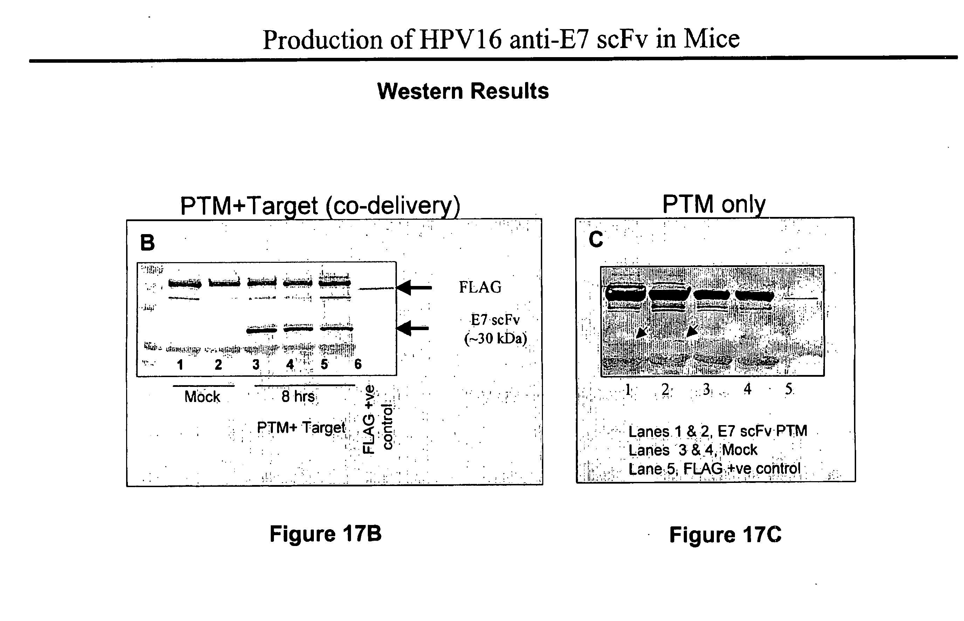

Western blot results indicated the appearance of HPV-16 anti-E7 scFv in the circulation of the mice as early as 8 hrs post-injection with the cDNA control expression plasmid (Figure 17A, lanes 3 and 4) and the levels dropped significantly at 24 hrs (Figure 17A, lanes 7 and 8). Efficient *trans*-splicing and production of predicted 30 kDa HPV 16 anti-E7 scFv was also detected in mice that received both the target and PTM (Figure 17B, lanes 3-5, left panel). On the other hand, no such band was detected in mock treated mice (Figure 17B, lanes 1-2, left panel). Finally, mice that received only the PTM (targeting endogenous target) also showed the presence of a 30 kDa HPV16 anti-E7 scFv (Figure 17C, lanes 1-2). These results clearly show: (a) successful and accurate *trans*-splicing of mouse albumin PTM into a mouse albumin target pre-mRNA, (b) production of HPV 16 anti-E7 scFv through *trans*-splicing. In addition, the above results further validate the targeting strategy of the present invention for the production of therapeutic antibody polypeptides and fragments thereof *in vivo.*

### EXAMPLE 5: DOUBLE CHAIN ANTIBODY PRODUCTION

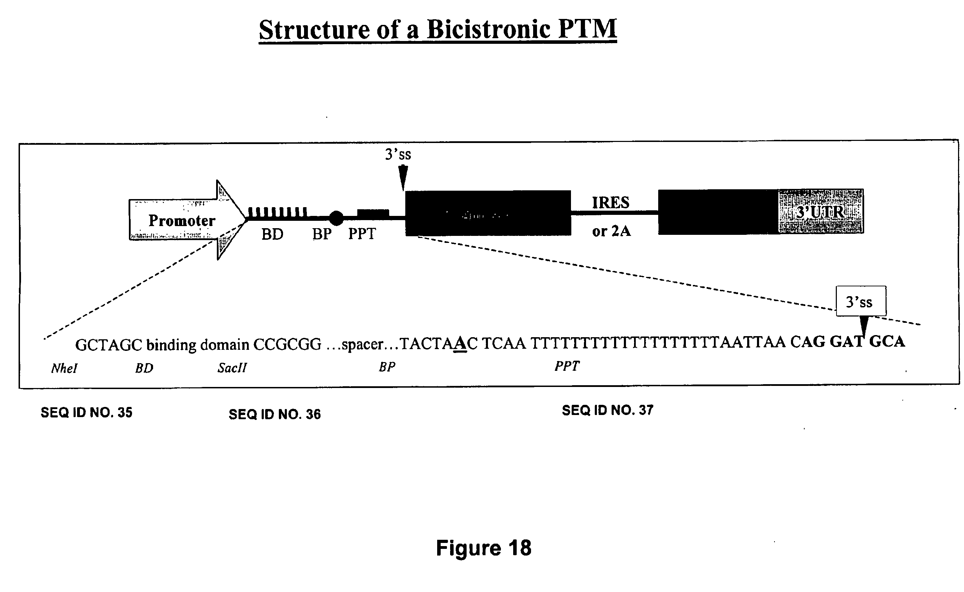

According to some embodiments, the PTM cassettes of the present invention may be modified to produce antibodies containing both the light and heavy chain. As illustrated in Figure 18, the bicistronic PTM cassette is similar to the HPV-16 E7 scFv PTM shown in Figure 15A, except that it may contain, after the coding domain for the single chain antibody sequence, 2A self-cleaving oligo peptide derived from Foot and Mouth Disease Virus (FMDV) (Fang et al., Nature Biotechnol, 23: 584, 2005, the disclosure of which is hereby incorporated by reference) or the encephlomayocardities (ECMV) internal ribosome entry site (IRES) (Martienz-Salas, Curr Opin Biotechnol, 10:458, 1999, the disclosure of which is hereby incorporated by reference) sequence followed by the full length coding sequence to induce high levels of translation of the second chain. The use of the 2A oligo peptide and/or the IRES sequence to express the second transgene has been well documented (Fang et al., Nature Biotechnol, 23: 584, 2005; Martienz-Salas, Curr Opin Biotechnol, 10:458, 1999). In addition, PTMs encoding single chain and the second chain (separate PTMs) could also be used for the production of double chain antibodies.

### EXAMPLE 6: EXPRESSION OF HUMAN APOLIPOPROTEIN (APO A-1) ALBUMIN-HUMAN APO A-1 FUSION PROTEINS

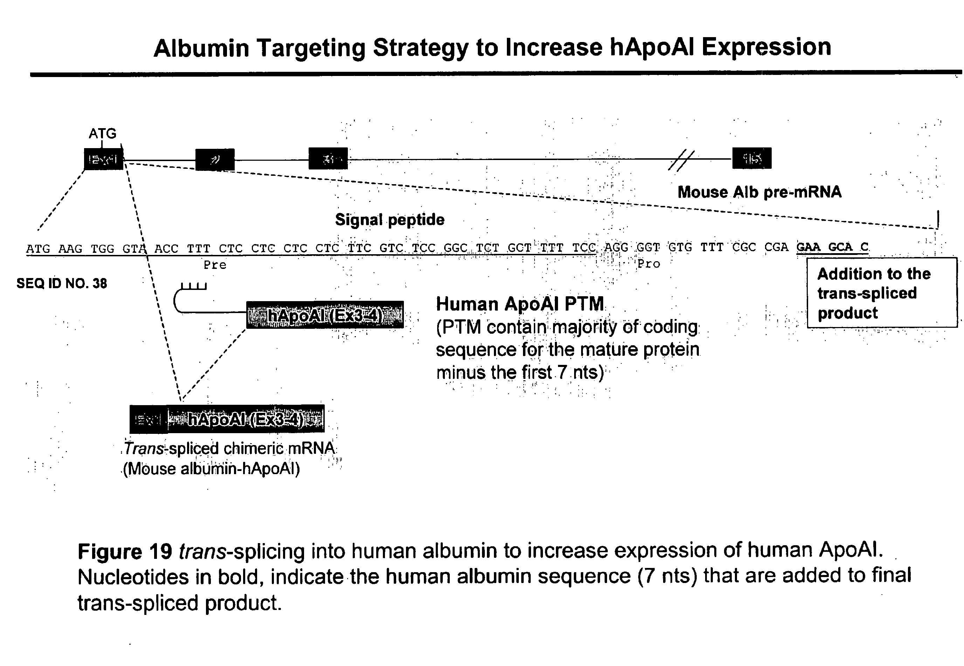

The present study was undertaken to evaluate albumin targeting strategy (Figure 19) for the production of human Apo A-1 protein, major component of high density lipoprotein (HDL) or other variants and subsequently increase HDL concentration as a treatment for individuals having or at risk for cardio vascular disease (CHD). The rationale for selecting albumin as a target is because of its elevated expression in liver. High albumin pre-mRNA concentration results in abundant targets for *trans*-splicing. The concept involves targeted *trans*-splicing of wild type human Apo A-1 or Apo A-1 analogues into albumin pre-mRNA target; and the goal is to increase Apo A-1 expression. This study evaluates the effect of albumin sequence human Apo A-1 protein expression, secretion and function.

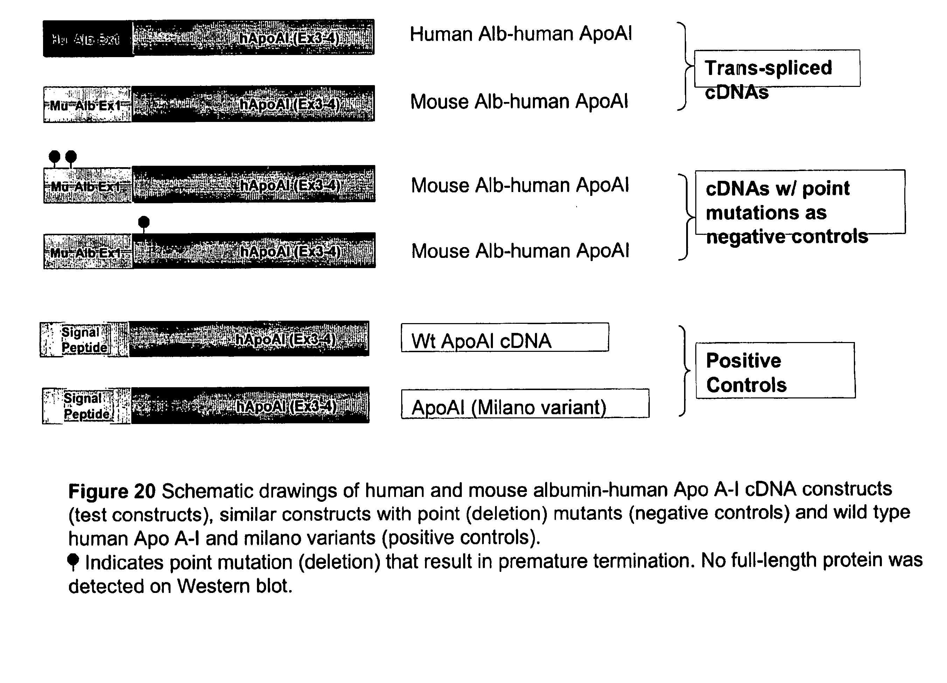

The fusion (albumin-Apo A-1) function *in vivo* was evaluated. Human and mouse versions of the albumin-human Apo A-1 cDNA controls (Figure 20) were constructed to mimic the final *trans*-spliced product for expression, processing and function in 293 and hepatoma cells (HepG2). The fusion cDNA constructs were constructed using long complementary oligonucleotides and PCR products consisting of albumin exon 1 and human Apo A-1 exon 3 and 4. Briefly, the coding sequence of mouse and human albumin exon 1 were assembled using the following long oligos:
mouse Alb forward primer (SEQ ID NO: 15): 
ATGAAGTGGGTAACCTTTCTCCTCCTCCTCTTCGTCTCCGGCTCTGCTTTTTCCAG
GGGTGTGTTTCGCCGAGAAGCACCC,
reverse primer (SEQ ID NO: 16): 
GGGTGCTTCTCGGCGAAACACACCCCTGGAAAAAGCAGAGCCGGAGACGAAGA
GGAGGAGGAGAAAGGTTACCCACTTCATG,
and human Alb forward primer (SEQ ID NO:17):: 
ATGAAGTGGGTAACCTTTATTTCCCTTCTTTTTCTCTTTAGCTCGGCTTATTCC
AGGGGTGTGTTTCGTCGAGATGCACCC,
reverse primer (SEQ ID NO:18)::
   GGGTGCATCTCGACGAAACACACCCCTGGAATAAGCCGAGCTAAAGAGAAAAAG AAGGGAAATAAAGGTTACCCACTTCATG. The underlined nucleotides indicate the end of albumin exon 1 sequence and 2 "C"s at the 3' end of the forward primers overlap to human Apo A-1.

Human Apo A-1 coding sequence was PCR amplified using a cDNA clone (ATCC: clone # MGC-1249) and primers: Apo23 (SEQ ID NO:19) (5'-CCCCAGAGCCCCTGGGATCGAGTG) and Apo5 (SEQ ID NO:20) (5'- CTAG **AAGCTT** CCCACTTTGGAAACGTTTAT TCTGAGCACC GG). The PCR product was blunted at the 5' end and then digested with Hind III (indicated in bold) restriction enzyme. The resulting product was first ligated with mouse or human albumin exon 1 and then cloned into pcDNA3.1 expression vector (Invitrogen). Expression plasmids containing the entire coding sequence of human Apo A-1 including the signal peptide into pcDNA3.1 to generate wild type human Apo A-1, and the Milano variant which contains an Arg to Cys substitution at position 173 (R173C) expression plasmids were also constructed as positive controls. The final constructs were verified by sequencing.

### PRODUCTION. EXPRESSION AND SECRETION OF ALBUMIN APO A-1 FUSION PROTEINS IN 293 CELLS

The effect of albumin exon 1 sequence on expression and processing of human Apo A-1 protein was evaluated by transfecting human and mouse fusion cDNA plasmids along with a negative (deletion mutant) and a positive control cDNAs (wt Apo A-1) into 293 cells. After transfection, cells were rinsed 2X with serum free DMEM and incubated with serum free advanced DMEM media (Invitrogen). After 48 hrs post-transfection, media was collected, concentrated, analyzed for the expression of human Apo A-1 protein.

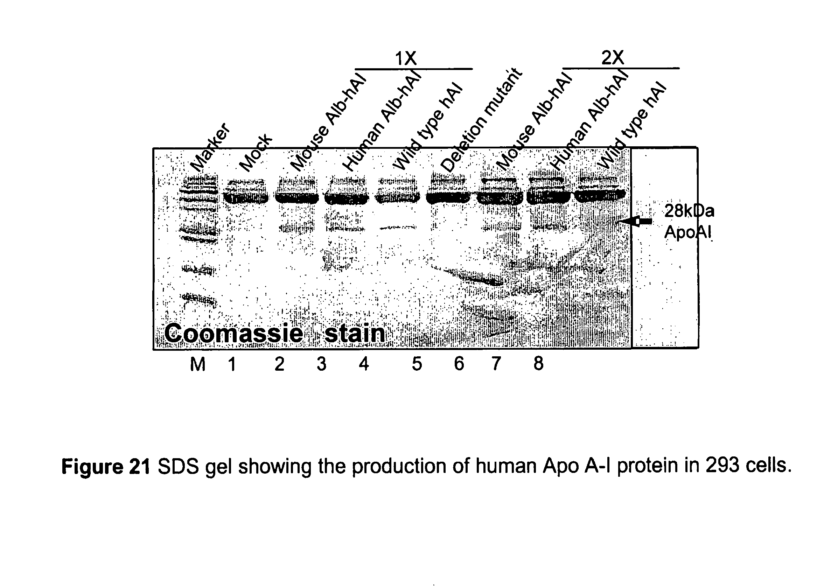

Coomassie Blue staining of the gel revealed that both the mouse and the human fusion cDNAs produced the predicted ∼28kDa protein band which co-migrated with that of wt Apo A-1 demonstrating good expression, processing and secretion in 293 cells (Figure 21, lanes 2-3, 6-7). In addition, these data also showed that the level of expression was similar to that of wt Apo A-1 (Figure 21, lane 4, 8) indicating no adverse effects of albumin sequence on human Apo A-1 expression and processing. On the other hand, no such band was detected in mock and in cells that received mouse fusion cDNA with 2 nucleotide deletion in the signal peptide (Figure 21, lane 1 and 5).

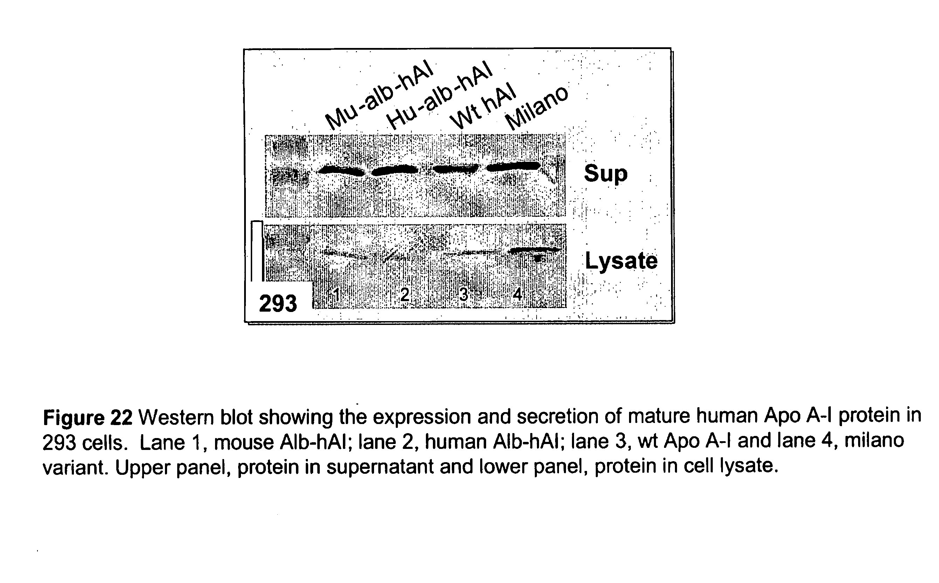

The identity of the band that was observed in SDS gel as human Apo A-1 was confirmed by Western analysis using a monoclonal human Apo A-1 antibody (Biodesign, Cat. # H45625). About ∼5-10 µg total protein from the supernatant or the total cell lysate from cells transfected with fusion cDNA constructs, wt Apo A-1 and Milano variant was analyzed on a 12% SDS gel and transferred onto nylon membrane and incubated with human anti-Apo A-1 antibody. Western results confirmed the production of human Apo A-1 protein with an apparent molecular mass of 28 kDa predicted for the mature protein. Western data also indicated the presence of >90% of the mature human Apo A-1 protein from the fusions or wt Apo A-1 in the supernatant compared to cell lysate demonstrating normal processing and secretion in 293 cells (Figure 22, compare lanes 1 & 2 with 3). Similar results were also observed with hepatoma (HepG2) cells transfected with fusion cDNA constructs.

### ALBUMIN APO A-1 FUSION PROTEIN IS FUNCTIONALLY ACTIVE

The effect of albumin sequence on human Apo A-1 function was evaluated by measuring ATP-binding cassette transporter protein (ABC1) mediated transfer of cellular cholesterol into Apo A-1 acceptor. The release of radio-labeled cellular cholesterol to lipid free human Apo A-1 was quantified and the efflux values obtained with fusion proteins was compared with those from wt Apo A-1 and negative control samples. Control HeLa and HeLa cells stably transfected with ABC1 plasmid were grown to near confluency. Cells were then loaded with 1 µCi/ml ³H cholesterol. After equilibrating for 24 hrs, cells were washed 3X with serum free media and incubated with a serial dilution of the media containing the fusion proteins (supernatant from 293 cells transfected w/ fusion cDNA constructs, normalized for Apo A-1 protein concentration) or with 10 µg/ wild type Apo A-1 protein as positive control. Cells were allowed to efflux for 18 hrs. After the efflux period, media was collected and an aliquot of the medium was then counted by liquid scintillation counting. The remaining counts in the cell fraction were determined after an over night extraction with isopropanol. The percent efflux was calculated by dividing the counts in the efflux media by the sum of the counts in the media plus the cell fraction. DMEM/BSA media was used as a blank and was subtracted from the radioactive counts obtained in the presence of an acceptor in the efflux media.

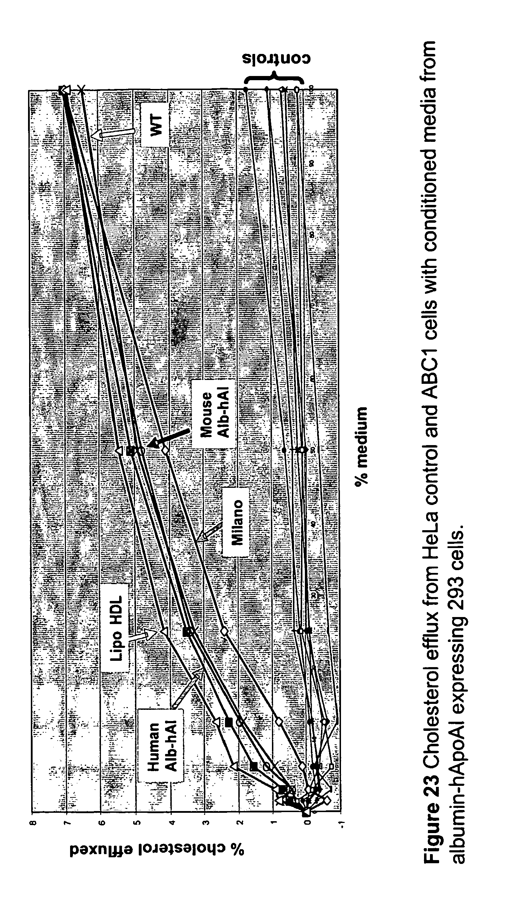

The amount of ABC1 mediated efflux observed with fusion proteins (mouse and human fusion proteins) was similar to that of wt Apo A-1 (Figure 23). The efflux data also demonstrated that the absolute efflux activity observed with the fusion proteins were comparable or slightly better than the wt Apo A-1 protein across the concentration range tested indicating the absence of any major adverse effects due to albumin sequence in the final *trans*-spliced product on Apo A-1 function. These results provide strong evidence about the effectiveness of the compositions of the present invention for the production of functional biologically active proteins *in vivo.*

### EXAMPLE 7: HIGH CAPACITY SCREENS FOR ISOLATION OF OPTIMAL BINDING DOMAINS FOR ALBUMIN TARGETS

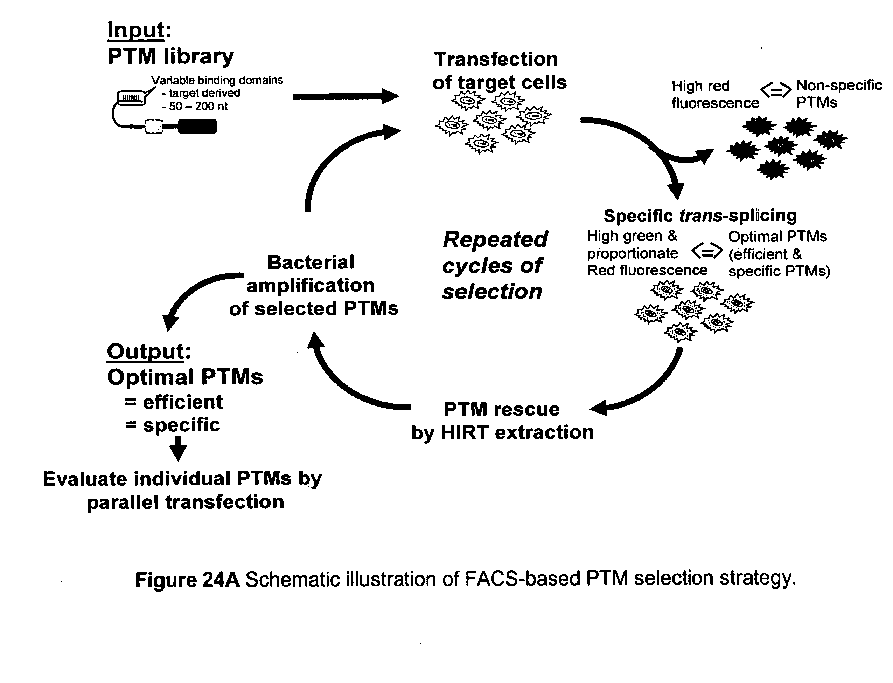

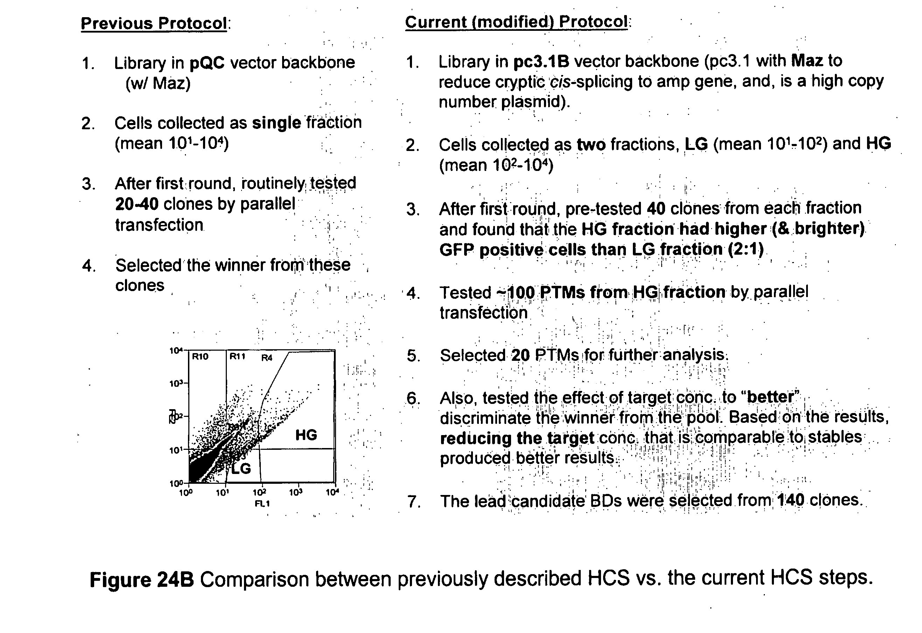

A high capacity screen (HCS) to identify optimal binding domains for mouse albumin pre-mRNA target was performed as described before (U.S. Patent Application Serial No. 10/693,192, filed 10/24/2003) (Figure 24A) with various modifications (Figure 24B).

### HIGH CAPACITY SCREEN PRE-MRNA TARGET

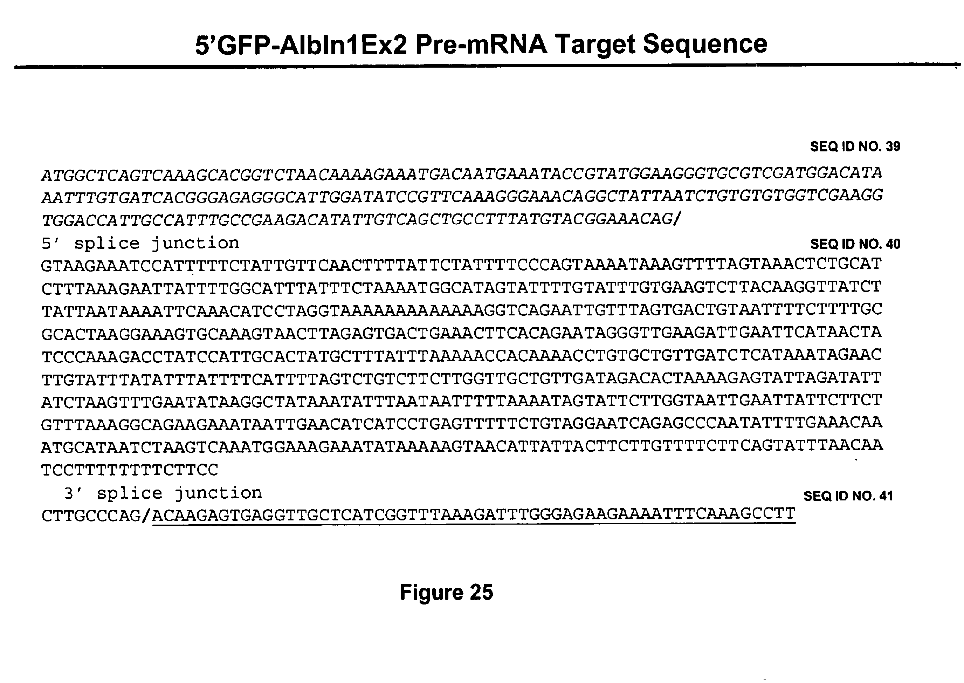

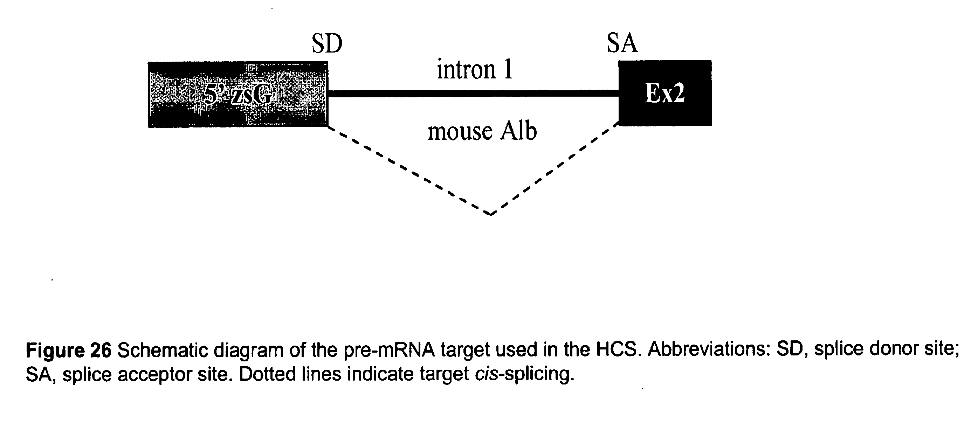

Mouse albumin intron 1 and exon 2 comprising of nucleotides 114 through 877 total of 763 bp (Ref. seq. NC_000071) (Figure 25) was PCR amplified using the genomic DNA and primers malb15 (SEQ ID NO:21) (5'- CTAG GGATCC GTTTTATGTTTTTTCATCTCTG) and mAlb8 (SEQ ID NO:22) (5'- CTAG GCGGCCGC AGGCCTTTGAAATGTTGTTCTCC). The PCR product was then digested with Bam HI and Not I (indicated in bold) and cloned into an existing HCS target plasmid to generate pc5'zsG-mIn1-Ex2 plasmid (Figure 26). Stable cells expressing the 5' half of the coding sequence for the green fluorescent protein (GFP) (zsGreen from Clontech) coupled to intron 1 and exon 2 of mouse albumin gene was established in 293 cells by transfecting the target plasmid followed by hygromycin selection. After 2 weeks of selection, hygromycin resistant clones were pooled, characterized by RT-PCR and used for HCS.

### MOUSE ALBUMIN PTM BINDING DOMAIN LIBRARY

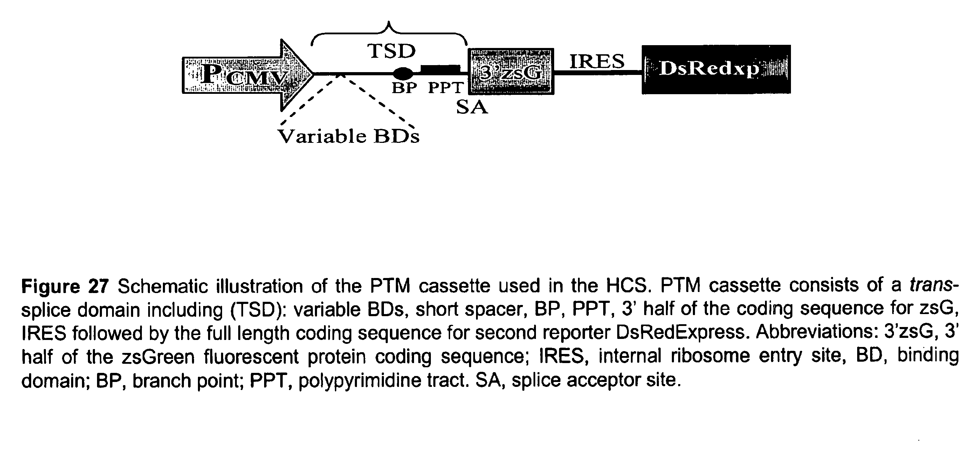

The mouse albumin sequence comprising intron 1 and exon 2 was PCR amplified using genomic DNA and primers as described above, digested with Bam HI and Not I and ligated to generate a large concatemerized fragment (~10 kb). This step was introduced to increase BD complexity. The concatemerized DNA was then fragmented into small pieces by sonication and fractionated on a 3% agarose gel. Fragment size ranging from 50-250 nucleotides were gel purified, ends were repaired using Klenow enzyme and cloned into PTM cassette described before (U.S. Patent Application Serial No. 10/693,192, filed 10/24/2003) (Figure 27).

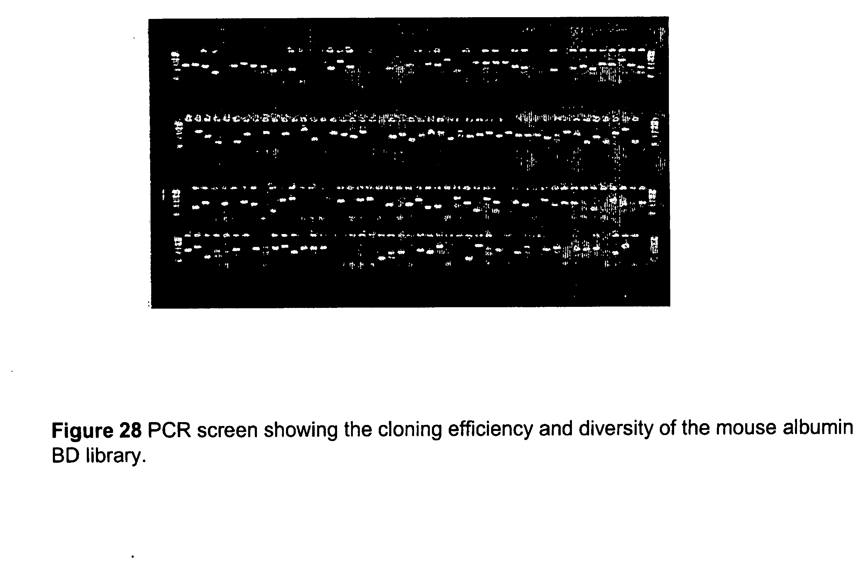

PCR analysis of the library colonies showed >87% recombination efficiency and produced a complex library with >10⁶ independent clones with BDs varying in size from 50-250 nts (Figure 28). The primary library was amplified in bacteria and used for screening the optimal BDs by HCS.

### PTM SELECTION STRATEGY

Following the FACS-based PTM selection strategy described before (U.S. Patent Application Serial No. 10/693,192, filed 10/24/2003), a mAlb binding domain (BD) library using the assay cells expressing the 5'zsG-mIn1-Ex2 pre-mRNA target was tested. *(See* Figure 24B)

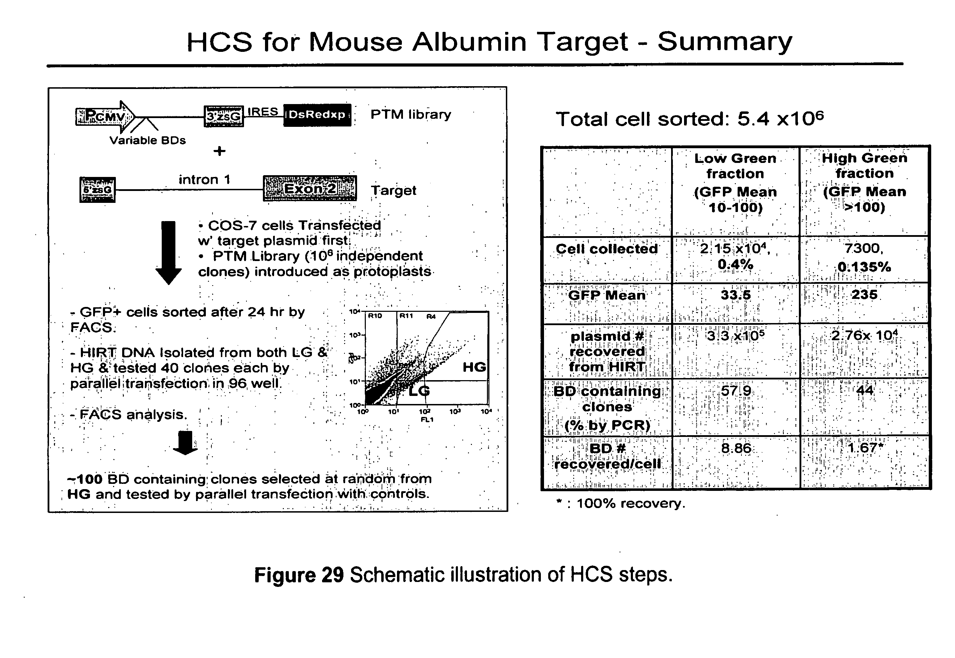

Briefly, on day 1, COS-7 cells were plated and transfected with 5'zsG-mIn1-Ex2 target plasmid using Lipo2000 reagent. On day 2, ~10⁶ independent PTM clones were delivered to assay cells expressing 5'zsG-mIn1-Ex2 pre-mRNA as protoplasts. As illustrated in the Figure 29, cells were sorted after 24 hr by FACS, and cells expressing high GFP and proportionate RFP were collected in 2 fractions i.e., high green (HG) and low green (LG) fractions, instead of a single fraction as previously described. PTMs from the collected cells were rescued by HIRT DNA extraction followed by EcoR V digestion to reduce target plasmid contamination in the final HIRT DNA preparation. About 40 binding domain containing PTMs from LG and HG fractions were initially tested by parallel transfection. Trans-splicing efficiency of these PTMs was assessed by FACS analysis.

As predicted, the percent GFP positive (GFP⁺) cells and the mean GFP fluorescence was higher in PTMs from HG fraction compared to LG fraction with a 2:1 ratio (Figure 29).

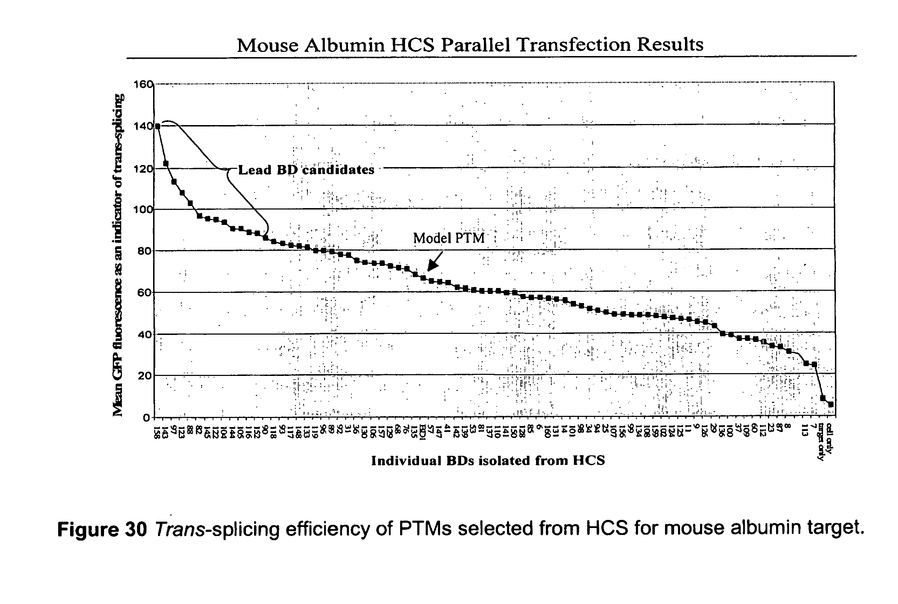

A hundred more BD containing clones from HG fraction was isolated and tested by parallel transfection and the results are summarized in Figure 30. GFP mean fluorescence was used as an indicator for assessing trans-splicing efficiency of the individual PTMs. Based on the GFP mean fluorescence, the trans-splicing efficiency of the majority of the PTMs selected from the HCS were either similar or slightly higher than the rationally designed model PTM (Figure 30). However, several PTMs with considerably higher (1.5 to 2-fold) trans-splicing compared with the model PTM were present. In the current screen, a ratio of 1:20 of superior PTMs vs. the rest was obtained.

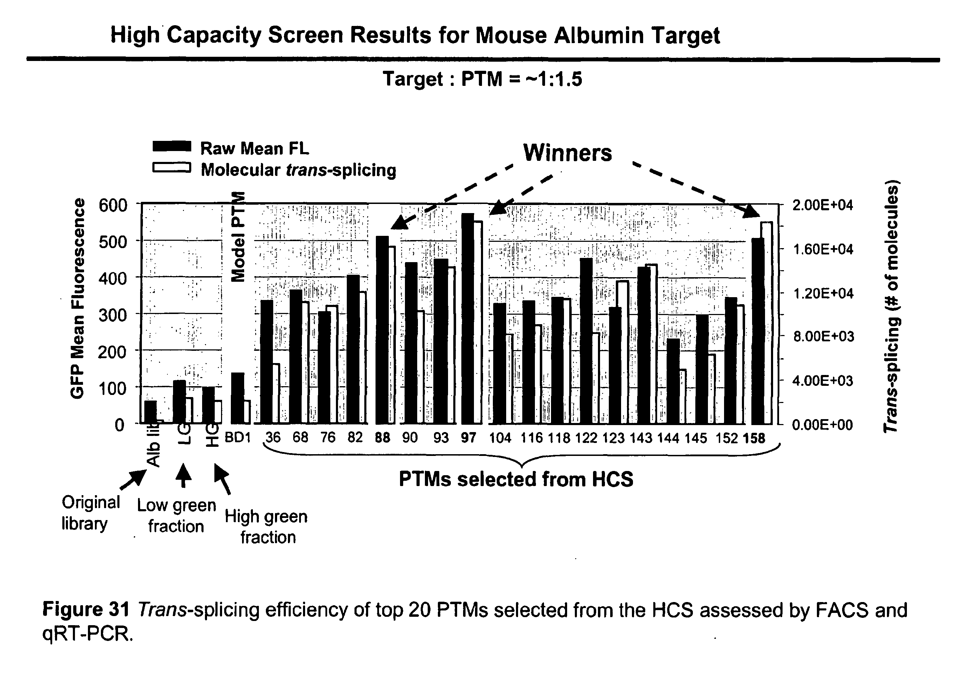

From this step, the top 20 PTMs were selected for further characterization by parallel transfection followed by molecular analysis using reverse transcription (RT) real time quantitative PCR (RT-qPCR) for specific *trans*-splicing and the results are summarized in Figure 31. Total RNA was isolated and *trans*-splicing efficiency was measured by RT-qPCR. Target and PTM specific primers were used for measuring specific *trans*-splicing, and total splicing was measured using primers specific for the 5'zsG exon as previously described. Based on the qPCR or GFP mean fluorescence values up to ~5-10 fold enrichment (after normalization) for *trans*-splicing efficiency was detected with PTMs selected from the HCS compared to a rationally designed model PTM (Figure 31). Similar results, i.e. enhancement in *trans*-splicing efficiency, was observed with the enriched library (LG and HG samples) compared with the starting library, which is consistent with previous screen.

The effect of BD orientation and sequence position on *trans*-splicing efficiency and specificity was also analyzed. The sequence of random clones from the starting PTM library were compared with the enriched library i.e., PTMs selected after one round of enrichment.

Sequence analysis of the PTMs from the starting library revealed that ~ 51% of the BDs were in correct (antisense) orientation compared to 49% incorrect orientation. The BD size varied from 40 nt and up to 336 nt and also showed good distribution indicating the complexity of the mAlb BD library. In contrast, sequence analysis of the PTMs selected from the enriched library, as expected, showed an increase in correct orientation BDs (88%) and the mean BD length was significantly higher than the starting library, which is consistent with previous work demonstrating that longer BDs are more efficient (Puttaraju *et al*., 2001).

### EXAMPLE 8: TRANS-SPLICING OF HUMAN APOLIPOPROTEIN APO A-1 IN CELLS HUMAN APOLIPOPROTEIN (APO A-1) PTM

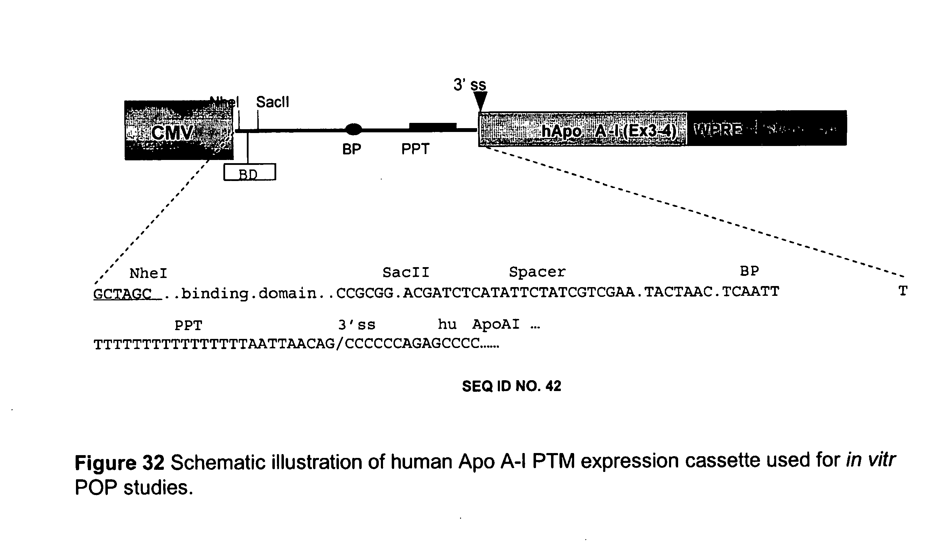

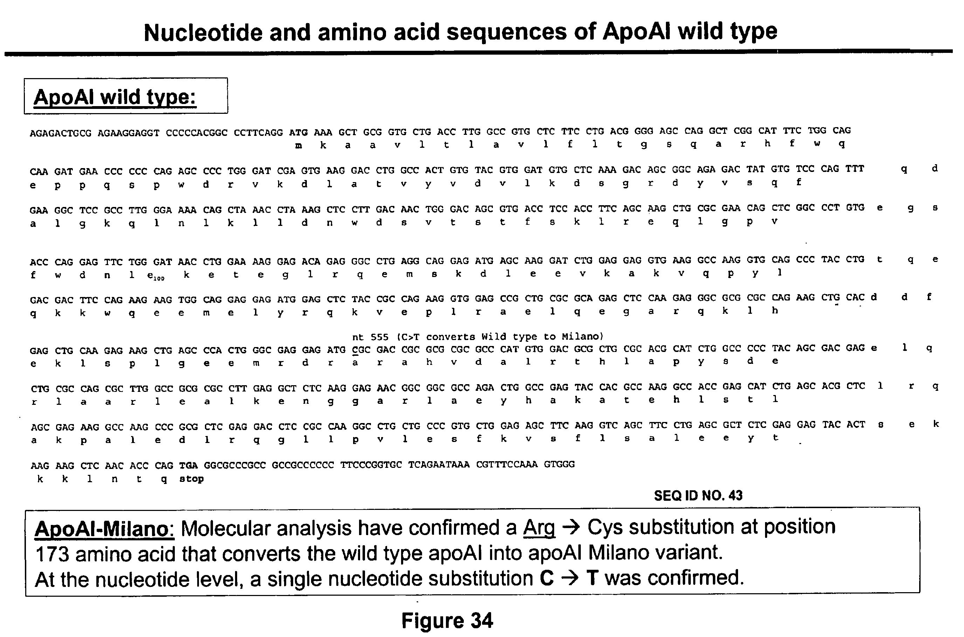

Detailed structure of a human Apolipoprotein A1 (Apo A-1) PTM used in this example to show proof of principle is shown in Figure 32. The PTM cassette consists of a *trans*-splicing domain (TSD) that include unique restriction sites, NheI and SacII, for cloning the lead binding domains (BDs), a 24 nucleotide spacer region, a strong 3' splice site including the consensus yeast branch point (BP), an extended polypyrimidine tract (19 nucleotides long), a splice acceptor site (CAG dinucleotide) followed by the majority of the coding sequence for wild type human Apo A-1 mRNA from nt 118 through nt 842 (Ref seq. NM_000039 and as shown in Figure 34). The PTM cassette also contains the SV40 polyadenylation site and woodchuck hepatitis post-transcriptional regulatory element (WPRE) to enhance the stability of trans-spliced message. The entire cassette is cloned into pcDNA3.1 vector backbone, which contains cytomegalovirus promoter (Invitrogen). In addition, the vector backbone was further modified to include Maz4 (transcriptional pause site) sequence to reduce cryptic cis-splicing between vector ampicillin gene and PTM 3' splice site. PTMs used for functional studies mAlbPTM97C2 and mAIbPTM158 were generated by cloning 279 bp and 149 bp BD sequence into the PTM cassette between NheI and SacII sites and were verified by sequencing.

### MOUSE ALBUMIN MINIGENE TARGET PRE-mRNA

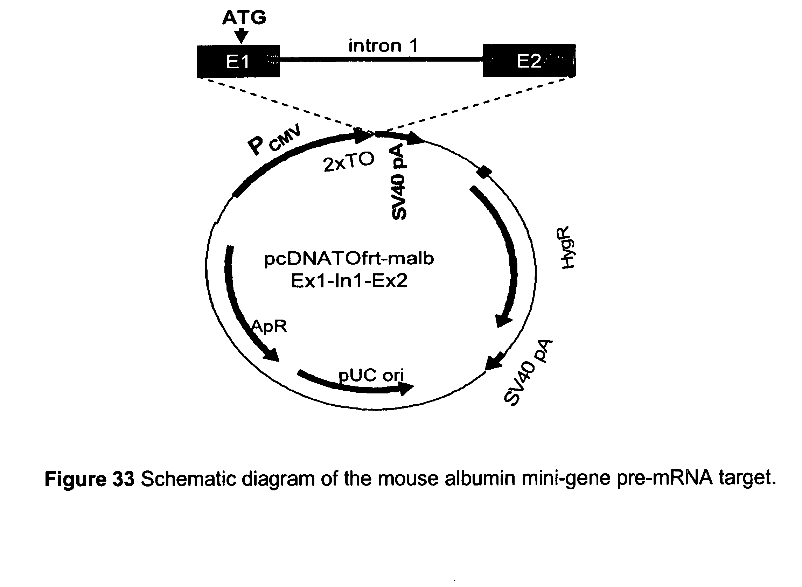

For demonstrating *in vitro* Apo A-1 function, a mouse albumin mini-gene target consisting of exon 1, intron 1 and exon 2 was used. A schematic diagram of the pre-mRNA target is shown in Figure 33. The mouse albumin coordinates are as described in Ref Seq. NC_000071. The mouse albumin Ex1-In1-Ex2 pre-mRNA target (mAlbEx1-In1-Ex2) constructed as follows: 877 bp fragment corresponding to nucleotides 1 through 877 was PCR amplified using the following mouse genomic DNA and primers: mAlb-Ex1F (SEQ ID NO:23) (5'- ctag**GCTAGC** ACCTTT CCTATCAACCCCACTAGC) and mAlb8 (SEQ ID NO:24) (5'- ctag**GCGGCCGC** AGGCCTTTGAAATGTTGTTCTCC). These primers contain unique restriction sites at the end of the fragment (indicated in bold). The PCR product was digested with Nhe I and Not I and cloned into inducible expression vector pcDNA5/FRT/TO designed to use with Flip-In T-Rex system (Invitrogen). The final construct (pcDNATOfrt-mAlbEx1-In1-Ex2) contains the following features: CMV promoter, Tet operator, SV40 polyadenylation site and hygromycin selection marker for establishing stable cell lines.

### GENERATION OF A STABLE CELL LINE EXPRESSING ALBUMIN TARGET

Using the target plasmid described above, a stable target cell line that expressed the mouse albumin mini-gene target consist of exon 1, intron 1 and exon 2 was generated. Analysis of total RNA from cells transfected with target plasmid (pcDNATOfrt-mAlbEx1-In1-Ex2) by RT- PCR produced the expected cis-spliced product, but no albumin protein. Upon confirming the splicing pattern of mouse albumin mini-gene target pre-mRNA, a stable cell line in Flip-In T-Rex 293 cells was established by transfecting the target plasmid followed by hygromycin selection. After selecting for a period of ~2 weeks, hygromycin resistant clones were pooled and maintained in hygromycin until used.

### EFFICIENT TRANS-SPLICING OF HUMAN APO A1 PTMs

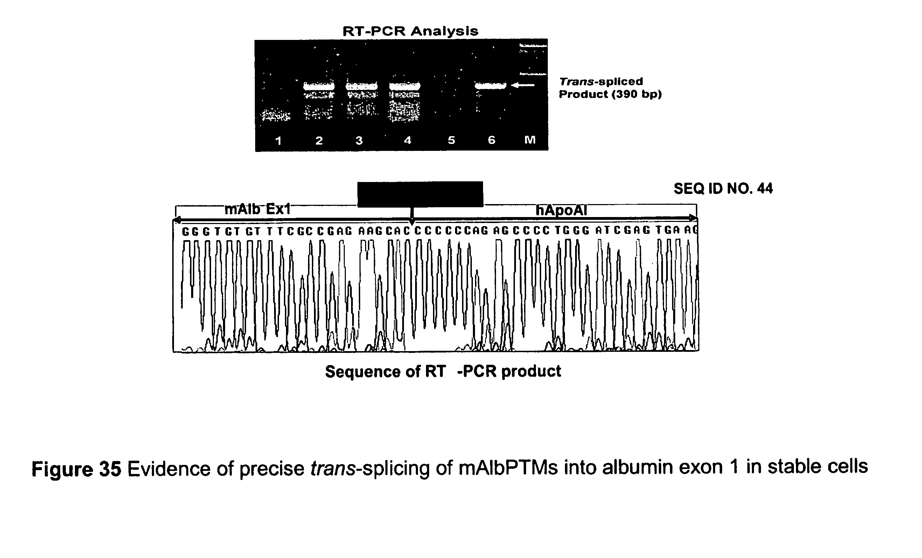

Human Apo A-1 PTMs selected from the HCS shows efficient and accurate *trans*-splicing to mouse albumin pre-mRNA in stable cells. PTM mediated *trans*-splicing and production of mouse albumin-human Apo A-1 chimeric mRNA was evaluated by transfecting stable cells with mAlbPTM97C2 and mAlbPTM158, along with a splice mutant lacking the TSD (splice incompetent PTM) and mock transfection. Total RNA isolated from these cells was analyzed by RT-PCR using mouse albumin target and human Apo A-1 PTM specific primers. These primers produced the predicted 390 bp product only in cells that received functional PTMs (Figure 35, lanes 2-4 and 6). No such product was detected in cells transfected with the splice mutant or in mock transfection (Figure 35, lane 1 and 5). The PCR product was purified and was directly sequenced, confirming the precise *trans*-splicing to the predicted splice sites of the PTM and the target pre-mRNA in stable cells (Figure 35).

Real-time quantitative RT-PCR was used to quantify the fraction of mouse albumin pre-mRNA transcripts converted into chimeric mRNAs by PTMs. Primers for real-time qPCR were designed to discriminate between target exon 1 and *trans*-spliced mRNAs. Using the protocols described previously, *trans*-splicing efficiency of mAlbPTM97C2 and rnAlbPTM158 was quantified.

Mouse albumin specific PTMs 97C2 and 158 showed a *trans*-splicing efficiency of 5.6% and 3.45%, respectively. These data confirmed robust *trans*-splicing between mouse albumin mini-gene target pre-mRNA and PTMs in stable cells.

### TRANS-SPLICING AND PRODUCTION OF FULL-LENGTH PROTEIN

The PTM-mediated *trans*-splicing was assessed for the ability to produce full-length mouse albumin-human Apo A-1 fusion protein in stable cells. Briefly, assay cells expressing the mouse albumin mini-gene pre-mRNA was transfected with mAlbPTMs (97C2 and 158), human albumin-Apo A-1 fusion as a positive control, and splice mutant with a point mutation (G>T) at splice junction as a negative control. Cells were washed after 5 hrs with serum free media and incubated with advanced DMEM serum free media. After 48 hrs, the media was collected, concentrated and analyzed by Western blot. Production of full-length human Apo A-1 protein was demonstrated using anti-human Apo A-1 antibody as described above.

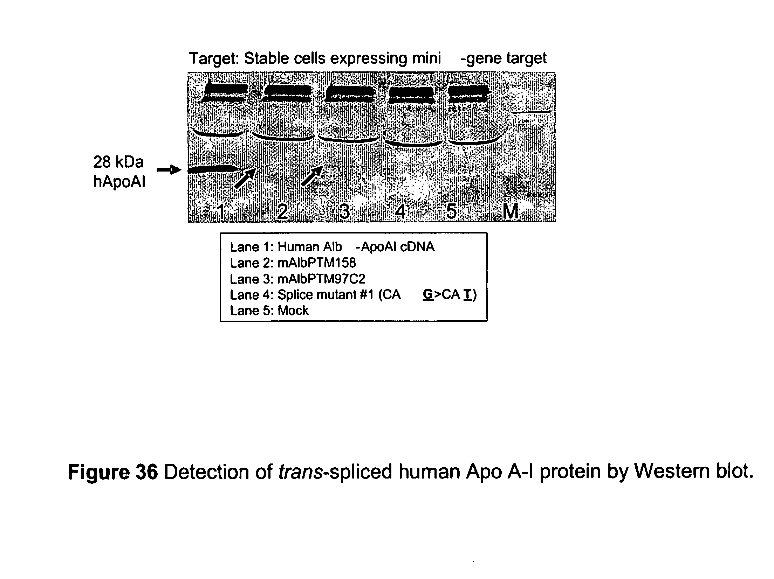

Accurate *trans*-splicing between mouse albumin exon 1 and PTM would result in a 28kDa albumin-human Apo A-1 fusion protein. *Trans*-splicing mediated production of full-length mature human Apo A-1 protein is evident in cells transfected with functional PTMs (97C2 and 158) (Figure 36, lanes 2-3) but not in controls i.e., cells transfected with a splice mutant or in mock (Figure 36, lanes 4-5) and it also co-migrated with the albumin-Apo A-1 fusion protein produced using cDNA control plasmid (Figure 36, lane 1-3). These studies again confirmed precise *trans*-splicing between the mouse albumin exon 1 and human Apo A-1 PTMs, resulting in the production of fusion albumin-human Apo A-1 protein in stable cells.

### EXAMPLE 9: TRANS-SPLICING TO ENDOGENOUS MOUSE ALBUMIN PRE-mRNA IN MICE

The efficacy of the lead PTMs selected from the high capacity screen (HCS) were evaluated *in vivo.* Fifty micrograms of mAlbPTM97C2 (PTM only) or 20 µg of mouse albumin mini-gene target plus 30 µg of mAlbPTM97C2 plasmids were mixed with *jet*-PEI-Gal (Q-Biogen) reagent and injected via tail vein into normal C57BL/6 mice. Liver and serum samples were collected at 24 and 48 hrs time points. Total and poly A mRNA was isolated and analyzed by RT-PCR using mouse albumin exon 1 specific and human Apo A-1 PTM specific primers.

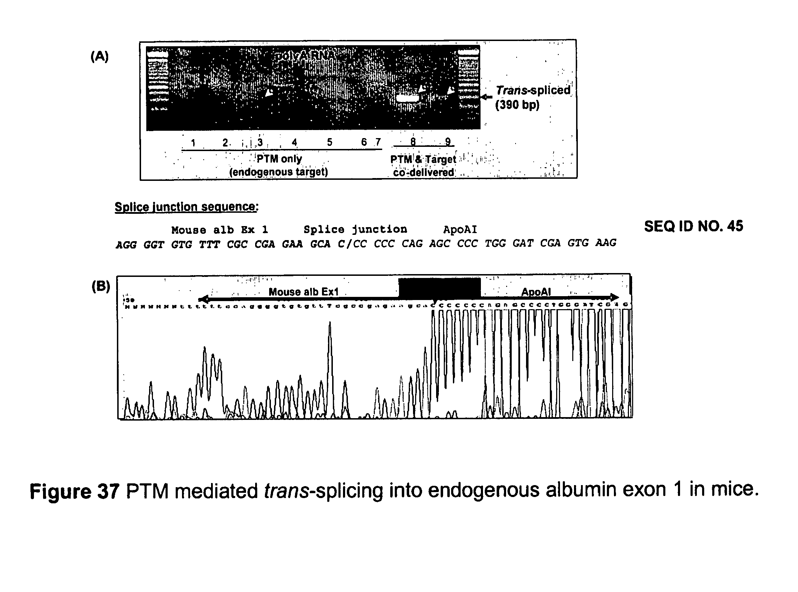

*Trans*-splicing was detected in a single round in mice that received both mini-gene target plus PTM plasmids, as well as in mice that received PTM only (Figure 37, lane 3, 8 & 9). Each positive RT-PCR product was purified and sequenced demonstrating the precise *trans*-splicing of mouse albumin exon 1 into human Apo A-1 coding sequence at the predicted splice sites (Figure 37, lower panel). These results demonstrated accurate *trans-*splicing between the PTM and the endogenous albumin pre-mRNA target in mice and further validated albumin targeting strategy *in vivo.*

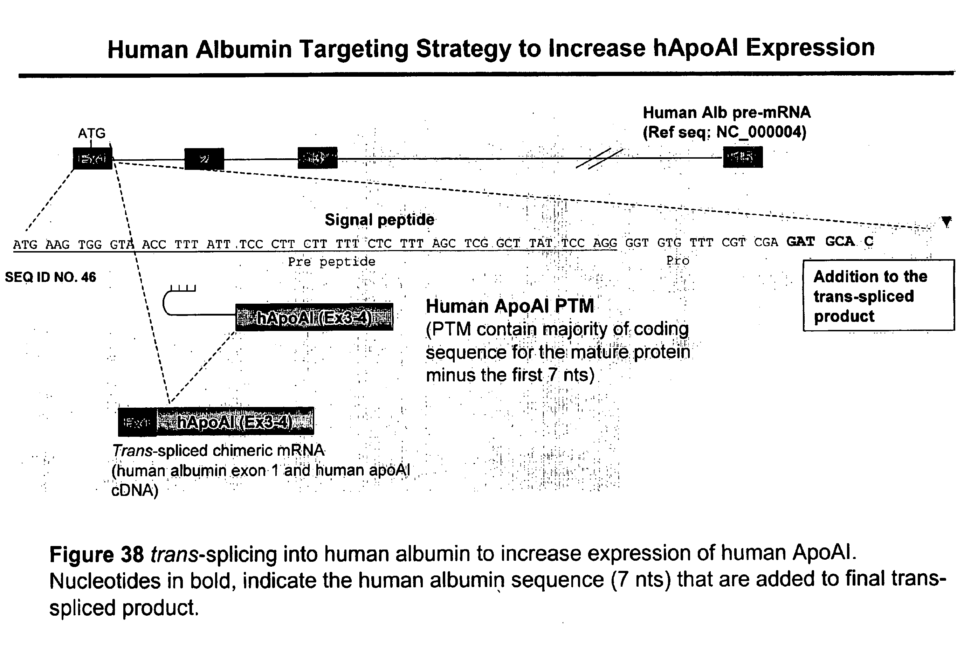

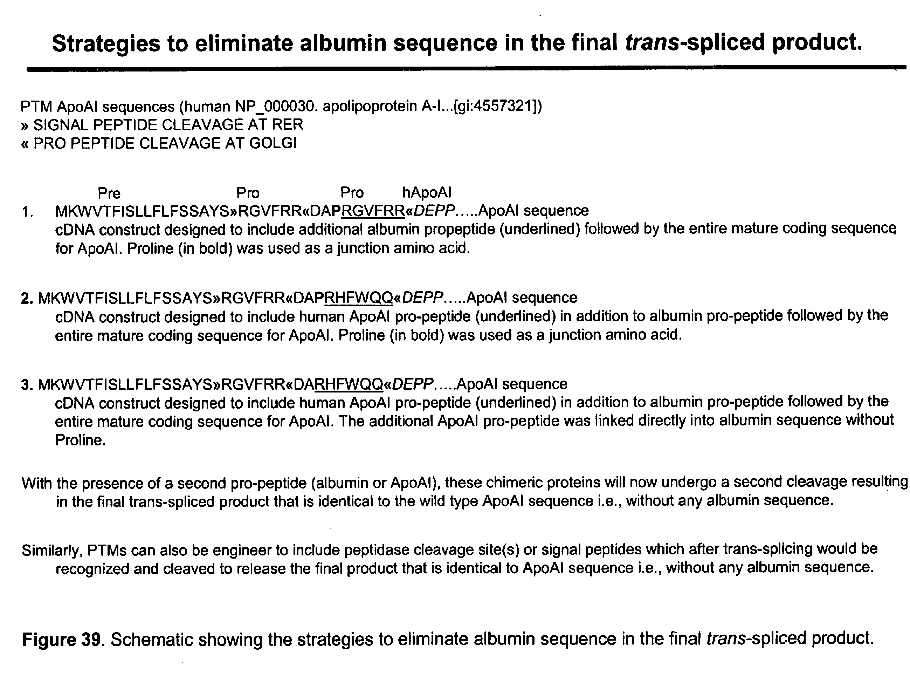

Figure 38 describes a strategy to increase ApoA1 expression by targeting to human albumin sequences. Figure 39 describes various means of eliminating albumin sequences in the final trans-spliced product, i.e. to produce a trans-spliced product that is identical to the wild type human ApoA1 without any albumin sequence.

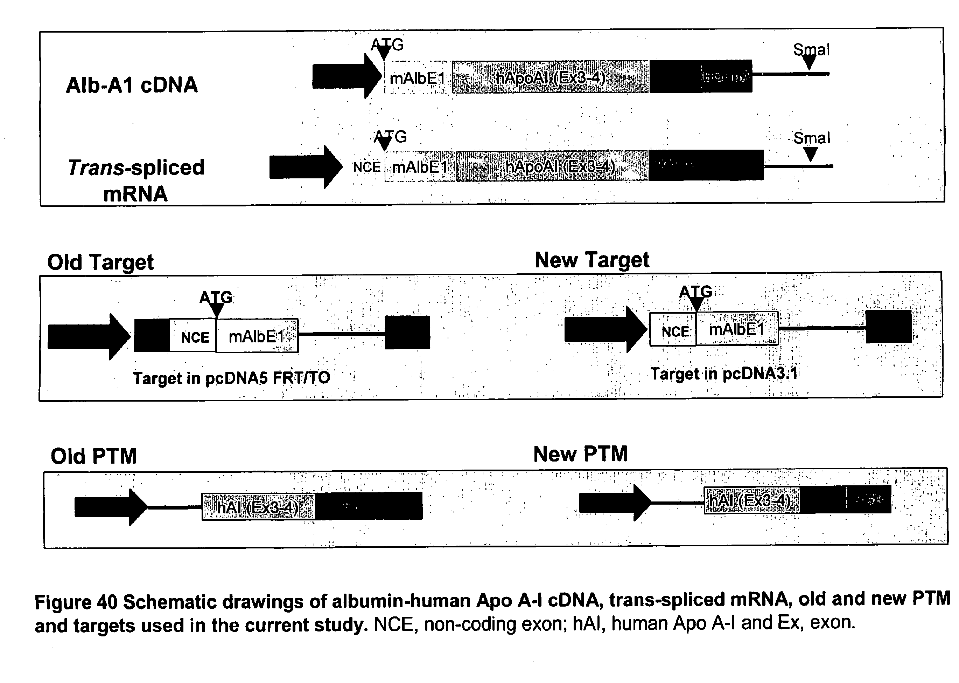

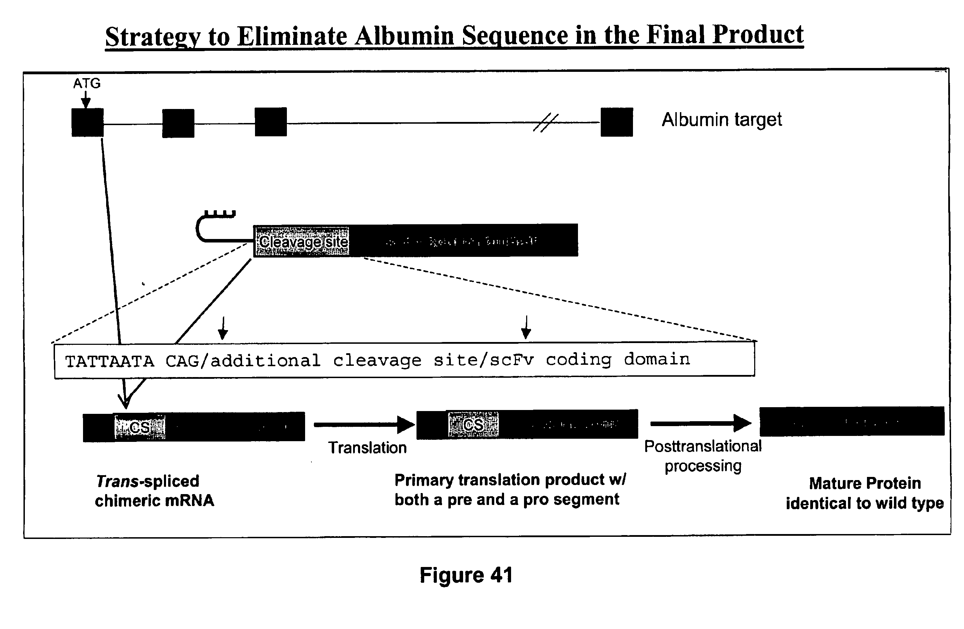

For example, the current albumin trans-splicing strategy results in the production of chimeric mRNA and protein. The final trans-spliced product contains 7 nucleotides or 2 amino acids from albumin target mRNA. For human applications it may be desirable to eliminate the albumin sequence in the final product to preclude immunological reactions. In one exemplary strategy, illustrated in Figure 40, the PTM will be engineered to encode "Furin" like endopeptidase (or pro-protein convertase) cleavage site which has been used to express proteins *in vivo* (Fuller RS, Brake AJ, Thorner J, Science, 246: 482-486, 1989; Bresnahan PA, Leduc R, Thomas L, Thorner J, Gibson HL, Brake AJ, Barr PJ, Thomas G., J Cell Biol. 111:2851-2859, 1990; van de Ven WJ, Voorberg J, Fontijn R, Pannekoek H, van den Ouweland AM, van Duijnhoven HL, Roebroek AJ, Siezen RJ, Mol Biol Rep. 14:265-75, 1990; Duckert P, Brunak S, Blom N. Protein Eng Design & Selection. 17:107-112, 2004). In another example, the PTM will be designed to include the protein's own native secretion signal, i.e., "pre-pro" signal (if it has one). This strategy was designed to take advantage of the endogenous native cellular machinery to enhance recognition, processing and secretion of the final *trans*-spliced protein to the site of action similar to wild type protein. For example, trans-splicing of PTM into albumin pre-mRNA target produces a chimeric mRNA and pre-pro-protein that in addition to signal peptide cleavage in rough endoplasmic reticulum undergoes several post-translational modifications in other cellular compartments and, finally, endopeptidase cleavage resulting in the release of a mature, fully processed biologically active protein that is identical to the wild type. (Figure 41).

### EXAMPLE 10: IN VITRO AND IN VIVO PRODUCTION OF RECOMBINANT PROTEINS BY TRANS-SPLICING INTO HIGHLY ABUNDANT TRANSCRIPTS

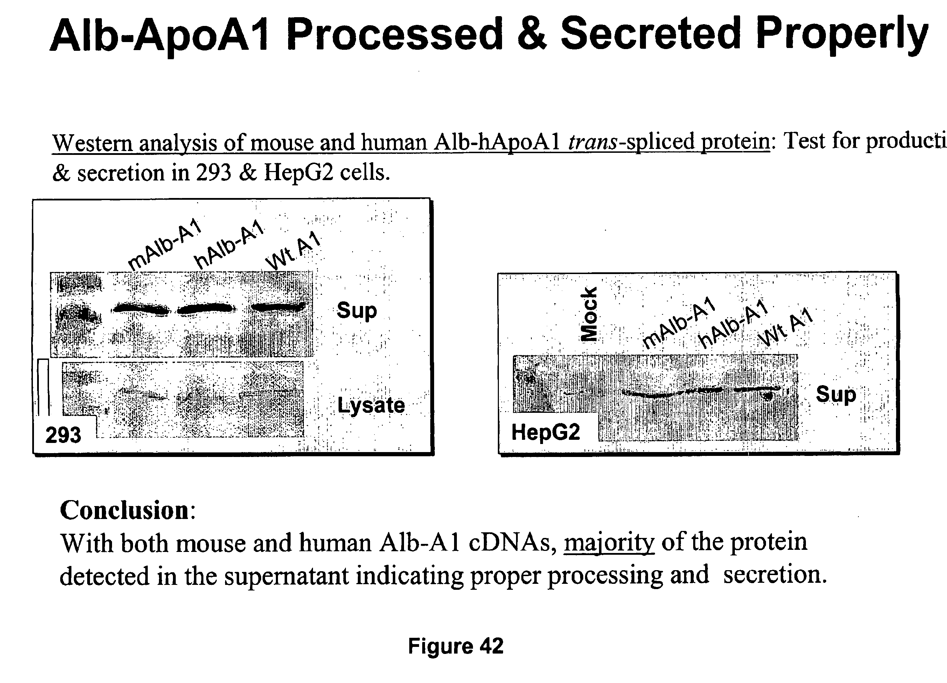

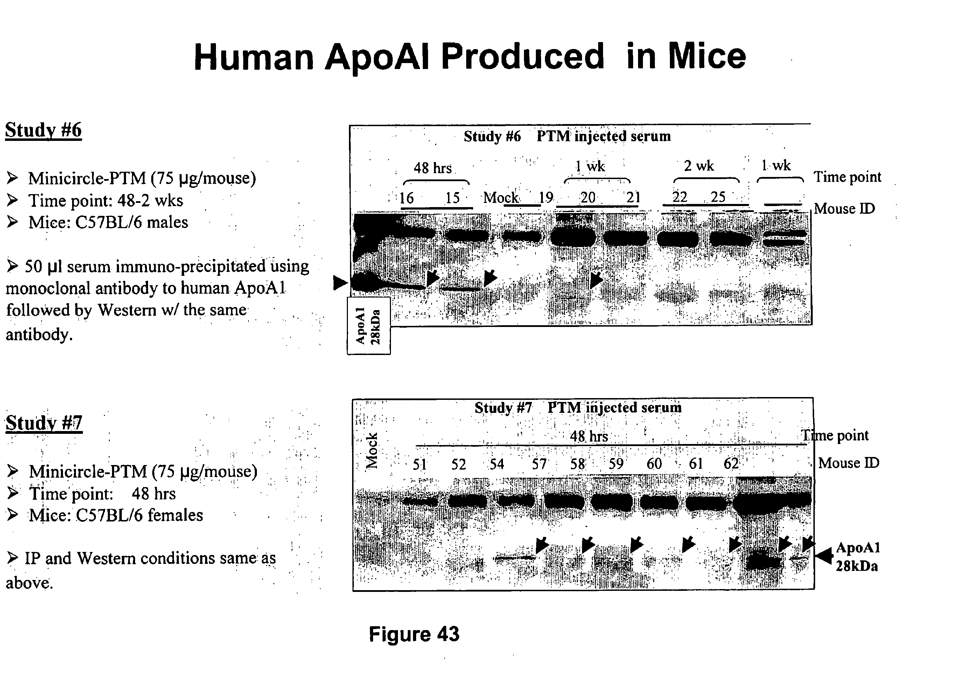

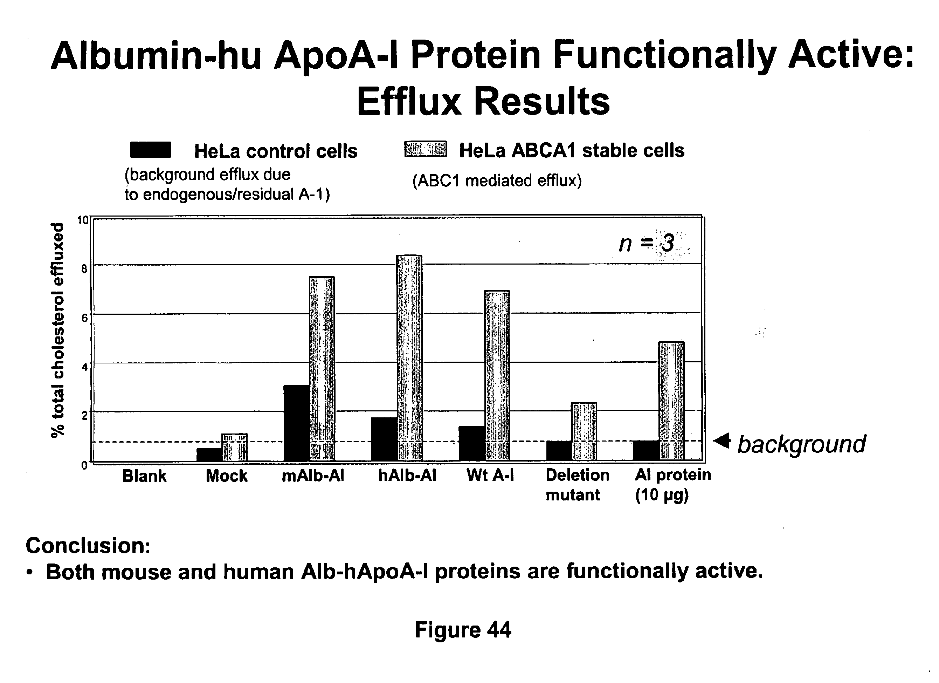

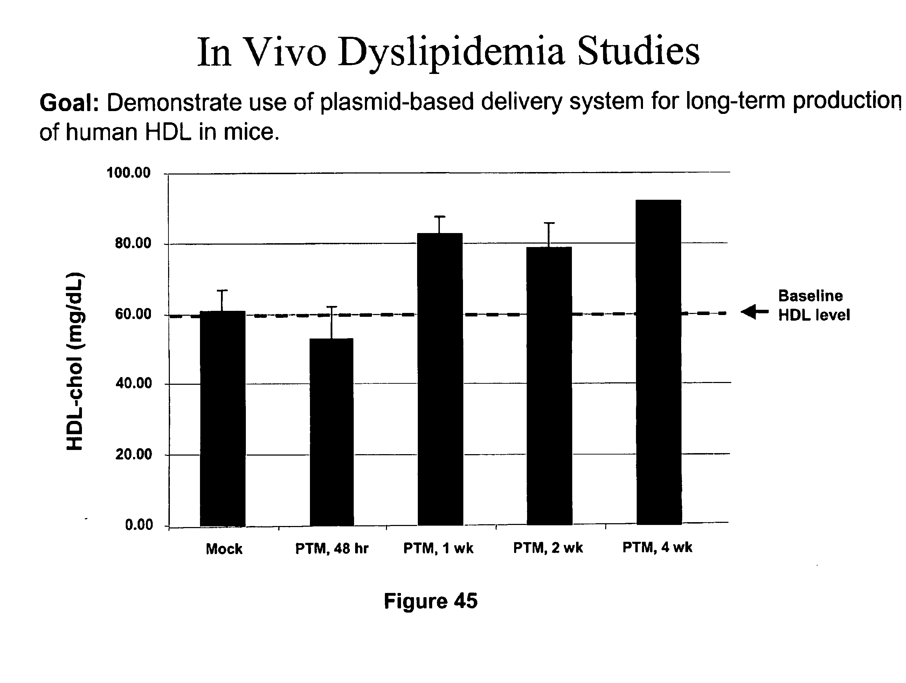

Three examples of *in vivo* protein production as a result of *trans*-splicing into albumin pre-mRNA have been demonstrated. The first is production of human ApoA-I. These studies have shown that human ApoA-I was produced in mice as evidenced by gene expression and secretion (Figure 42), identification of ApoA-I by Western blot analysis (Figure 43), and functionality as determined by cholesterol efflux assays (Figure 44). More appropriately, this *in vivo* production of human ApoA-I resulted in an increase of HDL in three separate experiments, each experiment increasing HDL by 20-25mg (Figure 45).

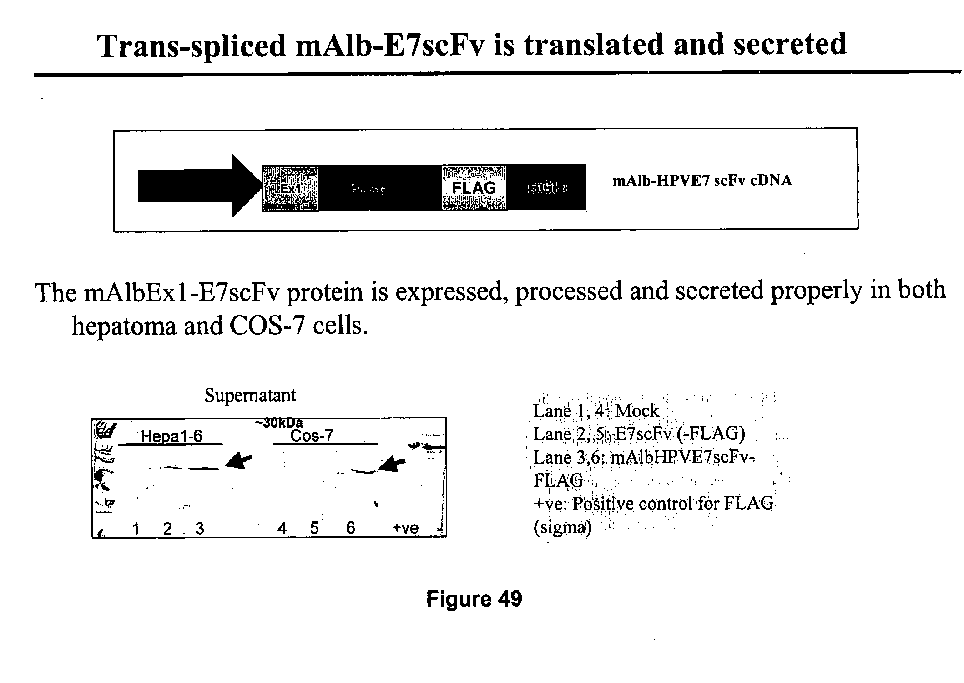

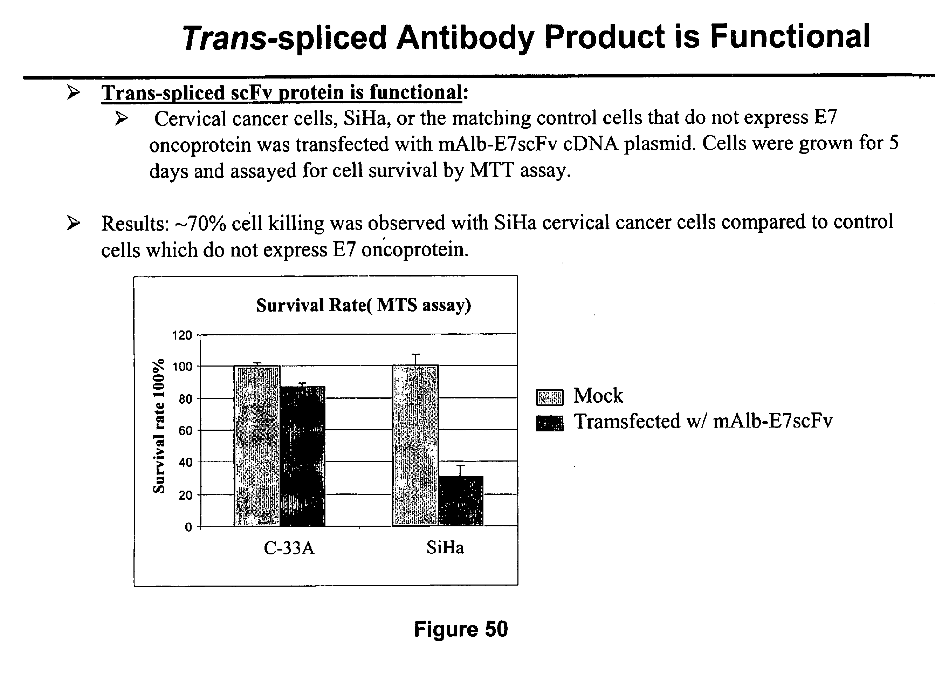

As a second example, gene sequences for a single chain antibody were encoded in the PTM and delivered by plasmid. In these studies, a single chain monoclonal antibody directed against E7 of human papillomavirus (HPV) was utilized. Results showed that the antibody was expressed and secreted (Figure 49). In addition, this *in vivo trans-*spliced product was effective in inhibiting the growth of HPV-infected cells (Figure 50). This study effectively reproduced the results of Wang-Johanning et al. (Cancer Research 58:1893-1900, 1998). These results address the challenge of monoclonal antibody commercialization: cost of production and achieving therapeutic levels on antibodies in the plasma, on the order of 3-30ug/ml, as reviewed by Bakker et al. (J. Mol. Ther. 10:411-416, 2004).

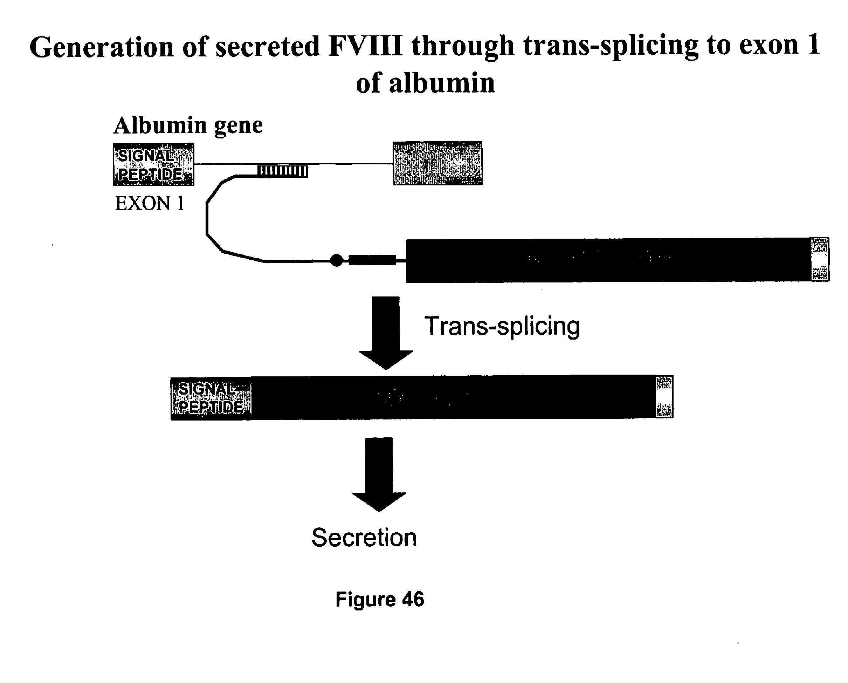

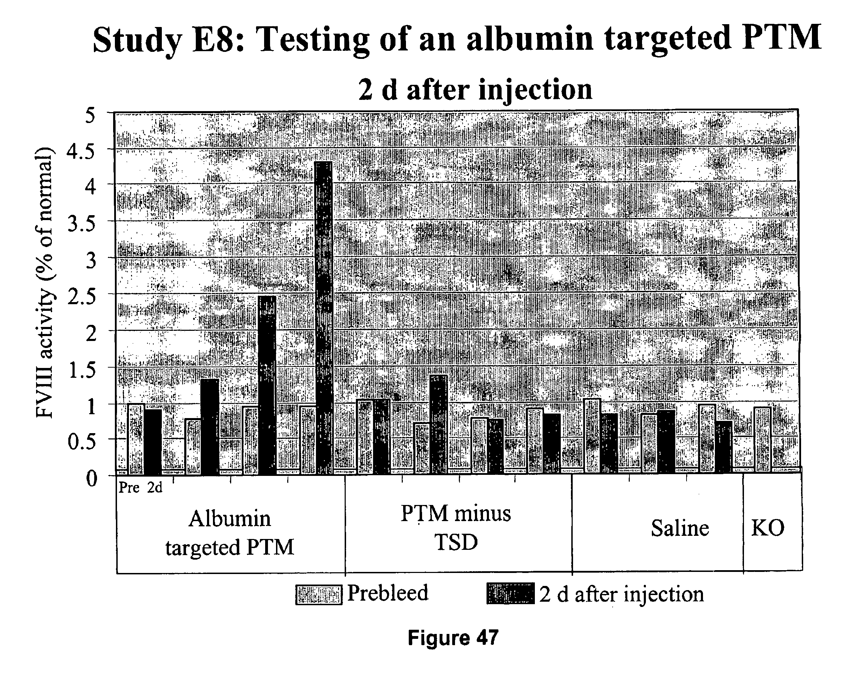

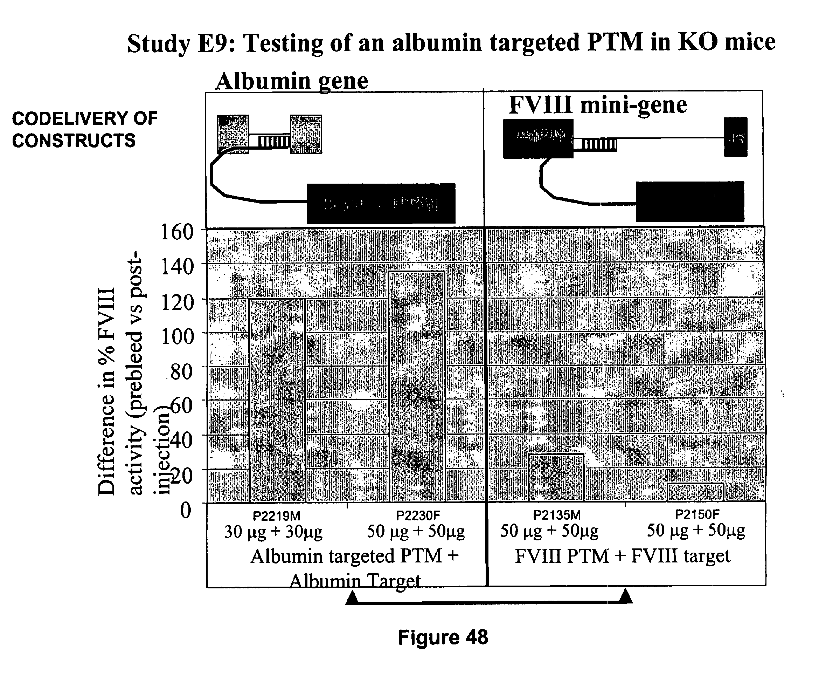

In a third example, a PTM targeting intron one of mouse albumin and encoding mouse Factor VIII was administered intravenously to Factor VIII knockout mice as shown in Figure 46. These mice have essentially zero Factor VIII levels in their plasma. Results of initial studies are shown in Figure 47 and demonstrate significant production of Factor VIII. Uninoculated controls as well as animals that received a PTM that were defective in splicing showed no Factor VIII. (*See* Figure 47) In subsequent studies, animals were inoculated with both albumin minigene target and the PTM encoding Factor VIII and these animals demonstrated significant plasma levels of Factor VIII (Figure 48). As a control, the Factor VIII minigene was used. Use of the albumin pre-mRNA was significantly better than the Factor VIII target.

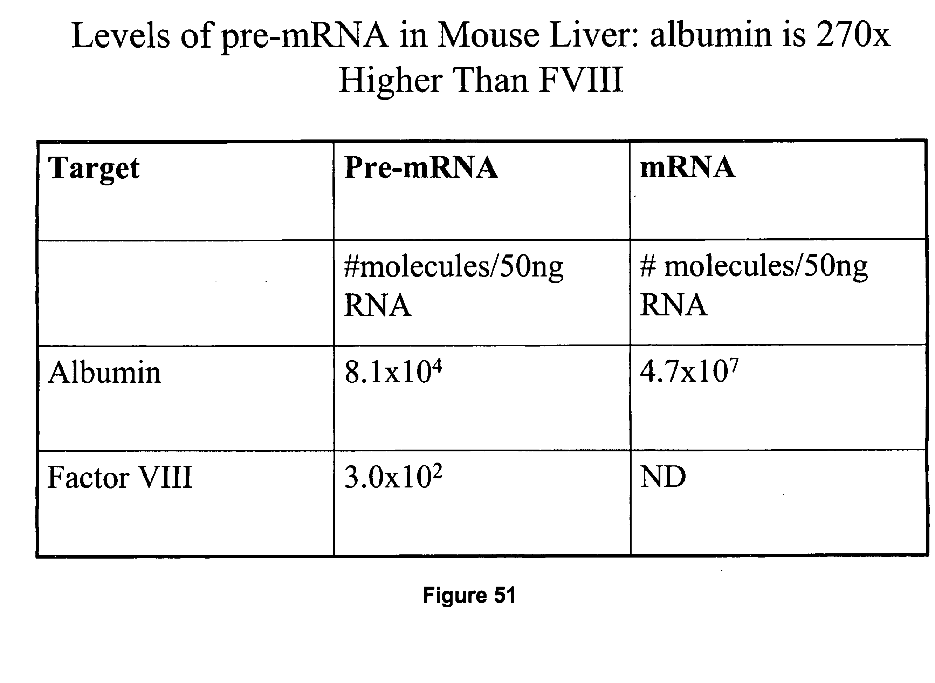

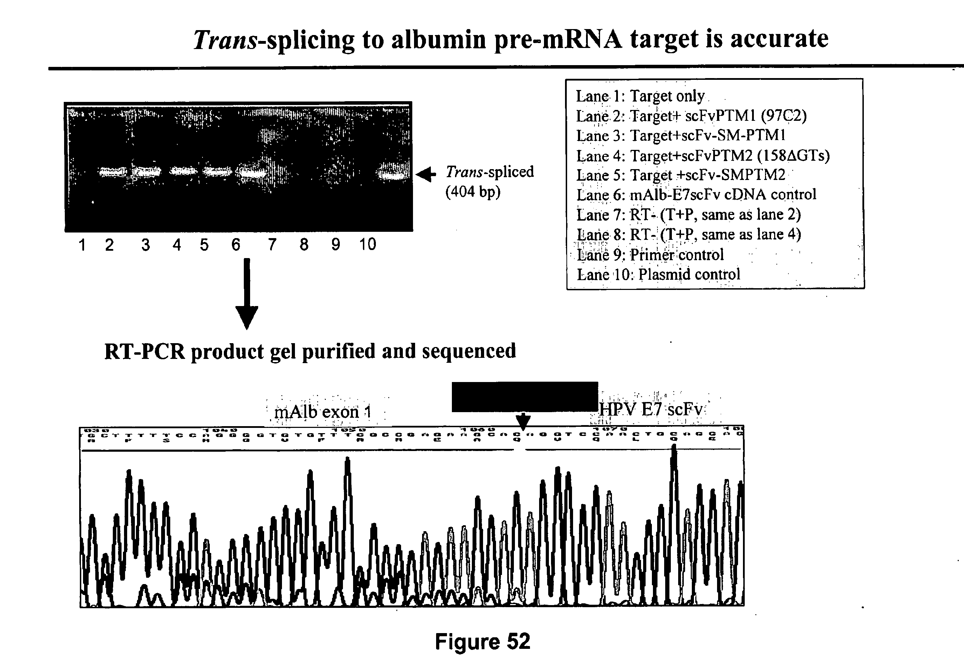

Additionally, the relative levels of mouse Factor VIII pre-mRNA and mouse albumin pre-mRNA were determined. The results are shown in Figure 51 and illustrate that the levels of albumin pre-mRNA are 270x more abundant than Factor VIII transcript, demonstrating the validity of *trans*-splicing into a highly abundant transcript. In each of these three studies, the PTM utilized the same binding domain, that of mouse albumin. The PTMs utilized three respective coding domains: human ApoA-I, mouse Factor VIII and a single chain monoclonal antibody against HPV. It has also been shown that the PTM encoding the single chain monoclonal antibody *trans*-splices specifically into the precise nucleotide sequence in mouse albumin pre-mRNA (Figure 52). It has also been separately shown that the same binding domain that encodes human ApoA-I also specifically *trans-*splices into the same nucleotide of the target (not shown).

This data demonstrate that PTMs targeting a highly abundant pre-mRNA can produce significant and therapeutic levels of recombinant proteins *in vitro* and *in vivo* and, as shown in the case of Factor VIII, can produce significantly higher plasma levels of protein than *trans*-splicing into homologous targets.

### 11 EXAMPLE: IN VITRO TRANS-SPLICING OF HUMAN APOAI INTO MOUSE ALBUMIN PRE-mRNA: FUNCTIONALITY OF THE PRODUCT

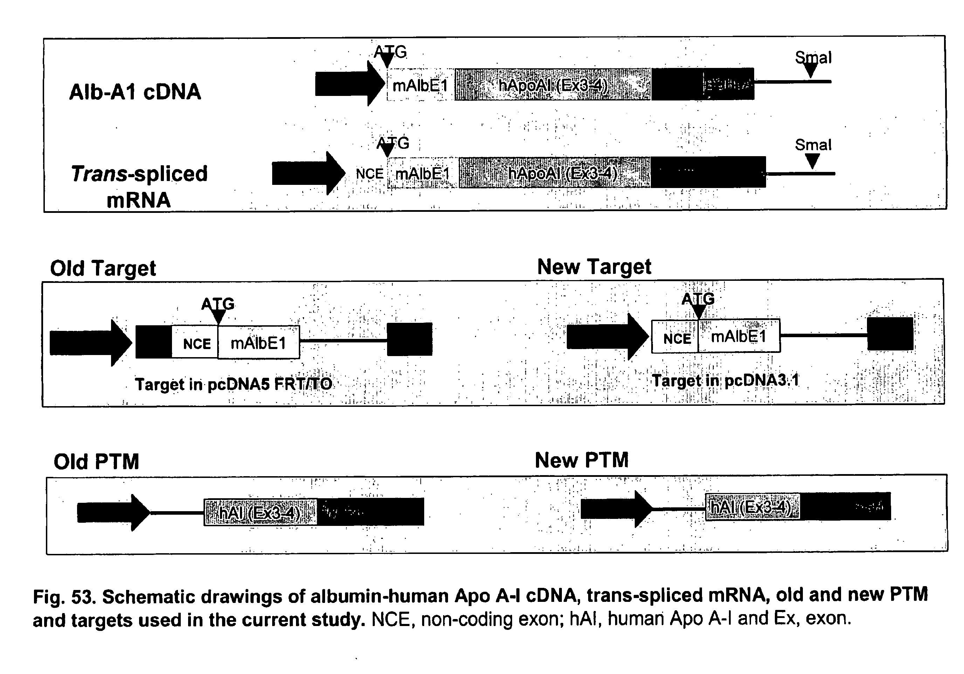

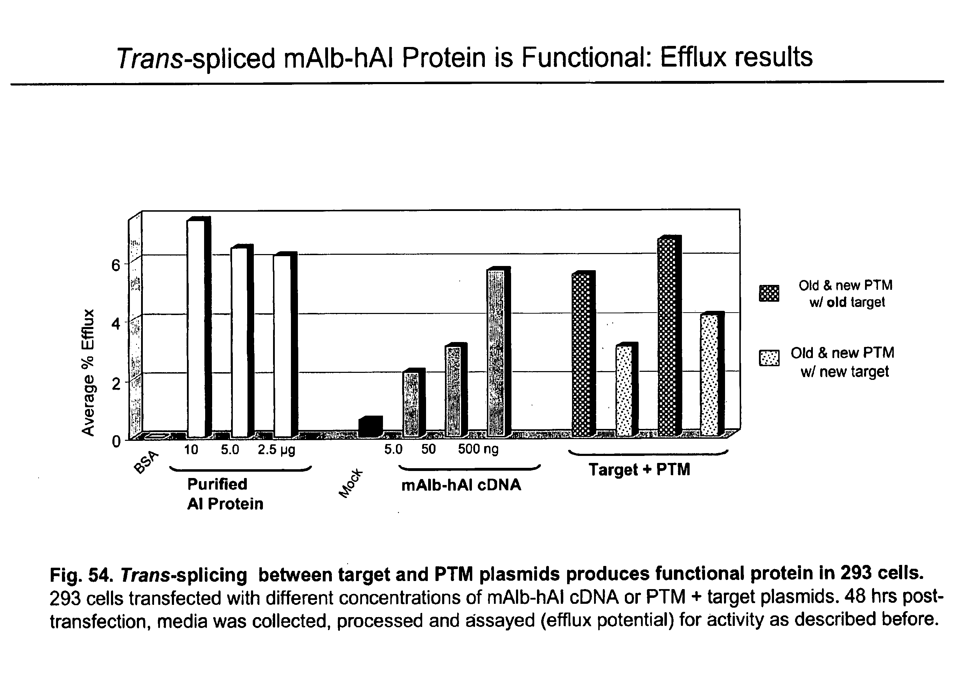

The function of the human apoAI protein produced through trans-splicing of human apoAI into mouse albumin pre-mRNA has been evaluated *in vitro.* This was assessed by measuring, ATP-binding cassette transporter protein (ABC1) mediated, transfer of cellular cholesterol into apoAI acceptor. The release of radio-labeled cellular cholesterol to lipid free human apoAI was quantified and the efflux values obtained with *trans*-spliced protein were compared with that from wild type human apoAI protein. Human embryonic kidney cells (HEK293) were transfected with mouse albumin PTM (mAlbPTM97C2) containing either: the apoAI natural 3'UTR + bovine growth hormone poly A signal (BGH pA); or WPRE 3'UTR + SV40 poly A signal (SV40 pA) along with mouse albumin mini-gene targets (Figure 53). 48 hrs post-transfection, supernatant was collected, concentrated and assayed for cholesterol efflux. HeLa cells transfected with ABC1 plasmid and HeLa control cells were grown to near confluency. Cells were then loaded with 1 µCi/ml ³H cholesterol. After equilibrating for 24 hrs, the cells were washed and incubated with media containing the i*trans*-spliced human apoAI protein (supernatant from HEK293 cells transfected with PTM+target or cDNA control plasmid that mimics trans-splicing) or with different concentrations (2.5 µg, 5 µg, or 10 µg) of wild type purified apoAI protein as positive control. Cells were then allowed to efflux for 18 hrs. After the efflux period, medium was collected and an aliquot was then counted by liquid scintillation counting. The remaining counts in the cell fraction were determined after an over night extraction with isopropanol. The percent efflux was calculated by dividing the counts in the efflux media by the sum of the counts in the medium plus the cell fraction. DMEM/BSA medium was used as a blank and was subtracted from the radioactive counts obtained in the presence of an acceptor in the efflux media. As shown in Figure 54, the amount of ABC1-mediated efflux observed with *trans-*spliced protein was significantly above the background and was similar to that of wt apoAI produced from control cDNA plasmid. The above described results indicate (a) that human apoAI protein produced through *trans*-splicing is functional and (b) the absence of adverse effects due to albumin sequence in the final *trans*-spliced mRNA on apoAI function.

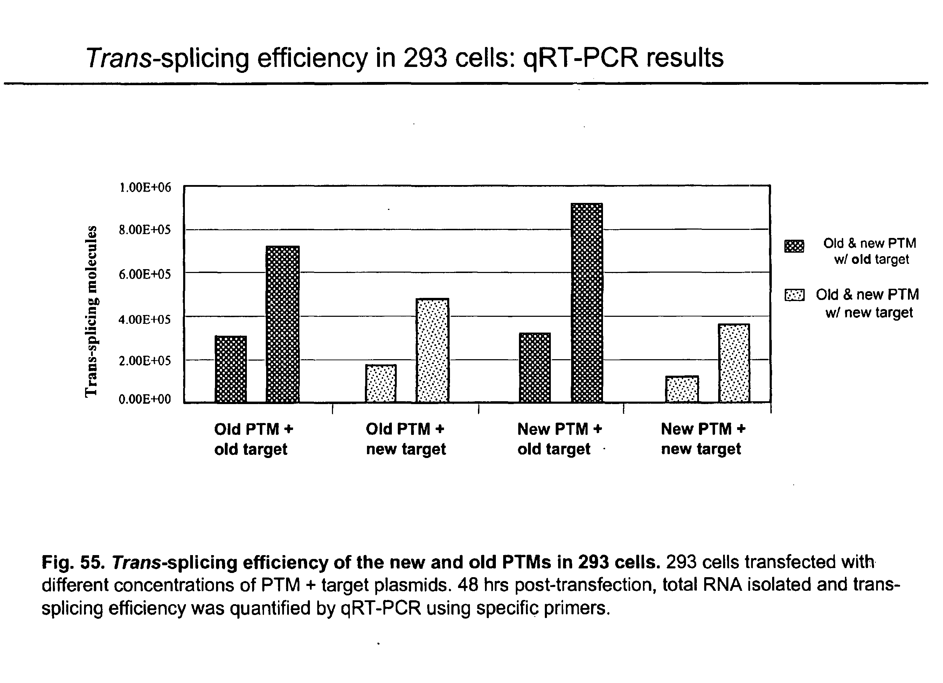

*Trans*-splicing efficiency at the RNA level was quantified by real time RT-PCR (qRT-PCR) and the results are shown in Figure 55. Based on qRT-PCR results it is clear that both PTMs, *i.e*., PTM with apoAI natural 3'UTR plus bovine growth hormone (BGH) poly A signal (new PTM) and the PTM with WPRE 3'UTR plus SV40 pA signal (old PTM), showed similar *trans*-splicing efficiency at the RNA level. The accuracy of *trans-*splicing was confirmed by direct sequencing of the RT-PCR product.

### 12 EXAMPLE: IN VIVO TRANS-SPLICING OF HUMAN APOAI INTO MOUSE ALBUMIN PRE-mRNA

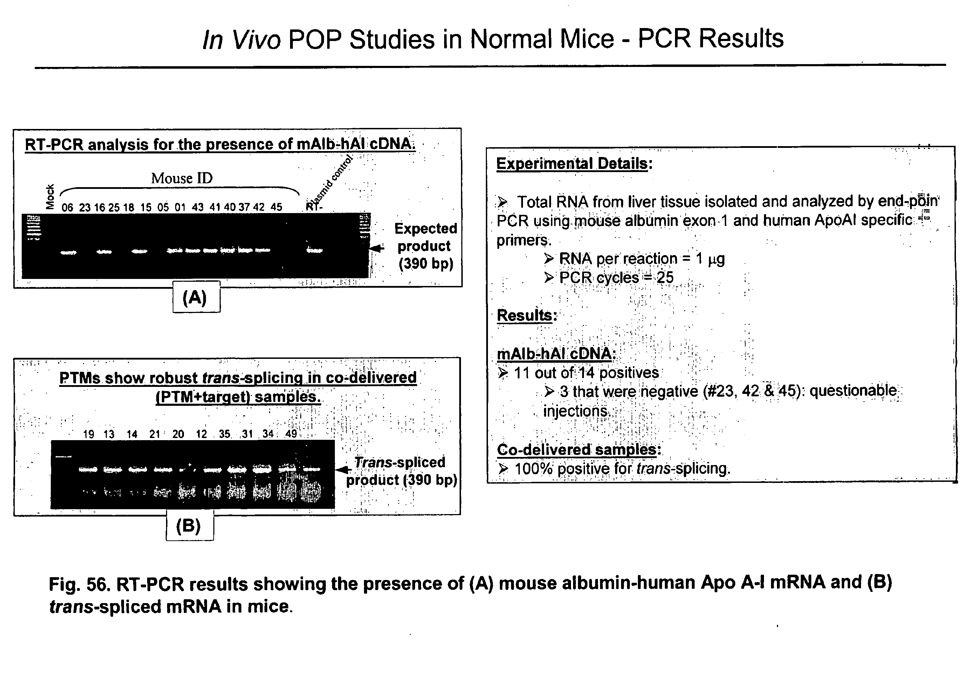

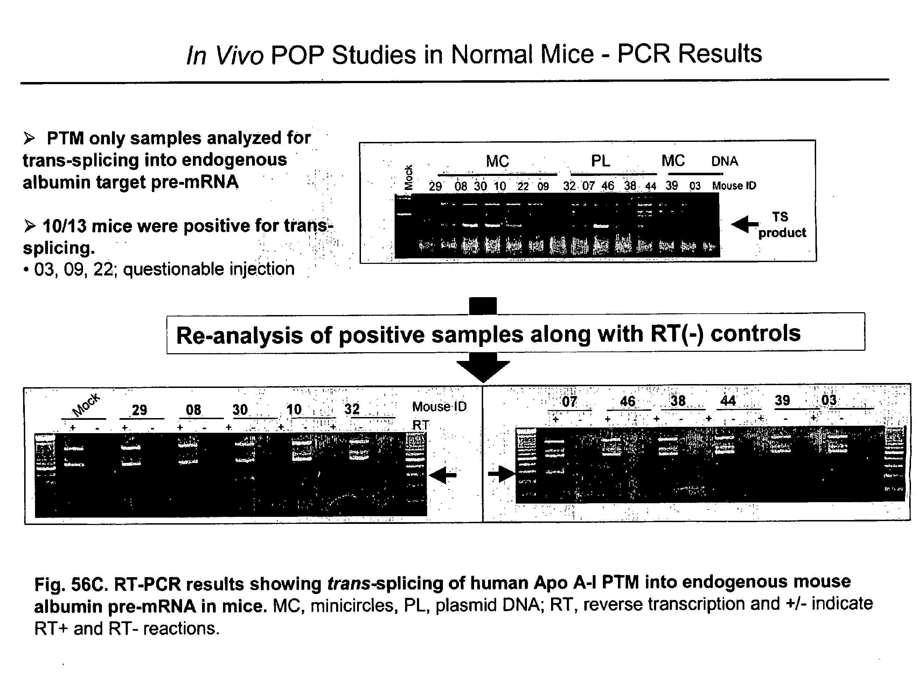

*Trans*-splicing to an endogenous mouse albumin pre-mRNA target has been shown to produce human apoAI protein and HDL in mice. In particular, to verify the efficacy of the lead PTMs selected from high capacity screen (HCS) and to demonstrate *trans*-splicing of PTM into endogenous mouse albumin target followed by production of human apoAI protein, the following experiment has been performed. Fifty micrograms of: mAlbPTM97C2 (PTM only); 30 µg of PTM + 15 µg of mini-gene target (additional target plasmid to increase pre-mRNA concentration); or 20 µg of control cDNA plasmid that mimic *trans*-spliced mRNA were hydrodynamically injected via the tail vein into normal C57BL/6 mice. Liver and serum samples were collected at 8, 16, 24 and 48 hrs time points. Total and polyA mRNA was isolated and analyzed by end point RT-PCR using mouse albumin exon 1 specific (SEQ ID NO:25) (ACCTTTCTCCTCCTCCTCTTCGT) and human apoAI PTM specific primers (SEQ ID NO:26) (ACATAGTCTCTGCCGCTGTCTTT). As shown in Figure 56A the presence of *trans*-spliced chimeric mRNA was detected in 11 out of 14 mice that were injected with cDNA control plasmid, indicating good delivery of the plasmid DNA. Next, PTM *trans*-splicing to endogenous mouse albumin pre-mRNA target was evaluated using the target and PTM specific primers as described above. *Trans*-splicing between mouse albumin target pre-mRNA and PTM was readily detected in a single round of PCR with 1 µg of total RNA and 25 cycles of amplification. All samples from mice that received both the mini-gene target and the PTM plasmids were positive for *trans*-splicing (Fig. 56B). In comparison, 10 out of 13 mice were positive for *trans*-splicing that received the PTM only (Fig. 56C). Each positive RT-PCR product was purified and sequenced demonstrating precise *trans*-splicing of human apoAI coding sequence into mouse albumin exon 1 at the predicted splice sites. These results demonstrate accurate *trans*-splicing between the PTM and the endogenous albumin pre-mRNA target in mice and further validate albumin targeting strategy for the production of therapeutic proteins *in vivo.*

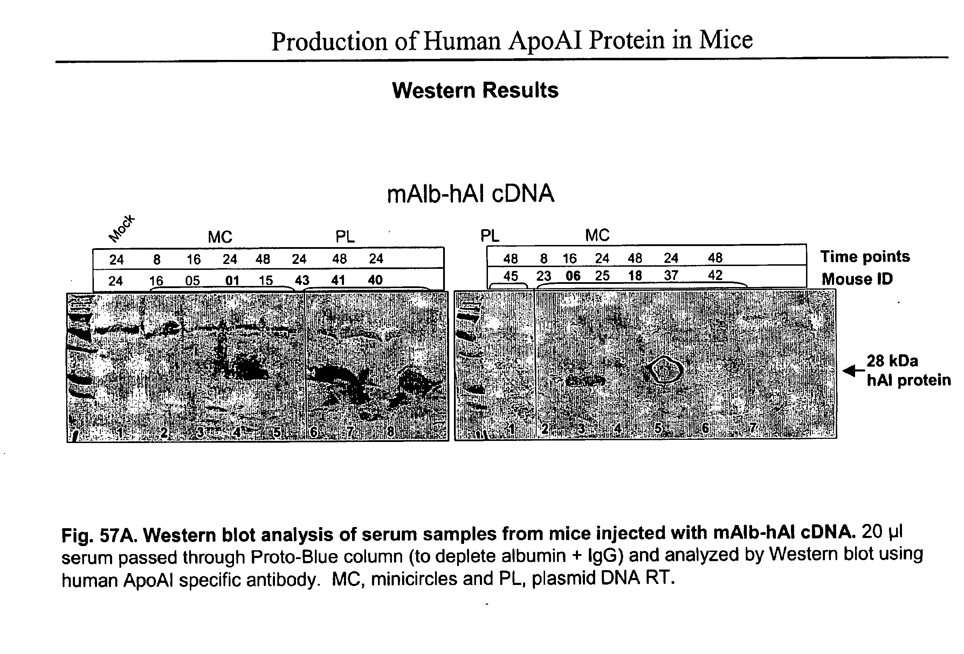

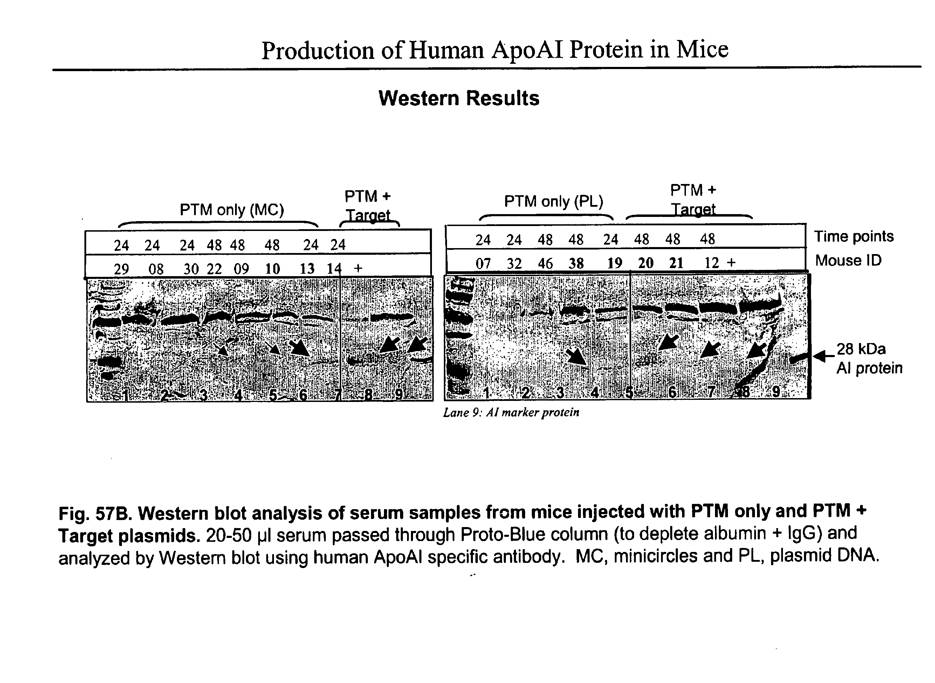

In addition, accurate *trans*-splicing to the endogenous mouse albumin pre-mRNA target to produce human apoAI protein in mice was demonstrated. Serum samples were collected from mice injected with PTM only, PTM+target and cDNA for the production of human apoAI protein were tested by Western blot. Approximately, 20-50 µl serum was passed through ProteoPrep^{™}Blue affinity column (Sigma-Aldrich, Product Code PROT BA). This step was introduced to eliminate albumin and IgGs which make up greater than 70% of the proteins in serum and to increase sample loads to better visualize lower abundant proteins. Samples separated by 12% SDS-PAGE were transferred to nitrocellulose membranes and probed with a human specific apoAI monoclonal antibody (Biodesign International, Cat # H45625M). Proteins were visualized by a chemiluminescence kit (Invitrogen, Cat# WB7103). Western blot results indicated the appearance of human apoAI protein as early as 16 hrs post-injection in mice injected with cDNA control plasmids. In this group, 7 out of 14 samples were positive for human apoAI protein. (Figure 57A). In mice that received both target and PTM, 5 out of 6 samples were positive for human apoAI protein. In mice that received the PTM only (targeting endogenous target), 4 out of 10 samples were positive for human apoAI protein. These results demonstrate the accurate *trans*-splicing of human apoAI sequence into endogenous mouse albumin exon 1 leading to the production of human apoAI protein (Figure 57B).

### 13 EXAMPLE: IN VIVO TRANS-SPLICING OF MINICIRCLE VECTOR DNA FOR EXPRESSION OF HUMAN APOAI PROTEIN

Minicircles are DNA vectors that lack the bacterial DNA sequence that is implicated in the silencing of gene expression *in vivo*. See, for example, Chen ZY, He CY, Ehrhardt A, Kay MA. (2003) DNA vectors devoid of bacterial DNA result in persistent and high-level transgene expression in vivo. *Mol Ther*. **8**:495-500; Chen ZY, He CY, Meuse L, Kay MA. (2004) Silencing of episomal transgene expression by plasmid bacterial DNA elements in vivo. Gene Ther. 11:856-864 the disclosures of which are hereby incorporated by reference.

Minicircles were tested by cloning the mAlbPTM97C2 expression cassette into minicircle vector. Fifty to seventy five micrograms of mAlbPTM97C2 (functional PTM), mAlbPTM97C2-splice mutant (defective PTM) or control cDNA (mimics *trans*-spliced mRNA) in the form of minicircles were hydrodynamically injected via tail vein into normal C57BL/6 mice. Liver and serum samples were collected at 48 hrs through 4 week time points. RNA analysis by qRT-PCR using mouse albumin exon 1 specific forward primer and human apoAI specific reverse primer confirmed the *trans*-splicing of mouse albumin PTM into endogenous mouse albumin pre-mRNA target. As shown in Table 1, the results obtained with a splice mutant PTM were similar to background observed in the mock group. The presence of *trans*-spliced mRNA was readily detected at 4 week post-injection indicating minicicrles can be used as a non-viral PTM delivery system.

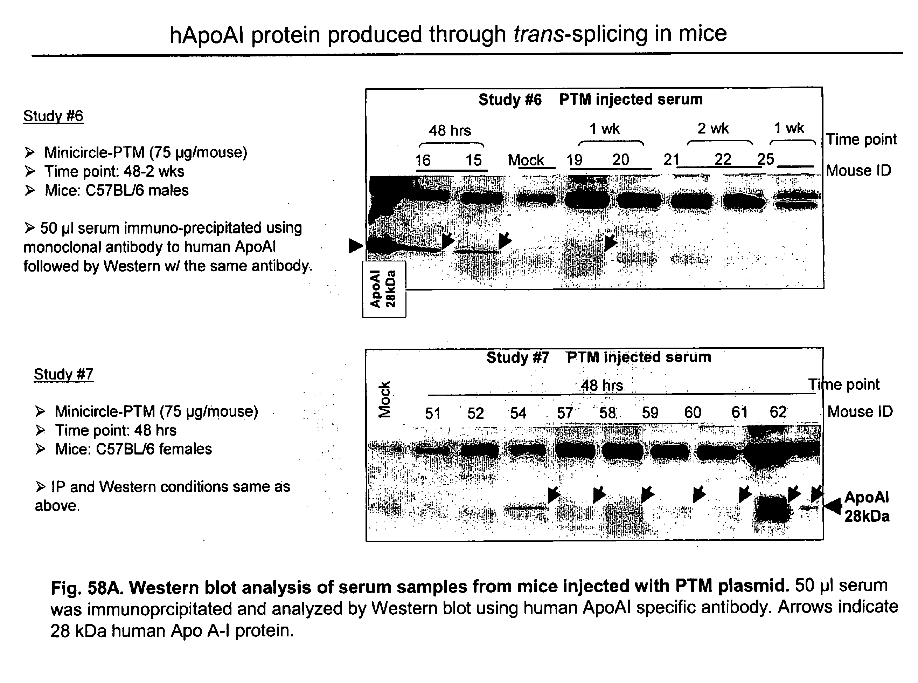

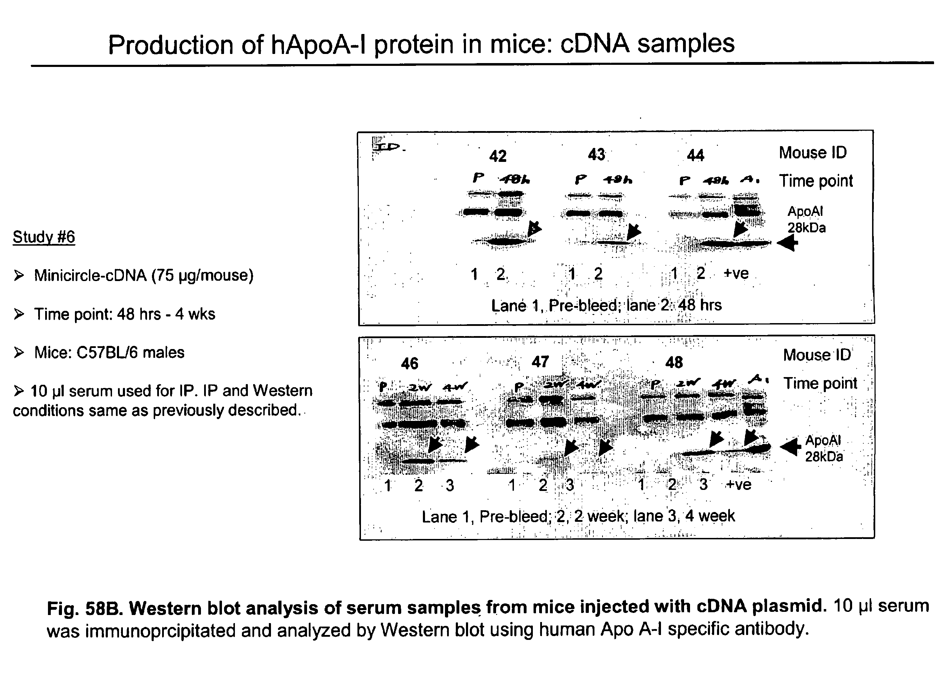

Western blot analysis of serum samples from mice injected with minicircles encoding the PTM confirmed the production of human apoAI protein through trans-splicing. Ten to fifty micro liter serum samples were immunoprecipitated using human specific apoAI antibody. After elution, samples were concentrated, analyzed on a 12% SDS-PAGE and probed with the same antibody (human specific apoAI antibody) that was used for immunoprecipitation. The blot was developed using an ECL kit (Invitrogen, Cat # WB7104). Western results clearly showed the presence of a 28 kDa protein band that co-migrated with the positive control purified apoAI protein (Fig. 58A). The presence of human apoAI protein was also detected in 4 week serum samples (Fig. 58B). These results not only confirmed the production of human apoAI protein through *trans*-splicing of PTM into endogenous mouse albumin pre-mRNA target in mice, but also demonstrated the utility of minicircles as a non-viral PTM delivery system.

**Table 1. Trans-splicing in mice - qRT-PCR Results**

| | | | | | |
|---|---|---|---|---|---|
| | | | | | |

| Group | Mouse ID | Weight | Injection remarks | time | nor-TS |
|---|---|---|---|---|---|
| A. Mock | 4 | 21.9 | ok | 48h | 1.18E+00 |
| | 6 | 19.2 | 1.8ml | **48h** | 1.48E+00 |
| **B. SM, 50ug** | 9 | 18.6 | 1.7ml | | 2.07E+00 |
| | 10 | 19.6 | 1.7ml | | 5.70E+00 |
| **B. SM 50ug** | 1 | 19.4 | 1.8ml, slow recovery | **4wk** | 1.48 |
| | 2 | 18.5 | 1.8ml, B | | 2.07 |
| | 7 | 18.3 | 1.8ml | | 5.70 |
| **C. PTM, 75ug** | 11 | 22.1 | 1.8ml, B | | 1.89E+00 |
| | 12 | 19.7 | 1.7ml, Brett tried, Jun completed | **48h** | 9.42E+01 |
| | 13 | 21.8 | 1.8ml, B | | |
| | 15 | 16.9 | 1.6ml | | 1.35E+03 |
| | 16 | 20.2 | 1.8ml | | 1.29E+02 |
| | 17 | 18.3 | 1ml | | 2.18 |
| **C. PTM, 75ug** | 18 | 18.5 | 1.8ml, B | **1 Wk** | 1.09 |
| | 19 | 18.6 | 1.6ml | | 31.50 |
| | 20 | 20.1 | 1.7ml | | 188.71 |
| | 25 | 20.9 | 1.8ml | | 44.55 |
| **C. PTM, 75ug** | 21 | 18.9 | 1.7ml | | 47.84 |
| | 22 | 17.4 | 1.6ml | **2 wk** | 64.03 |
| | 23 | 19.9 | 1.7ml | | |
| | 24 | 20.1 | 1.7ml, B several attemps | | 1.40 |
| | 28 | 18.6 | 1.7ml, B | | |
| **C. PTM, 75ug** | 26 | 18.2 | 1.7ml, B | **4 wk** | 0.19 |
| | 27 | 19.6 | 1.7ml | | 25.92 |
| | 29 | 21.3 | 1.8ml, B | | 1.33 |
| | 30 | 16.5 | 1.5ml | | 3.93 |
| | 35 | 19.4 | 1.7ml | | 7.25 |
| **D. mAlb-hAI cDNA, 50 ug** | 42 | 18.9 | 1.7ml | **48h** | 1.53E+05 |
| | 43 | 18.4 | 1.7ml, B | | 1.25E+05 |
| | 44 | 15.4 | 1.5ml | | 8.56E+05 |
| | 46 | 20.2 | 1.8ml, B | **4 wk** | 9.2E+03 |
| | 47 | 22.2 | 1.8ml, B 3hrs later | | 4.6E+03 |
| | 48 | 18.1 | 1.7ml | | 9.8E+01 |

### 14 EXAMPLE: IN VIVO TRANS-SPLICING OF HUMAN APOAI PTM INTO MOUSE ALBUMIN PRE-mRNA INCREASES HIGH-DENSITY LIPOPROTEIN (HDL)

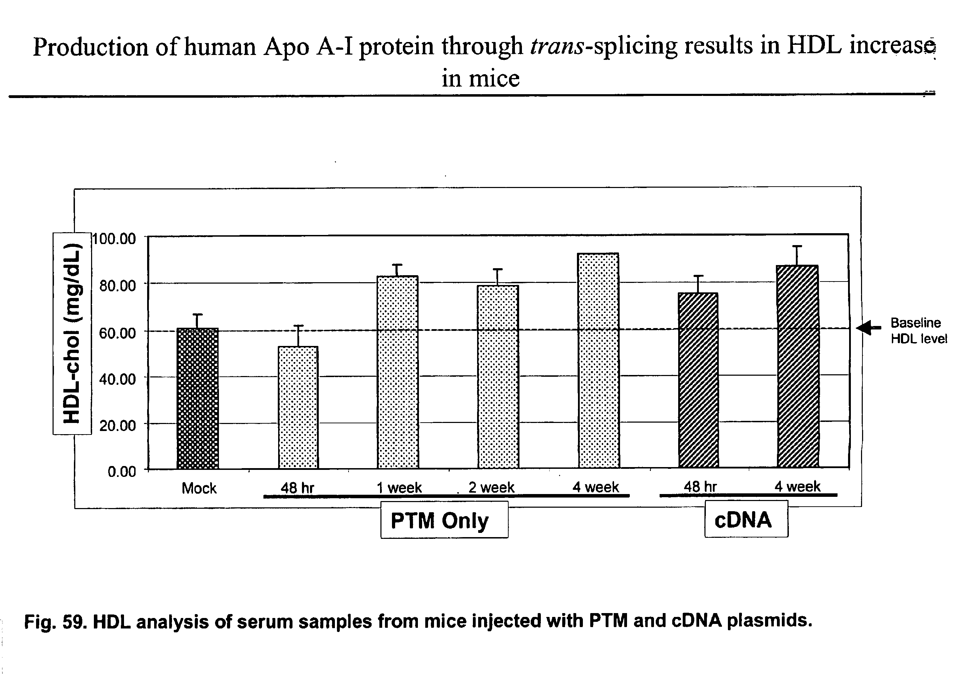

One of the main objectives of the current study is to determine whether production of human apoAI protein through trans-splicing contributes to HDL increase *in vivo.* To test this, mice were injected with mouse albumin PTM (PTM only) and control cDNA plasmid (mimics *trans*-spliced mRNA), as described above. Serum samples were collected at different time points (48 hrs through 4 weeks) and total HDL-cholesterol was determined by dextran sulfate precipitation method and the results were compared with controls. Specifically, 12 µl of serum was mixed with 4 µl dextran sulfate precipitation reagent plus 30 µl saline and, after 10 min at room temperature, was centrifuged for 30 min (4°C) at 12,000 rpm. The clear supernatant (40 µl) was mixed with 169 µl saline and total cholesterol was measured using FPLC. The baseline total HDL-cholesterol in the control group averaged to about 60 mg/dL. At 48 hrs time point, ~25% increase in total HDL-cholesterol was observed in the control cDNA group that expresses an mRNA that is identical to trans-spliced mRNA. In contrast, no significant increase was observed in the PTM group at 48 hrs. However, as shown in Figure 59, significant increases (25-50%) in total HDL-cholesterol was observed in serum samples collected at 1, 2 and 4 week time points in mice treated with PTM only and also mice treated with cDNA. Accordingly, the results presented in this application clearly show: (a) successful and accurate *trans*-splicing of mouse albumin PTM into mouse albumin target pre-mRNA, (b) production of human apoAI protein through trans-splicing and, most importantly, (c) production of human apoAI protein through trans-splicing in mice leads to significant (25-50%) increase in HDL level over the baseline. Increases in HDL blood levels are associated with reduced risk of cardiovascular disease. Numerous reports have indicated that "increasing the HDL cholesterol level by 1 mg may reduce cardiovascular risk by 2-3 percent (Castelli WP. Cholesterol and lipids in the risk of coronary artery disease - the Framingham Study. Can J Cardiol 1988; 4 [Supp1 A]:5-10A; Third Report of the National Cholesterol Education Program [NCEP] Expert Panel on Detection, Evaluation and Treatment of High Blood Cholesterol in Adults [Adult Treatment Panel III]. Final Report. Bethesda [Md]: National Cholesterol Education Program, National Heart, Lung, and Blood Institute, National Institutes of Health; September 2002. NIH Publication 02-5215. Brewer BH, 2004, *Am Heart J,* **148**, S 14-S 18; Brewer BH, 2004, *N Engl J Med*, **350**, 1491-1494)

The present invention also provides a pack or kit comprising one or more containers filled with one or more of the ingredients of the compositions of the invention. The pack or kit may include a notice in the form prescribed by a governmental agency regulating the manufacture, use or sale of pharmaceuticals or biological products, which notice reflects approval by the agency of manufacture, use or sale for human administration.

## Claims

1. A nucleic acid molecule which is recognised by nuclear splicing components within a target cell and comprises:
a) one or more target-binding domains that target binding of the nucleic acid to an albumin pre-mRNA expressed within the target cell;
b) either (i) a 3'- splice region including a 3'-(splice acceptor site, a branchpoint and a poly-pyrimidine tract or (ii) a 5'-splice donor site;
c) a spacer region that separates said 3'-splice region or 5' splice site from the target-binding domain and
d) a nucleotide sequence encoding a therapeutic protein or polypeptide of interest to be trans-spliced to the albumin pre-mRNA to provide a chimeric RNA, with the proviso that said therapeutic protein or polypeptide is not apolipoprotein A1 or a variant thereof,

2. A nucleic acid molecule as claimed in claim 1 wherein said one or more target binding domains target binding of the nucleic acid to albumin pre-mRNA such that the coding sequence for said therapeutic protein or polypeptide of interest is trans-spliced to exon 1 of said pre-mRNA.

3. A nucleic acid molecule according to claim 1 or claim 2 which provides in the protein product encoded by said chimeric RNA a protease cleavage site whereby said therapeutic protein or polypeptide of interest can be cleaved from the amino acids originating from albumin.

4. A nucleic acid molecule as claimed in claim 3 which encodes said therapeutic protein or polypeptide of interest joined to its native secretion signal.

5. A nucleic acid molecule according to any one of claims 1 to 4, wherein said therapeutic protein or polypeptide of interest is selected from cytokines, growth factors, insulin and antibody polypeptides.

6. A nucleic acid molecule according to any one of claims 1 to 4, wherein said therapeutic protein or polypeptide of interest is selected from hormones and enzymes.

7. A nucleic add molecule according to any one of claims 1 to 3, wherein the protein of interest comprises Factor VIII.

8. A DNA vector which comprises a DNA sequence which encodes a nucleic acid molecule as claimed in any one of the preceding claims.

9. A nucleic acid molecule or vector according to any preceding claim for use in therapy.

## Patentansprüche

1. Nukleinsäuremolekül, das von nuklearen Spleißkomponenten innerhalb einer Zielzelle erkannt wird und umfasst:
a) eine oder mehrere zielbindende Domänen, die auf die Bindung der Nukleinsäure an eine Albumin-prä-mRNA gerichtet sind, die innerhalb der Zielzelle exprimiert wird;
b) entweder (i) eine 3'-Spleißregion, die eine 3'-Spleißakzeptorstelle, einen Verzweigungspunkt und einen Polypyrimidintrakt enthält, oder (ii) eine 5'-Spleißdonorstelle;
c) eine Spacer-Region, die die 3'-Spleißregion oder die 5'-Spleißstelle von der zielbindenden Domäne trennt, und
d) eine Nukleotidsequenz, die für ein therapeutisches Protein oder ein Polypeptid von Interesse kodiert, das es mit der Albumin-prä-mRNA zu transspleißen gilt, um chimäre RNA bereitzustellen, unter der Maßgabe, dass das therapeutische Protein oder Polypeptid kein Apolipoprotein A1 oder eine Variante davon ist.

2. Nukleinsäuremolekül nach Anspruch 1, wobei die eine oder mehreren zielbindenden Domänen auf die Bindung der Nukleinsäure an Albumin-prä-mRNA gerichtet sind, so dass die kodierende Sequenz für das therapeutische Protein oder Polypeptid von Interesse mit dem Exon 1 der prä-mRNA transspleißt wird.

3. Nukleinsäuremolekül nach Anspruch 1 oder 2, das eine Proteasespaltungsstelle in dem von der chimären RNA kodierten Proteinprodukt bereitstellt, wobei das therapeutische Protein oder Polypeptid von Interesse von den Aminosäuren, die vom Albumin stammen, gespalten werden kann.

4. Nukleinsäuremolekül nach Anspruch 3, das für das therapeutische Protein oder Polypeptid von Interesse kodiert, das mit dessen nativem Sekretionssignal verbunden ist.

5. Nukleinsäuremolekül nach einem der Ansprüche 1 bis 4, wobei das therapeutische Protein oder Polypeptid von Interesse aus Cytokinen, Wachstumsfaktoren, Insulin und Antikörperpolypeptiden ausgewählt ist.

6. Nukleinsäuremolekül nach einem der Ansprüche 1 bis 4, wobei das therapeutische Protein oder Polypeptid von Interesse aus Hormonen und Enzymen ausgewählt ist.

7. Nukleinsäuremolekül nach einem der Ansprüche 1 bis 3, wobei das Protein von Interesse Faktor VIII umfasst.

8. DNA-Vektor, der eine DNA-Sequenz umfasst, die für ein Nukleinsäuremolekül nach einem der vorstehenden Ansprüche kodiert.

9. Nukleinsäuremolekül oder Vektor nach einem vorstehenden Anspruch zur Verwendung in der Therapie.

## Revendications

1. Molécule d'acide nucléique qui est reconnue par des composants nucléaires d'épissage au sein d'une cellule cible et qui comprend :
a) un ou plusieurs domaines de liaison à une cible qui ciblent la liaison de l'acide nucléique à un pré-ARNm de l'albumine exprimé au sein de la cellule cible ;
b) soit (i) une région d'épissage en 3' comprenant un site accepteur d'épissage en 3', un point de branchement et une séquence polypyrimidine ou (ii) un site donneur d'épissage en 5' ;
c) une région espaceur qui sépare ladite région d'épissage en 3' ou site d'épissage en 5' du domaine de liaison à une cible et
d) une séquence de nucléotides codant pour une protéine ou un polypeptide thérapeutique d'intérêt devant subir un trans-épissage au niveau du pré-ARNm de l'albumine afin d'obtenir un ARN chimérique, à condition que ladite protéine ou ledit polypeptide thérapeutique ne soit pas l'apolipoprotéine A1 ou un variant de celle-ci.

2. Molécule d'acide nucléique selon la revendication 1, dans laquelle ledit ou un plusieurs domaines de liaison à une cible ciblent la liaison de l'acide nucléique à un pré-ARNm de l'albumine de sorte que la séquence codant pour ladite protéine ou ledit polypeptide thérapeutique d'intérêt subisse un trans-épissage au niveau de l'exon 1 dudit pré-ARNm.

3. Molécule d'acide nucléique selon la revendication 1 ou la revendication 2, qui permet d'obtenir, dans le produit protéique codé par ledit ARN chimérique, un site de clivage par une protéase moyennant quoi ladite protéine ou ledit polypeptide thérapeutique d'intérêt peut être clivé(e) des acides aminés provenant de l'albumine.

4. Molécule d'acide nucléique selon la revendication 3, qui code pour ladite protéine ou ledit polypeptide thérapeutique d'intérêt joint(e) à son signal de sécrétion natif.

5. Molécule d'acide nucléique selon l'une quelconque des revendications 1 à 4, où ladite protéine ou ledit polypeptide thérapeutique d'intérêt est sélectionné(e) parmi des cytokines, des facteurs de croissance, l'insuline et des polypeptides d'anticorps.

6. Molécule d'acide nucléique selon l'une quelconque des revendications 1 à 4, où ladite protéine ou ledit polypeptide thérapeutique d'intérêt est sélectionné(e) parmi des hormones et des enzymes.

7. Molécule d'acide nucléique selon l'une quelconque des revendications 1 à 3, où la protéine d'intérêt comprend le facteur VIII.

8. Vecteur d'ADN qui comprend une séquence d'ADN qui code pour une molécule d'acide nucléique selon l'une quelconque des revendications précédentes.

9. Molécule d'acide nucléique ou vecteur selon l'une quelconque des revendications précédentes, destiné(e) à être utilisé(e) dans une thérapie.
